# EUROPEAN PATENT APPLICATION

(11) **EP 3 178 525 A1**
(43) Date of publication of application: **14.06.2017**
(21) Application number: 15796538.5
(22) Date of filing: 13.05.2015
(51) Int. Cl.: A62B 18/02, A62B 18/08, A62B 23/06, A62B 23/00

(54) **SMART MASK FOR HEALTH CARE SERVICE**

(30) Priority: 21.05.2014 KR 20140060709
(71) Applicant: Choi, Chong-sik, Seoul 140-729 (KR)
(72) Inventor: Choi, Chong-sik, Seoul 140-729 (KR)
(74) Representative: TBK
(86) International application number: PCT/KR2015/004753
(87) International publication number: WO 2015/178615

(57) **Abstract**

The present invention provides a smart mask which is configured to have skin compliance that is maintained over time, so as to serve to protect a user's respiratory system from contaminated outside air while protecting face skin from ultraviolet rays, wind, cold and pollutants during outdoor activities; which is configured to support a lower jaw which sags downwards due to oral muscle relaxation and gravity during sleeping, so that the mouth does not open, thereby preventing mouth breathing and leading to nasal breathing so as to prevent snoring; and which has moistening and warming functions to alleviate allergic symptoms and oral inflammatory symptoms, enables comfortable breathing during inhalation and quickly discharges carbon dioxide generated by the human body to the outside during exhalation, thereby preventing anatomical dead space and providing a smart mask which is beneficial to health. In addition, provided is a smart mask having skin compliance that is maintained over time, the smart mask leading to deep sleep by using a bio -signal measurement device configured separately and improving the hydration of facial skin, thereby being capable of adjusting the face and facial skin to be pleasant on the eye. A s a result, the smart mask can be worn by children as well as the elderly; can be worn by respiratory patients, snoring patents, allergic patients, pregnant women, the elderly and the infirm, etc. for the purpose of mitigation and treatment of the symptoms; and is wearable during work, sleep and strenuous exercise. Accordingly, the smart mask is a new concept of mask having a very prominent function of health care and facial skin care that has not existed conventionally.

## Description

### Technical Area

This invention can be worn to the many types of facial structures by forming whole with the SMARTMASK and the skin. There is no difficulty because the user's nose, mouth, and jaw are not pressed by the mask or contacted. Anyone including patients can use the mask. This mask can be used during daily activities, during sleep, and exercise. This mask has outstanding function for health management and it can be work comfortably.

Also, this is about the SMARTMASK that has many functions including comfortable wear for long hours, free activity of the jaw, and protecting the respiratory system of the user from pollution.

Also, this is about the SMARTMASK that has skin conformability that is maintained for a long time.

Also, this is about the SMARTMASK that can help sleeping when wearing during sleep.

Also, this is about the SMARTMASK that adjust the facial skin and the face while wearing during sleep.

Also, this is about the SMARTMASK that has the function for humidity and temperature so that cold air does not contact the face and it also maintains humidity of air at a certain level from the nasopharynx to the lower airway.

Also, this is about the SMARTMASK that has the function of humidification and heat to ease the inflammatory disease within the mouth and also ease allergic reaction that occurs to a user with parts where the diameter is reduced within the airway.

Also, this is about the SMARTMASK that has the function of preventing snoring by holding the lower jaw so that the mouth does not open in the case of opening the mouth to take a breath with the mouth during daily activity. The mask also induces breathing with the nose so that the mouth does not open by holding the lower jaw which moves due to ease of oral muscle and gravity during daily activity and during sleep.

Also, this is about the SMARTMASK that has the function of preventing the anatomical dead space by composing ventilating part and the filter part close to both nostrils in the internal gas space which is formed where only the nose is positioned by separating the mouth and the nose on the upper part of the cover part so that the carbon dioxide can quickly be discharged to the external air when breathing outs o the user can take comfortable breaths.

Also, this is about the SMARTMASK that has the function of using the body signal measuring device composed additionally and which can be attached/detached, to make body signal of the body easier in special cases and daily activity for exact measurement and it has the function of measuring the body signal so that the user can immediately detect the body signal.

Also, this is about the SMARTMASK that provides useful information to health management by attaching/detaching the communication device and measuring device easily.

### BACKGROUND TECHNOLOGY

The internet was developed for computers operated by people to communicate through TCP/IP Protocol. However, there are studies that are done to execute communication through internet for the things or objects possible of classification, calculation, and communication. Like this, the technology where objects (things) can communicate is called Internet of Things and/or Internet of Objects.

The term Internet of Things (IOT) first appeared in the Auto-ID lab of Massachusetts Institute of Technology (MIT) in 1998. Later, ITU-T presented annual report called 'The Internet of Things' . Like this, the Internet of Things was predicted to become the foundation that holds all structures of Future Information Technology (IT) Industry Revolution. The report defined Internet of Things as "All objects (things) that exist in the world are connected through internet to become the new information and communication basis so that people can communicate with objects and objects can communicate with each other anywhere and anytime." Thus, Internet of Things is an infrastructure to implement ubiquitous space. This ubiquitous space starts from being intellect from environment and objects to the computing devices with specific function.

This skill related to ubiquitous can be applied to all areas in the human lives. Especially, recently ubiquitous health care (U-Health Care) has been focused as a notable technical area due to the well-being phenomenon. Ubiquitous health care is installing a chip or sensor regarding medical service to the daily spaces of humans and means the ubiquitous technology where all people can be naturally provided with medical service anywhere and anytime. According to ubiquitous health care, people do not have to go to the hospital and meet with doctors for disease management, emergency management, or health diagnosis.

The technology of detecting Photo-Plethysmography (PPG) and Electocardiogram (ECG) is being developed as a type of ubiquitous health care. Here, the ECG method uses the electric status that is created during heart beat to monitor electrocardiogram, and the PPG method uses light sensor to monitor heart beat and blood oxygen saturation (SP02).

On the other hand, modern people are being threatened from fine dust, yellow dust, and exhaust. Therefore, it is essential to provide a mask that can protect people to maintain health from environmental pollution. Especially, the mask covers the face to play the key role of fulfilling safety from cold, ultraviolet rays, or the pollution environment.

Along with this, more and more people in the modern society are putting effort in executing outdoor activities and outdoor exercises to find vitality of life or maintain health. However, the biggest interference is blocking the ultraviolet rays. To block the ultraviolet rays, people wear sun cap (that blocks ultraviolet rays) or cover the whole the face. The sub cap or existing mask has the problem of reducing the effect of blocking ultraviolet rays only near the eyes. In the case of the mask, there is problem with humidity when breathing and the vision also become s small where the face of the mask user cannot be classified. Due to this, there are cases where others can feel uncomfortable or feel hatred.

In the past, the mask was only used to execute the function in the prevention of inhaling pollution in places such as industrial sites. Also, the mask was used to block the coldness during the winter season. Recently, the mask is worn for the purpose to prevent being exposed to pollution or germs from others or to prevent respiratory diseases due to gas, or it is used to prevent ultraviolet rays according to use.

This kind of design causes several problems. One of the problems is that the facial form is straight where the mask does not match to the face. Thus, it can bother the mask user. This interference can be aroused especially when the mask rubs the skin near the nose and mouth, when opening or closing the mouth, or when the mask user speaks. This problem also gives bad influence in motivating the mask user. Therefore, if the both ear hanging parts are loosened to lessen interference, the inappropriate tension of the ear part can break away from the arrangement of the purpose of wearing the mask.

Also, another issue is that the mask is hung to the ears and relies on the string that is adjusted to the ear and the status of wear is maintained. Thus, the pressure on the ear part is serious and the mask cannot be worn in fixed status. Also, the mask moves up and down during wear where it feels very uncomfortable and there is the issue of wearing the mask for a long time.

Especially, normally masks create friction by contacting the mouth and the nose when worn. The covering part according to wearing the mask makes breathing difficult. Also, the material that is used for masks make ventilation area smaller because it directly contacts the nose and the surface of the face. Moreover, the inside part of the mask become wet when talking with others or exercising. Thus, it makes it difficult for the user to breathe. This is very unhealthy for patients with respiratory disease or pregnant women.

Also, for the users that wear glasses, the hot breath inside the mask is not able to be discharged to the front due to the mask. The air is discharged to the nose and the part where the nose and cheek meets to make the glasses foggy. Thus, the sight of the user becomes blurry.

Also, from the existing masks, the masks that are made of upper panel, lower panel, and front panel press the nose and mouth of the user to give inconvenience. To relieve this kind of inconvenience, the interval between the front panel and the user's face can be increased, but the mask becomes bigger where it can cover the sight of the user.

Also, from the existing masks, the masks that have curve according to the face part that covers the user is made of flexible material. This covers the user's face. Because the space between the user's face and mask is small, it can cause inconvenience during activities such as opening the mouth.

On the other hand, the mask prevents inflammation for doctors or nurses from hospital germs and harmful substances. However, the mask is also worn by doctors or nurses to protect the patient.

Also, in the case of researchers and students that execute experiments using chemical products or for children that play with sharp things like guns or knives, also people that cook need to protect the face.

However, the existing mask is made of cloth or paper material where the mask becomes foggy due to breathing. In the case of users that wear glasses, the dampness also influences the glasses to cover up the sight. Also, the existing mask is contacted to the face when wearing to give inconvenience in becoming wet due to moisture when breathing or when wearing the mask for a long time. Also, the mask that is made of cloth or paper material does not have a proper structure of the material where virus can be infiltrated when breathing.

On the other hand, snoring is the informal term that indicates occlusive breathing. The characteristic is having comparatively high induction rate. As airway physiology developed, snoring has been considered as a disease. Snoring occurs when the throat becomes narrow before the air passes the airway. It is a symptom that occurs when having difficulty breathing during sleep. The frequency of occurrence of snoring occurs in 20% of men and 5% of women from ages 30∼35, and 60% of men and 40% of women after the age of 60. Also, the frequency of occurrence of sleeping apnea occurs is 4% of men and 2% of women.

Generally, people tend to open the mouth because the lower jaw drops due to the influence of gravity and ease of oral muscles during sleep. When the mouth opens during sleep, the muscle tongue is pushed towards the airway to block the airway, and this interferes enough breathing to induce snoring or sleep apnea syndrome.

Breathing through the mouth during sleep can lead to snoring and apnea which can lead to short sleep. This is very dangerous. Short sleep and apnea can sometimes cause sudden death. The most important thing for a person is breathing exercise. The next thing is eating. The next thing is rest and sleep. In breathing, there is breathing with the nose and breathing with the mouth by using the airway. Humans are known to be the only living habitat that can breathe with the mouth. The mouth is an organ to speak, and to eat food or drink. When breathing with the mouth, a person can have allergies or breathing problems, and it can also cause malocclusion of the teeth. Also, when breathing with the mouth, the moisture in the mouth is quickly evaporated to make the mouth dry, and the dust in the air does not filter where it is taken in to the body. This can lead a person to be exposed to inflammation. Also, the speed of discharging carbon dioxide becomes faster and the brain starts to form sputum leading to slow breathing and contracting the blood vessels. Also, there is not enough time to extract the oxygen from the air that is taken in to the lungs to reduce blood oxygen. Like this, breathing with the mouth can induce abnormality of the body functions such as xerostama, sleeping disorder, headache, and chronic tiredness. Thus, tiredness does not become relieved in the case of sleeping while breathing with the mouth.

Drawing 21 a is the drawing of the specific part of the airway.

When referring to Drawing 21, the drawing is the side view of a snoring patient. When looking at the drawing, the airway of the snoring patient has the part where the airway rapidly becomes narrow like a canyon. It is indicated in white color. This part is formed according to the sagging of muscles around the airway while the tongue inside the mouth is pushed towards the back side of the brain when breathing with mouth opened during sleep. Also, this is the cause of obesity. Like this, when there is the part where the airway suddenly reduces, the speed of the air that passes the narrow airway becomes faster and the pressure within the airway reduces as well. When the pressure within the airway reduces, the surrounding muscles start to gather towards the part where the air flows quickly. Thus, when specific part starts to become narrow in the center of the airway, the speed of the air that passes the narrow airway part starts to flow faster and the pressure of the air that passes the narrow airway part reduce to form low pressure. Therefore, the pressure of the air that passes the narrow airway part is formed lower than the surrounding pressure, and the muscle surrounding the blocked airway forms suction to be pulled towards the inner part. Thus, the airway of the patient becomes more blocked. Therefore, the symptom becomes worse as the patient breathes. In this case, the patient starts to snore because sound wave occurs due to the vibration of the air molecule as the air is pressured in the airway part and then let out. Snoring can also induce issues of interfering sleep.

Also, patients with narrow diameter of the airway show increase of resistance in the narrow airway part and the blood in the airway area with increased resistance repeatedly opens and closes to show oxidization. Thus, a person with acidified blood in the airway part is created with flower powder allergies leading to continuous sneezing and nasal discharge. Furthermore, a person with acidified blood in the airway part is acidified with saliva. In this case, the acidified saliva increases the number of bacteria in the saliva. Then, the saliva with increased number of bacteria flows within the mouth to create inflammation within the mouth. Also, mouth ulcer can occur due to the saliva. Especially, the in dry state or when the moisture of the air that goes into the nasopharynx and lower airway is low, various respiratory diseases can occur. In the state where the temperature of the air in the nasopharynx and lower airway is slow, various respiratory diseases can occur.

This dried state of respiratory organ and continuous inlet of cold air have been reported to be related to natural death during sleep for elders.

On the other hand, rhinitis is the inflammation that occurs in the nasal mucous membrane inside the nose, which is the breathing organ of the body. Rhinitis can be classified into chronic rhinitis, virus nasal mucous membrane due to cold, and allergic rhinitis. Like this, there is difficulty in breathing when having rhinitis and it also causes inconvenience along with reduction of attention. Especially, there are more rhinitis patients due to the increase of air pollution and environmental pollution. The cause of inducing allergic rhinitis is usually due to bed bugs, flower powder, mold, fur of animals, and fragments of cockroaches. However, allergic rhinitis can be induced due to additives or food wastes. Generally, in the treatment of allergic rhinitis, the best and most important treatment is to avoid the causes that create allergies.

### Details of Invention

### Technical Assessment.

This invention was made to resolve the issues described above. By providing SMARTMASK that can be worn comfortable to the face, this invention has the purpose in providing health management to be worn for the purpose of treatment and ease of symptoms for snoring patients, rhinitis patients, respiratory patients, and elders.

Also, there is the purpose of providing SMARTMASK with skin conformability that can be maintained over time.

Also, there is the purpose of providing SMARTMASK to adjust the skin and the face by wearing the mask during sleep.

Also, there is the purpose of providing appropriate SMARTMASK to be used during sleep while fulfilling clean wear by protecting the face and the facial skin.

Also, there is the purpose of providing SMARTMASK where users can freely talk without interference due to friction or vibration.

Also, there is the purpose of providing SMARTMASK to protect the skin and breathing from ultraviolet rays, wind, cold, and pollution during outdoor activities.

Also, there is the purpose of providing SMARTMASK to protect hospital germs and contaminants from being exposed due to the user or other person by preventing harmful substances going into the respiratory system of the user.

Also, there is the purpose of providing SMARTMASK with separate measuring device on the mask and/or the communication device can be attached/detached simply to provide useful information for health management.

Also, a small internal gas space where only the user's nose is fit to the upper internal part of the cover part when wearing the mask where the cover part covers the user's nose and mouth at the same time. The nose is also located in the small opening space where the air is indirectly filtered through the filter part, and the mouth can breathe clean air with prescribed temperature and humidity because it is positioned in the opening space that is directly ventilated with the external part.

Also, breaths can be taken easily in and out. During short breaths, the carbon dioxide that is formed in the body is quickly discharged to the external space where the nose and mouth on the upper part of the cover part are separated to form a small internal gas space. The nostrils in the internal gas space composes filter part and discharge part where there is a purpose of providing useful SMARTMASK to the health by preventing anatomical dead space.

Also, while the humidity of the air flows into the lower airway from the upper airway, the cold air is blocked to have humidity and heat functions. The diameter within the airway eases inflammatory symptom within the mouth along with allergies, and snoring occurring in the user.

Also, there is a purpose of providing SMARTMASK that induces snoring by preventing snoring with the mouth by preventing the mouth to open while supporting the lower jaw due to ease and gravity of muscle of the mouth during daily life and during sleep.

Also, there is a purpose of providing SMARTMASK where the user can immediately detect the body signal by accurately measuring the body signal of the user in special cases and daily life of the user by using body signal measuring device that can be attached/detached.

Also, there is a purpose of providing safe system when using the SMARTMASK to appropriately respond to body signal change by measuring accurate body signal during daily life, during exercise, and during sleep.

This invention is a SMARTMASK with skin conformability that can be maintained over time. The invention has an open space where the mouth is located with the small internal gas space with the nose completely separating the nose and the mouth while cover the nose and mouth at the same time. The invention also prevents anatomical dead space to breathe comfortably and it plays the role of protecting polluted air from the outside. Users can speak freely. The lower law due to ease and gravity is supported during daily life and during sleep to induce breathing with the nose. The SMARTMASK also has the function of humidity and heat to resolve the technical assessment.

Also, the body signal of the user is accurately and easily measured in specific case and daily life. The pattern of the change in the saved body signal is compared and judged. The result can be immediately detected by the user. When the user is in danger, the SMARTMASK is provided to quickly be handled by family, by doctor, and by hospital to resolve technical assessment.

### Effect of Invention

According to the SMARTMASK on one side of this invention, SMARTMASK helps maintain skin conformability that is maintained over time. In any case, the mask can be work for a long period because comfortable breathing is possible without any interference according to friction or circulation. The mask can be worn by children to elders. It is worn for the purpose of easing symptoms and treatment for respiratory patients, snoring patients, allergy patients, and weak elders. The mask can also be worn during exercise, during sleep, during work, and during daily activities. This is a health management mask that has never existed in the past.

According to the SMARTMASK on one side of this invention, the upper internal part of the cover part can be acquired with a small internal gas space where only the user's nose can fit when wearing the SMARTMASK. The nose is positioned in the small opening space where the air is indirectly ventilated through the filter part. Small internal gas space can be acquired compared to existing masks. Thus, appropriate temperature and humidity of clean air are maintained in the internal gas space. The users can breathe in clean air and it can also minimize inconvenience while preventing the mouth from opening. Outstanding effect can be acquired when wearing the mask.

Also, small internal gas space where only the user's nose fits to the upper part of the cover part when wearing SMARTMASK can be acquired. The cover part covers the nose and mouth and the nose is positioned in the small opening space where air is indirectly ventilated. The mouth is positioned in the open area where the air directly ventilates from outside. Small internal gas space can be acquired compared to existing masks. Thus, when the user breathes in and out, the user can breathe clean air comfortably. Also, carbon dioxide created from the body is quickly discharged to prevent anatomical dead space where user can see outstanding effect to health.

Also, according to the SMARTMASK on one side of this invention, the SMARTMASK does not have side effects to the body where it is made of silicon with skin conformability, and it is also composed to be closely contacting the skin. Thus, the surface contacting the skin becomes a whole where there is no interference of friction according to the circulation of the SMARTMASK during daily life, during exercise, and during sleep, and it has the effect to be comfortably worn for a long period of time.

Also, according to the SMARTMASK on one side of this invention, the SMARTMASK is made of silicon with outstanding flexibility and the thickness is thin while the weight is light. It can be folded based on the left-right contact part. Thus, the SMARTMASK can be used several times and it has the structure of completely being folded where it can be easily used and mobilized.

Also, according to the SMARTMASK on one side of this invention, the mask has effect of being protected from polluted air due to the ventilation of air that is filtered through the filter part, and it can also protect the face skin from cold, wind, contaminants, and ultraviolet rays during outdoor activities.

Also, according to the SMARTMASK on one side of this invention, the mask can maintain certain standard of humidity of air that enters from the upper airway to the lower airway and also blocks cold air from coming in. It also has humidity and heating function. Users with minimized diameter within the airway can be eased with inflammation symptoms within the mouth, allergy symptom, and snoring symptom.

Also, according to the SMARTMASK on one side of this invention, the mask supports the lower jaw that sags due to ease and gravity of the oral muscles during daily activity and during sleep and has the effect of inducing breathing with the nose by preventing breathing with the mouth.

Also, according to the SMARTMASK on one side of this invention, the user can sleep while the skin is pulled upwards. The mask also maintains the status of pulling up the skin and it can adjust the face to a good shape.

Also, according to the SMARTMASK that has body signal measuring device on one side of this invention, the mask uses a body signal measuring device that can be attached/detached and continuously detects the health status of the user such as blood pressure, pulse, glucose, temperature, and psychological status in special cases and daily activity. The mask has effect to directly manage health by the user.

Also, according to the SMARTMASK that has body signal measuring device on one side of this invention, the exact body signal is measured during sleep, during exercise, and during daily activity to possess effect to appropriately respond to the body signal change.

Also ,the mask as the effect to provide useful information to health management because attachment/detachment is simple for the measuring device and/or communication device on the mask.

Also, the mask has effect for health management and aesthetic effect where the recognition of use of the user using the SMARTMASK will increase to purchase the product.

### Explanation on the Figures

Figure 1 is the drawing that shows the SMARTMASK according to Execution Example 1 of this invention.
Figure 2 is the drawing that shows the lid based on the left-right contact part on the SMARTMASK according to Execution Example 1 of this invention.
Figure 3a) is the cross-sectional plan following A-A' in Figure 2.
Figure 3b) is the cross-sectional plan following B-B' in Figure 2.
Figure 3c) is the cross-sectional plan following B'B in Figure 2.
Figure 4 is the principle plan for penetrating humidity and air of the left-right contact part according to the execution example of this invention.
Figure 5 is the image of the heat/burn measuring result after wearing the SMARTMASK according to execution example of this invention.
Figure 6a) is the cross-sectional plan following the center point of the left-right adjustment part.
Figure 6b) is the cross-sectional plan to explain the combination of the left-right upper and lower bands and the attachment part of the left-right adjustment part.
Figure 6c) is the cross-sectional plan to explain the attachment part of the left-right adjustment part.
Figure 7a) is the drawing that displays the composition of the left-right upper and lower bands according to the execution sample of this invention.
Figure 7b) is the cross-sectional plan that displays the left-right upper and lower bands.

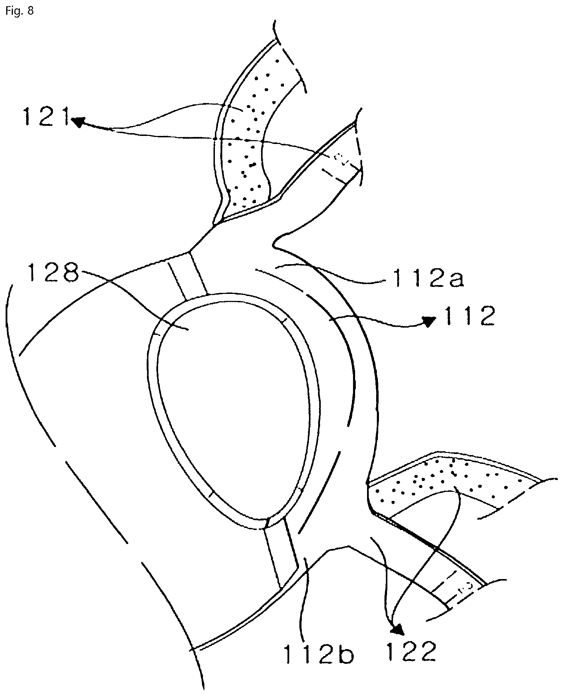

Figure 8 is the drawing that displays other composition of the left-right upper/lower band according to the execution example of this invention.
Figure 9a) is the perspective view that expanded the cover part in Figure 1.

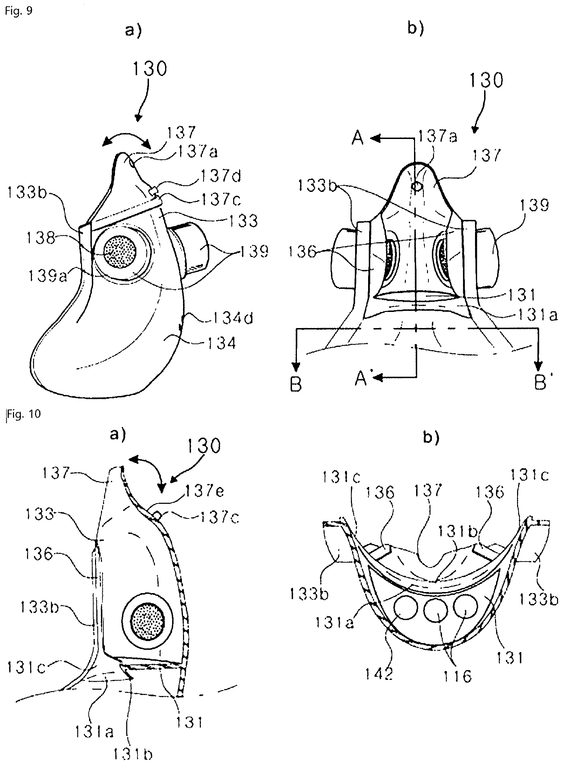

Figure 9a) is the drawing that explains the layer part in Figure 9a).
Figure 10a) is the cross-sectional drawing that follows A-A' in Figure 9b)
Figure 10 b) is the cross-sectional drawing that follows B-B' in Figure 9b)

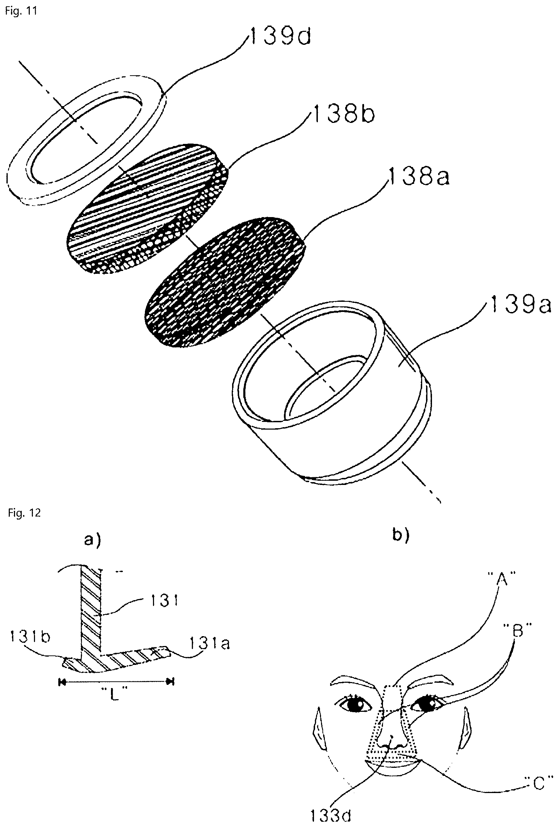

Figure 11) is the drawing that explains the filter part.
Figure 12a) is the drawing to explain the wall part.
Figure 12b) is the drawing to explain the internal gas space.

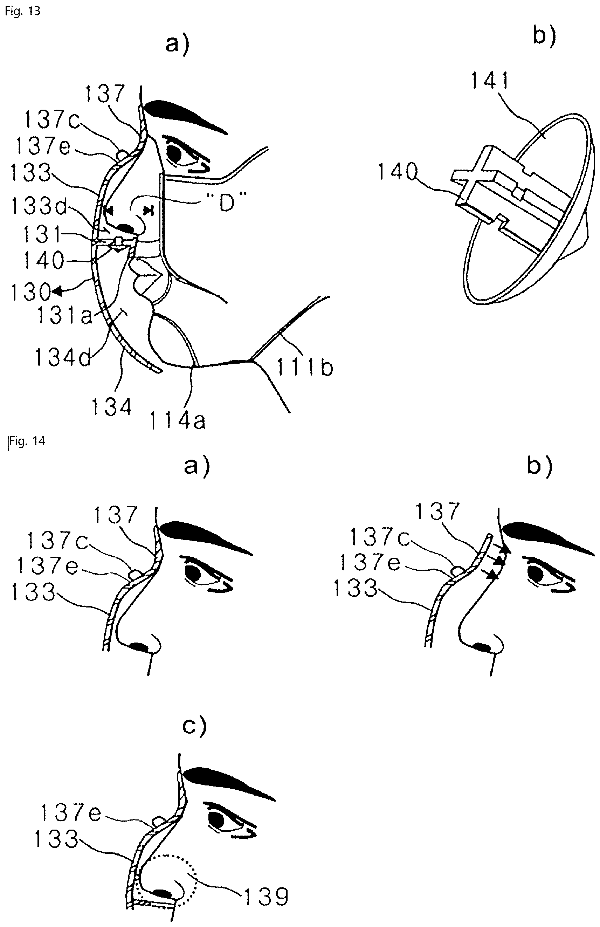

Figure 14a∼ Figure14b) are drawings to explain the lid part.
Figure 13b) is the drawing that displays the ventilation part
Figure 14a∼ Figure14b) are drawings to explain the lid part. Figure 14a∼Figure14b) are drawings to explain the lid part.
Figure 14c) is the drawing to explain the position of the filter part in the internal gas space.

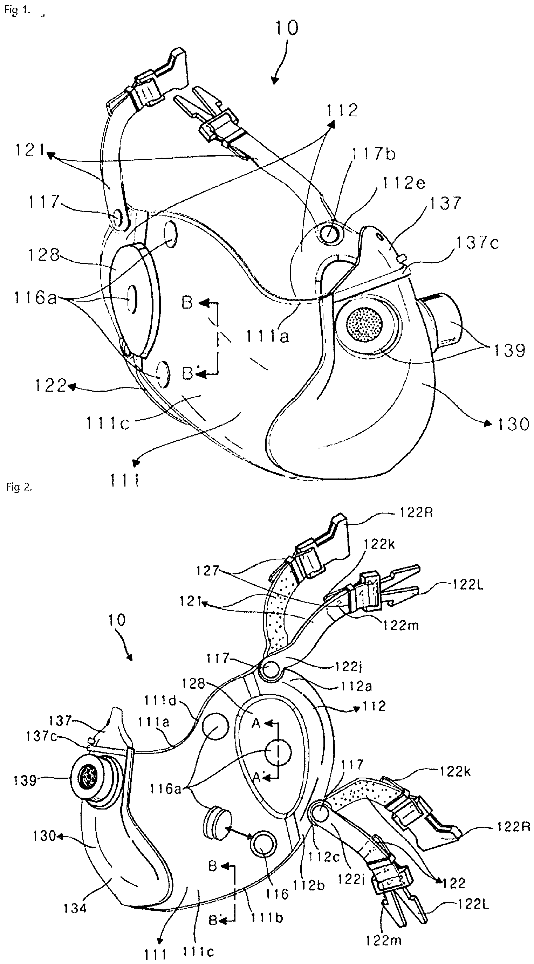

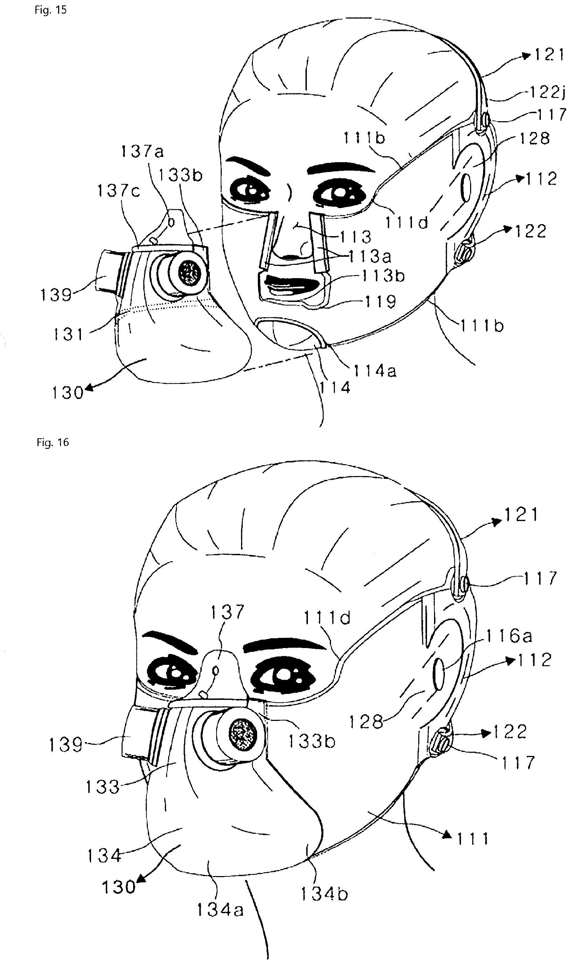

Figure 15 ∼ Figure 16 are the conditional drawings when the SMARTMASK is worn according to Figure 1.

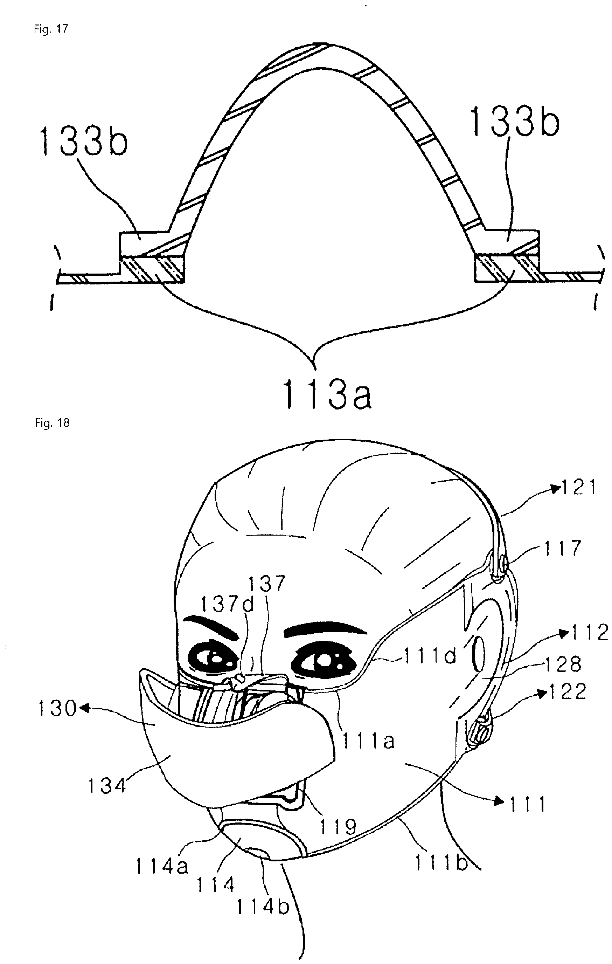

Figure 17 is the drawing to explain the combination of the opening hole and the cover part.
Figure 18 is the overall conditional drawing of when the user is wearing the mask.

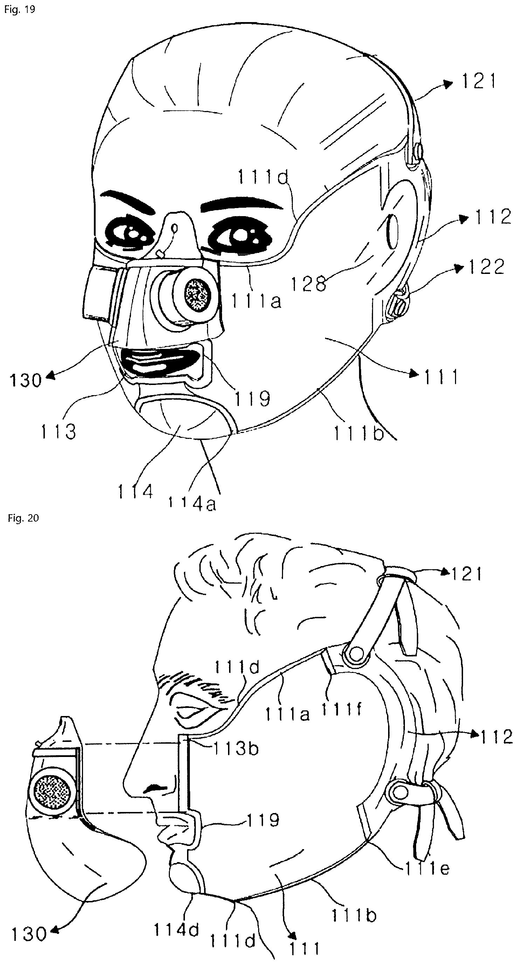

Figure 19 is the drawing the drawing of wearing the SMARTMASK without the front cover according to the execution example of this invention.
Figure 20 is the drawing to explain the size of the left-right contact part according to Drawing 1.

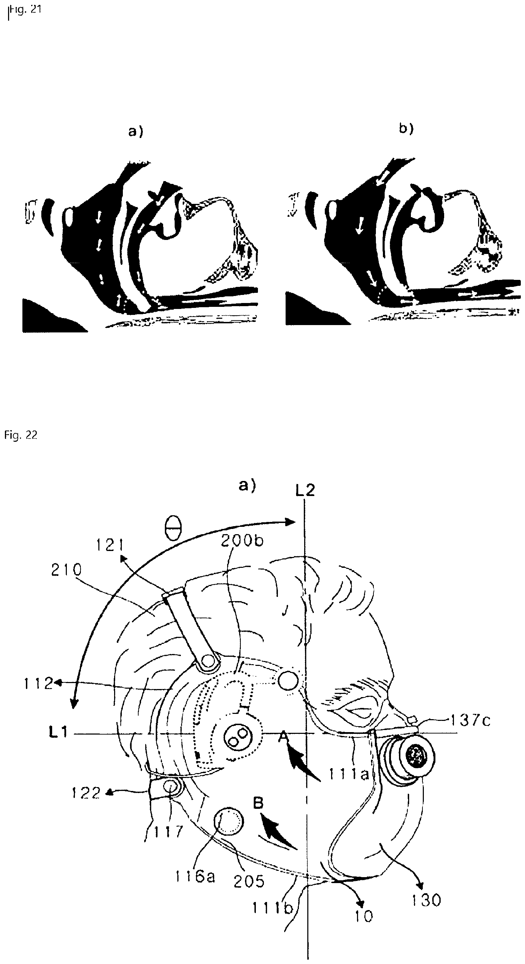

Figure 21a) ∼Figure 21b) are the drawings that displayed the specific space of the airway.

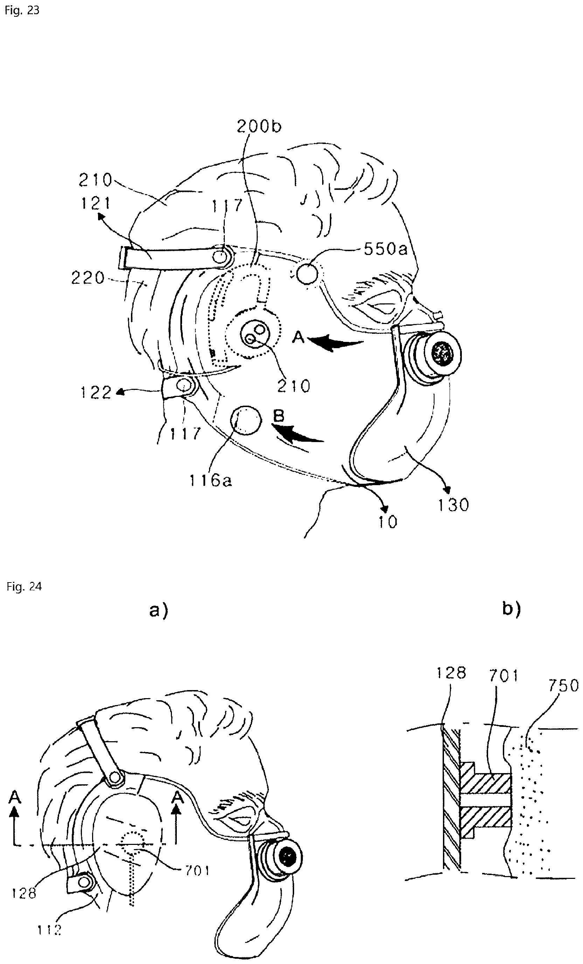

Figure 22 ∼ Figure 23 are the conditional drawings of when the user is wearing the SMARTMASK and body signal measuring device according to Figure 1.
Figure 24a) is the conditional drawing when the ear cover part is covering the sound device on the ear part.
Figure 24b) is the cross-sectional drawing that follows the A-A' in Figure 24a)

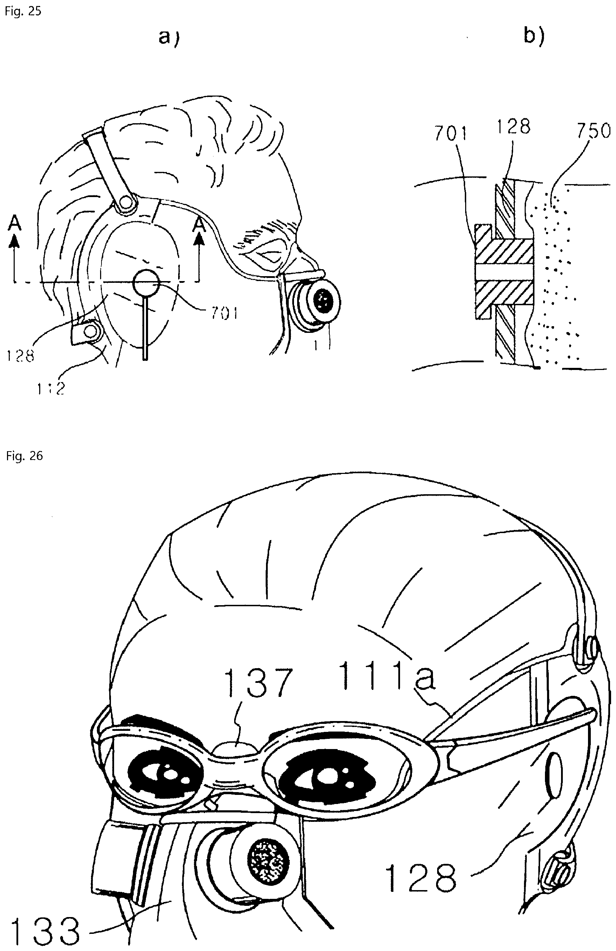

Figure 25a) is the adjusted conditional drawing through the ear cover part for the sound device that is worn on the ears.
Figure 25b) is the is the cross-sectional drawing that follows A-A' in Figure 25a)
Figure 26 is the conditional drawing of wearing the glasses on the SMARTMASK according to Figure 1.

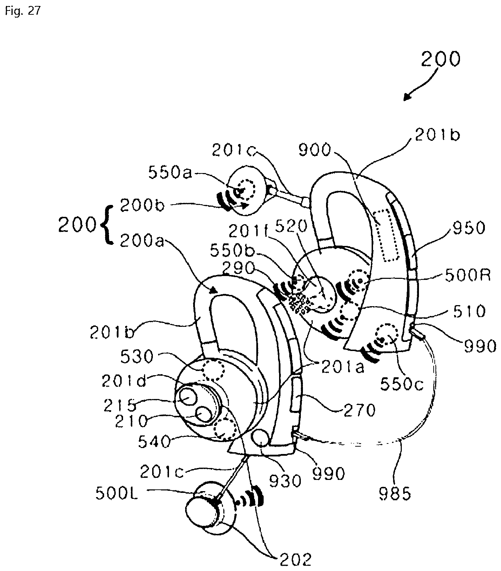

Figure 27 is the drawing that shows the body signal measuring device according to this invention.

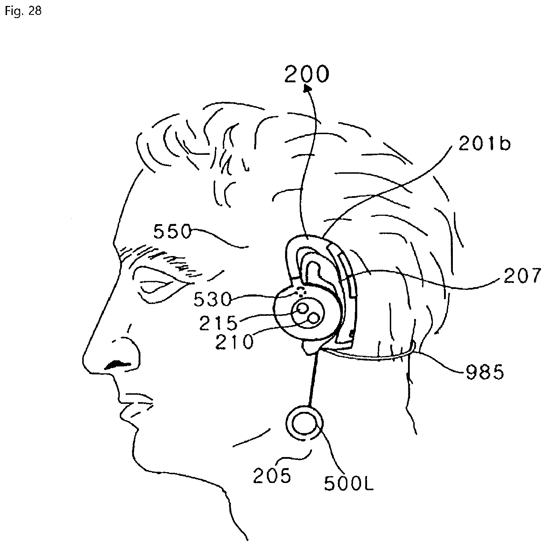

Figure 28 is the conditional drawing of wearing the body signal measuring device on the user's ears and carotid.

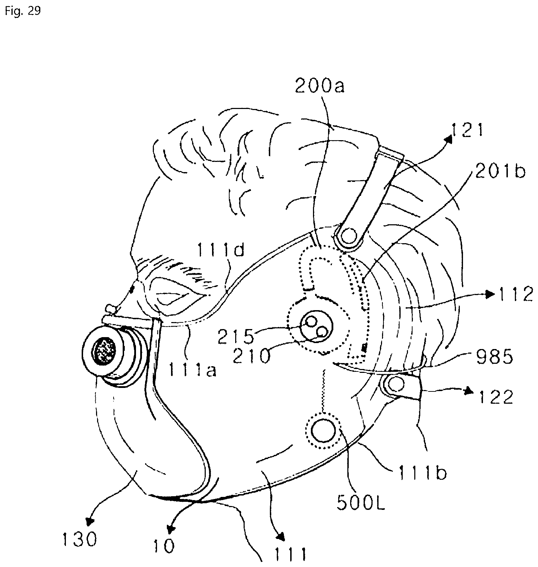

Figure 29 is the conditional drawing that measures the body signal by using the body signal measuring device on the left side of Figure 27 and the SMARTMASK of Figure 1.

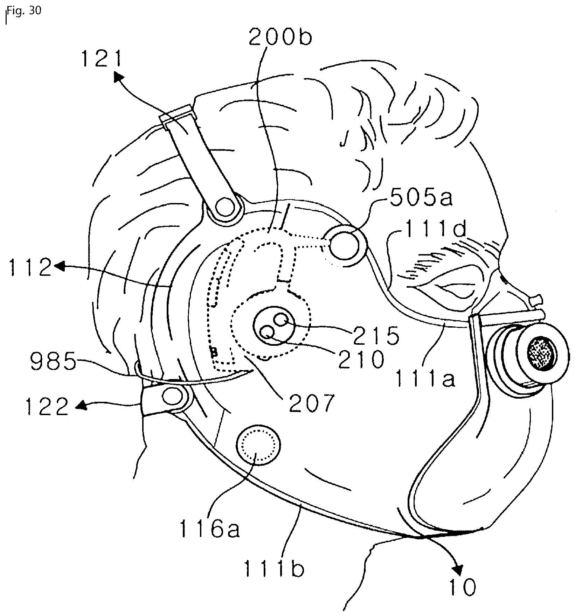

Figure 30 is the conditional drawing of measuring the body signal using right body signal measuring device of Figure 27 and the SMARTMASK of Figure 1.

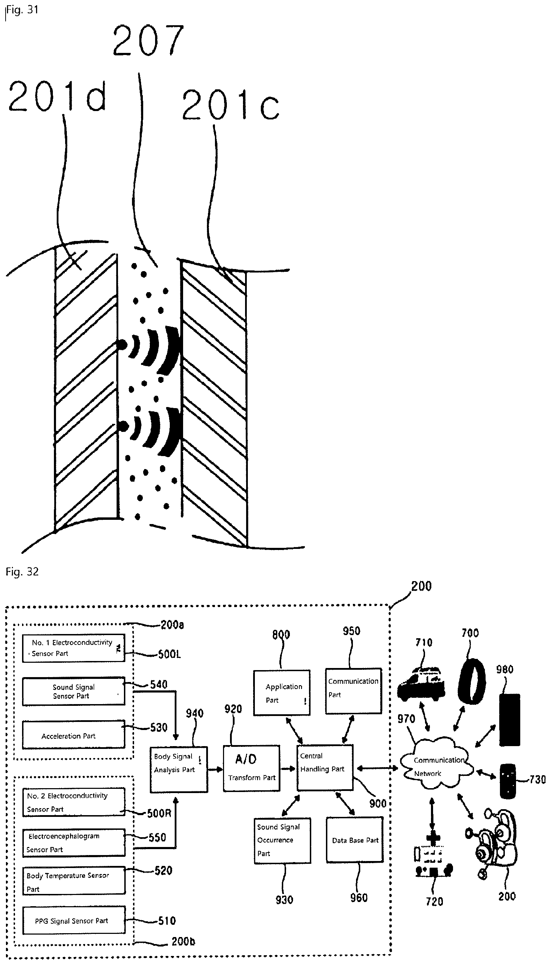

Figure 31 is the cross-sectional drawing to explain the body signal measuring device according to Fig. 27
Figure32 is the drawing that shows the composition of the whole system using the body signal measuring method using the body signal measuring device of Figure 27.

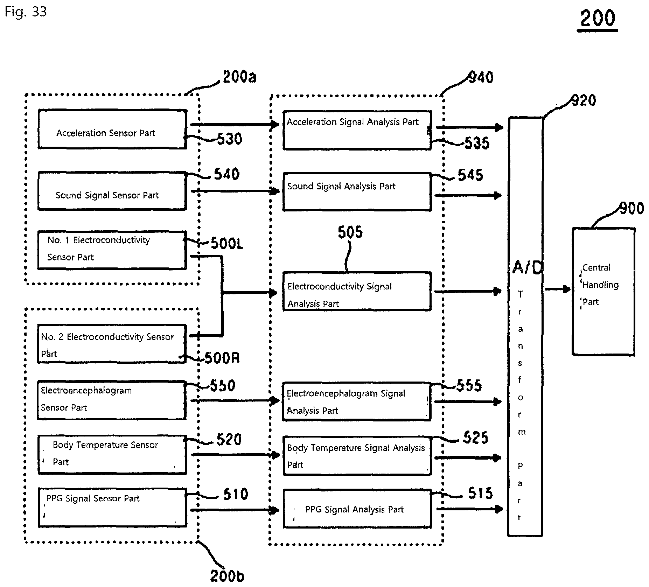

Figure 33 is the block diagram to explain the body signal detection process through body signal measuring device of Figure 27.

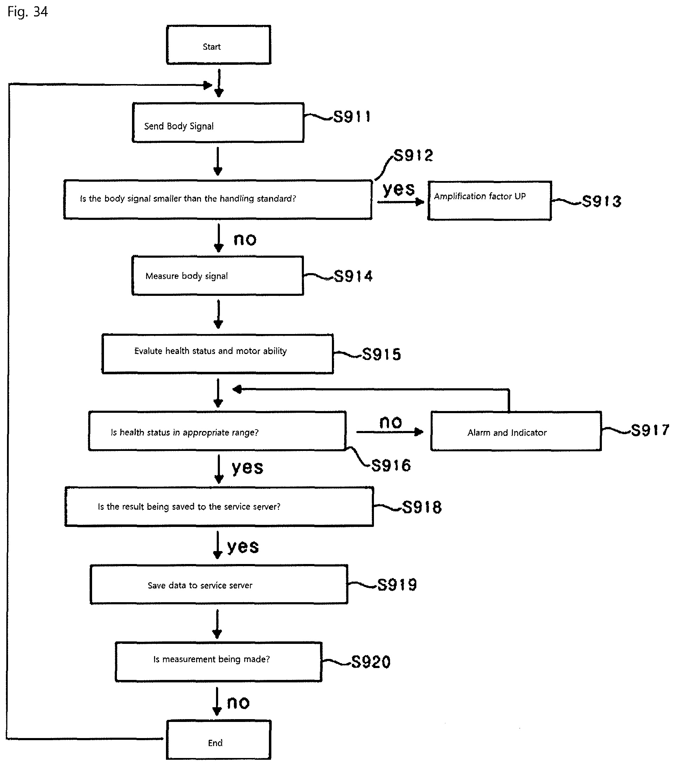

Figure 34 is the flow chart to explain the behavior of the central handling part of Drawing 32.

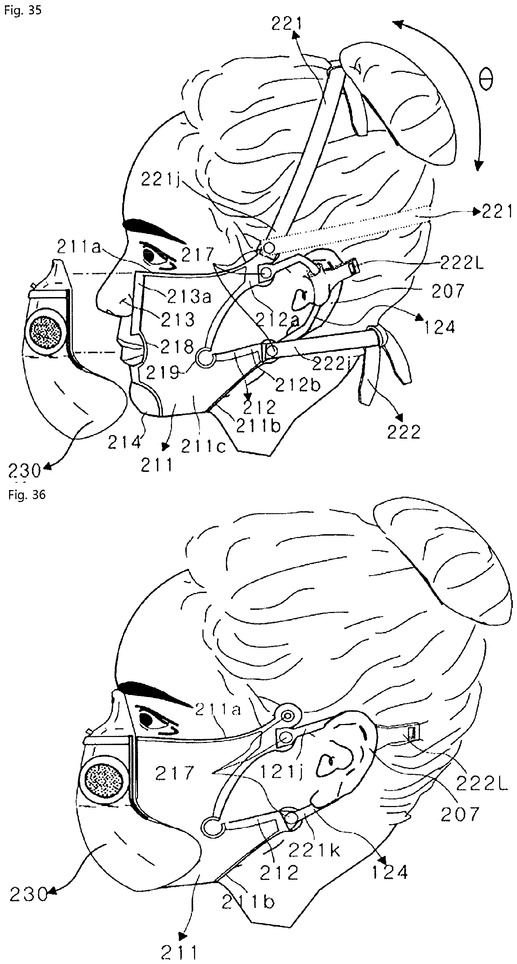

Figure 35 is the conditional drawing of wearing the SMARTMASK of execution example No. 2 according to this invention.
Figure 36 is the conditional drawing of wearing the SMARTMASK of execution example No. 2 according to this invention.

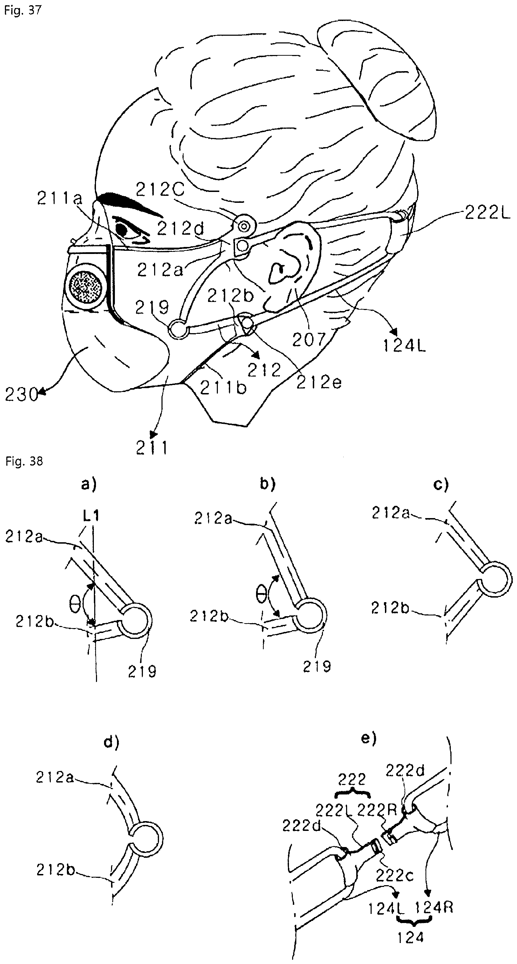

Figure 37 is another conditional drawing of wearing the SMARTMASK of execution example No. 2 according to this invention.
Figure 38a) ∼ Figure 38d) are drawings to explain the left-right adjustment part in the SMARTMASK of execution example No. 2.
Figure 38e) is the drawing to explain the hook in the SMARTMASK of execution example No. 2.

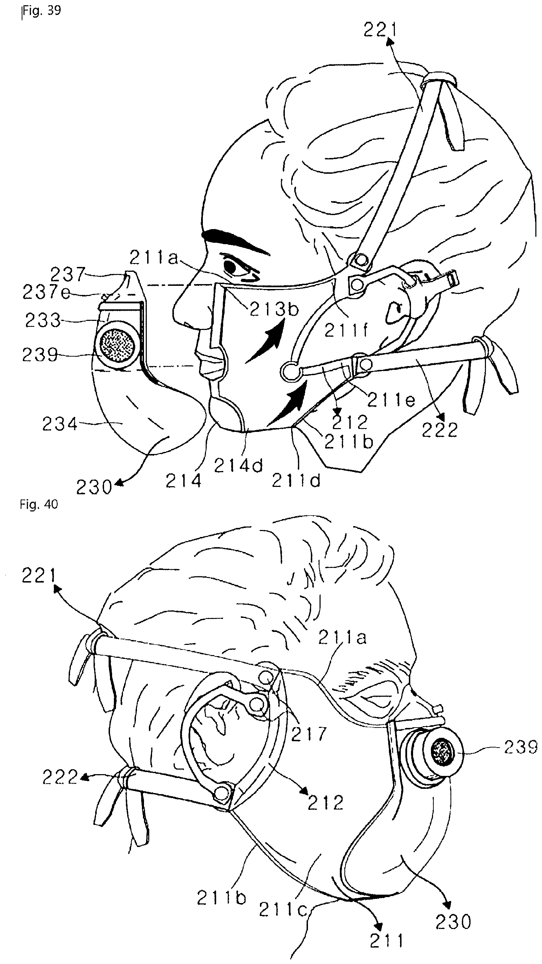

Figure 39 is the drawing to explain the size of the left-right contact part of Execution No. 2
Figure 40 is the conditional drawing wearing the other SMARTMASK of execution No. 2.

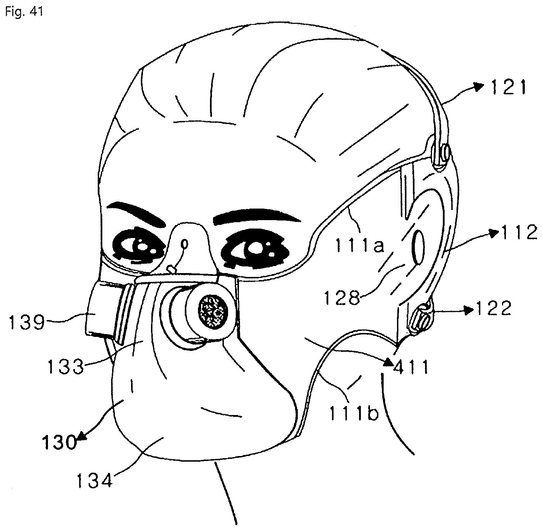

Figure 41 is the conditional drawing wearing the SMARTMASK of another execution example according to this invention.

### Best Type to Execute Invention

Left-right contact part (111) that can be composed of attach/detach of the body signal measuring device (200) by being combined of left/right upper/lower curve part (111a, 111b) by including left-right ear cover part (128) covering the left-right ears and the contact left-right contact part (111c) with skin conformability that contacts the skin; the jaw part (114) where the lower jaw is adjusted; living hinge (119) where it is opened for the mouth to stick out; and opening hole (113) where the user's nose sticks out.

Composed of extension as a whole with the left-right contact part (111) and has left-right adjustment part (112) composed to oppose to the left-right contact part (111) by including the upper part (112a) and lower part (112b)

Composed to be attached/detached to the upper/lower attachment part (112c) of the left-right adjustment part, and has left-right upper/lower band (121, 122) to wear the SMARTMASK (10); and

The mask indirectly ventilates through the filter part (139) that is separately prepared to not contact the nose that sticks out through the opening hole (113). It is combined with the opening hole frame (113a) to be closed near the nose according to the cover part, wall part, layer part, upper frame, and lid part. Only the nose can be placed inside where the small internal gas space (133d) is formed. In that place, there is the upper part (133) and living hinge (119) which do not contact the mouth but rather covers the mouth and ventilate directly with the external environment. The opened space (134d) is composed of cover part (130) including the front cover in the side of the opened space (134d). The SMARTMASK has these characteristics.

### [Complete Composition]

The terminologies and terms used in this claim and details are limited to dictionary and common meanings and shall not be interpreted. The inventor shall explain the best methods for own invention to appropriately define the concept the concept of terminology. The terminology must be interpreted by the meaning and concept that fits the technical ideas of this invention. Thus, the execution example in this details and the composition on the drawings are only executions examples and do not represent the technical ideas of this invention. It should be understood that the examples of transformation and various balance can replace these during application. The advantages and characteristics of this invention and the facts that are detailed along with the drawings can become clear when referring to the execution examples. The reference items are the same components over all details.

The surface that is exposed to the exterior part in these details is called the exterior surface and the inner part that contacts the face is called the internal surface. With no relations to wearing or not wearing the SMARTMASK (10, 20), the left direction is called left side, right direction is called right side, upper direction is called upper part, and lower direction is called the lower part. The terms used in this details use "about" "actually" and "in details" to show that there may be allowance error of manufacture and substance. To help understand this invention, the absolute or exact values are not used to explain the mentioned contents.

The term 'flexibility used in these details can be expanded up to 700% in length. It indicates recovering the length when removing the power. Thus, 'flexibility' can be used as 'elasticity'.

The term 'Combined" and 'Seal' used in these details include combining, connecting, stitching 2 objects as one and are not limited.

The terms "configure", "configuration", and "formation" used in these details are used for the purpose of applied area

And means design, arrangement, set up, and/or formation. For example, military vehicle configured for the rugged ground.

The term 'Location" and/or "arrangement" used in these details are used in the original source and mean the method of placement in specific area; For example, "Arrange clock needle".

"Skin Conformability" used in these details block liquid while ventilating gas and humidity. There is no stimulation or side effects to the skin, and it has softness with conformability without surface fiber. It means the softness to the skin.

Hereinafter, before explaining by referring to the figures, the inventor reveals of not explaining or displaying the compositions that can be announced to add by the persons working in the same field with common knowledge or unnecessary matters.

Figure 1, Figure 2, Figure 35, and Figure 36 are execution examples of SMARTMASK (10, 20) according to this invention. As displayed, the SMARTMASK (10, 20) according to the execution example of this invention include the SMARTMASK (10, 20) worn to the skin of the user and the body signal measuring device (200) composed to attach/detach selectively according to situation of the SMARTMASK (10, 20). However, this invention has been explained as a new SMARTMASK, but it can also be applied for medical treatment, protection against dust, and protecting oneself from poison.

First, the opening hole (113) opened to expose the user's nose and the living hinge (119) that is opened for the mouth, the jaw support (115) adjusting the lower jaw, left-right contact part (11c) with skin conformability, and the left-right ear cover part (128) covering the left and right ears are included, and the mask is also composed of combination of left-right upper/lower curve parts (111a, 111b) where the measuring device is composed to be attached and detached. The mask is also formed with extension as a whole with the upper left-right contact part (111) which includes the upper part (112a) and lower part (112b). Furthermore, the mask is composed to oppose the left-right contact part (111) where the left-right adjustment part (112) and the left-right adjustment part's upper/lower attachment part (112c) can be attached and detached. The SMARTMASK (10) has a filter part (139) where it does not contact the nose through the opening hole (113) and the left-right upper/lower bands (121, 122) to be worn on the face so that the air can be indirectly ventilated. It is combined with the opening hole frame (113a) to completely close the surrounding of the nose due to
the lid part, upper frame, layer part, wall part, and cover part. The small internal gas space (133d) where only the nose can be placed is composed so that the upper part (133) and the living hinge (119) are not contacted. It also covers the mouth while being able to directly ventilate. The opened space (134d) is composed with front cover (134) and cover part (130).

Also, the SMARTMASK (10) has a No. 1 electrocardiogram that is connected with a connecting line to the body to be contacting the carotid of one side. It also has No.2 electrocardiogram sensor composed to the body part so that the body signal measuring device (200a) and can contact the ear lobes of either the left or right ear. There are also several electrocardiogram sensors and PPG sensor to include right side body signal measuring device (200b).

According to the execution examples according to this invention, the opening hole (113) opened to expose the user's nose and the living hinge (119) that is opened for the mouth, the jaw support (115) adjusting the lower jaw, left-right contact part (11c) with skin conformability, and the left-right ear cover part (128) covering the left and right ears are included.

As intended by the inventor, by wearing the SMARTMASK (10, 20) during sleep, it can help the health of the user which is the purpose of implementing the SMARTMASK (10). To implement for this purpose, the most important factor starts from the effort of contacting closely to the skin, and the mask implements function of more than 7.5 MPa, above 15 N/mm, 0.2∼0.8 cc/ccm 7 sec, pressure from 7,000 to 10,000 mm H2I, above 700 growth rate (%), above 500∼ 10,000 g/m724hr, and 2-10 m² Pa/2. Even after time passes, the skin conformability material development is becoming stronger regarding health and these materials are used to make the SMARTMASK to make humidity and heat of air possible. The anatomical dead space is prevented and comfortable breathing is possible without breathing resistance.

The inventor discovered that the mask can be made by using silicon to protect the skin with strong durability to harmful substances along with ultraviolet rays and ozone without change to the structure or property even when pressure is given continuously, and the mask is close to the skin through transformation according to the structure of the face with skin conformability and transparency of gas. This was discovered during research.

The silicon used to make the SMARTMASK (10,20) of this invention has high gas transparency and is known to be harmless to the body. Because the mask has various density and properties, it can be usefully applied to medical products and/or body protection products. There is plenty of silicon material on earth where there are no worries about wasting the material. Thus, various industrial developments are being executed. Also, the silicon has outstanding flexibility, UV resistance, accessibility, friction power, stability, balance, and elasticity, which are basic factors and properties. Moreover, silicon is being developed as high function to satisfy glare and transparency.

With this characteristic, the silicon that is used to manufacture the SMARTMASK (10, 20) has strong durability to ultraviolet rays, ozone, and chemical products. Thus, the mask completely protects the skin. Not only that but when the mask is worn, it is closely contacted to the skin where air is infiltrated. It also has increased skin conformability and most users can use the mask conveniently. Moreover, without surface material, the mask is soft and conformable where it is soft when touching the skin and the surface contact power is superior.

Also, the silicon is designed to allow aesthetic design and flexibility to be focused at the same time of using the special characteristics on SMARTMASK (10, 20) that has been finally handled. The complex manufacturing process or removing or minimizing other components can maintain the aesthetic appearance.

Also, silicon can be produced to have various thicknesses and various densities. Like this, the flexibility can be selectively differed in areas. The SMARTMASK (10, 20) has different thickness and density with different smoothness of elasticity. SMARTMASK (10, 20) has increased comfortableness. For sure, the left-right contact surface (111c, 211c) is thin from 10mm to 40mm. This is because the thin silicon can be expanded from 700% ∼ 1,000% for the smoothness. The left-right upper/lower curve part (111a, 111b, 211a, 211b), opening hole frame (113a, 213a), living hinge (119, 218), jaw support frame (114a, 214a), left-right upper/lower band (121, 122,221, 222) are from 20∼200mm thickness. Because the thick silicon can be damaged, the left-right upper/lower curve part (111a, 111b, 211a, 211b), left-right contact part (111c, 211 c) are difficult to be damaged and it is best to be thick. Left-right adjustment part (112, 212) is from 50mm ∼ 110mm, and the thickness is 300µm ∼ 800 µm, and the width is from 10mm ∼ 20mm. The form is ")" shape. This is because the thick silicon has higher adjustment power to maintain the shape. As for sure, the left-right adjustment (112, 212) opposing the left-right contact part (111, 211) does not fall apart and the left-right contact part (111, 211) can be dimensionally pulled.

Also, for the silicon contact force, the silicon is very slippery and it sticks. Molding is used in the process of making the left-right contact part (111, 211) to smoothly form the lapping technology on the molding surface to the contact surface (111c, 211c). Like this, the molding surface can be smooth and the left-right contact surface becomes soft and the left-right contact surface increases for sticking. Thus, it sticks to the skin. Also, to make the glasses not fall, the molding surface can be formed smoothly due to the lapping technology on the exterior surface of the left-right contact surface. To assure quick wear and taking off the SMARTMASK, the left-right upper/lower bands (121, 122, 221, 222) are smoothly formed due to the lapping technology where simple solidarity sticks the left-right band materials. Also, the lid part (137) of the cover part, layer part (136), upper frame (133b), and cover part (131a) can be formed smoothly according to lapping technology.

In the execution status of this invention, the SMARTMASK (10, 20) can be used for the purpose to protect respiratory organ harmful environment. Also, it can be used to effectively block the user's face from harmful environment. Also, the face and/or the skin can be adjusted by 3/4. The mask can be used to improve health as well. Also, snoring, breathing disease, rhinitis, atopy, cold, and pimples can be treated. Also, It can be used while sleeping as well. Also, the body signal measuring device is used to measure the body signals such as blood pressure, glucose, and body temperature.

In the execution status of this invention, this mask can be worn for more than 10 days over long period of time when working or when sleeping, and it can be used by washing repeatedly. Also, infants, elementary school students, middle school students, and even adults can use the mask appropriately. The form can be expanded to fit adults, elders, respiratory patients, and other patients.

In the execution status of this invention, SMARTMASK (10, 20) can be used on the harmless industrial use and/or harmful environment. Example of use in harmful environment includes protection on manufacturing products, pesticides, temporary, salt, acid, and base. For example, the mask provides blocking with reliability on the exposure contaminated particles. Also, harmful industry use includes wearing the mask on site such as in factories, work place, industrial power plant, farm, and construction site.

In the execution status of this invention, SMARTMASK (10, 20) can be used to effectively block users from harmful environment as a method that is not just a face covering product. Thus, the SMARTMASK (10, 20) can be used in places to be separated by the user. This SMARTMASK (10, 20) cleans the clothes and other vestiges do not contact the user's skin. Using the SMARTMASK (10, 20) to separate people from other particles that can exist in accidents and or work places is very useful. On the other hand, SMARTMASK (10, 20) protects the environment from fragments and dusts that can be moved within the environment according to the user.

In the execution status of this invention, the SMARTMASK (10, 20) can be used with hats, jumper, underwear, clothing for hospital, experimental gown, and/or white coat for doctors. For example, it can be used for helmet, sunglasses, and glasses. Also, it can be used when riding car, motorcycle, bikes, and also when riding inline skates, walking, and running.

In general, staff will change their masks daily or every other day, depending on their industrial conditions or standards. In some cases, staff will change their masks more frequently. Disused masks used after use will not only have a negative impact on the environment, but will also cause considerable costs. Therefore, through the SMARTMASKs to eliminate contaminants, washing and / or sterilization process, constitute a recycling use is very important.

According to the present invention, the other parts of the above-mentioned SMARTMASK (10, 20) are not limited to the fact that at least the left and right attaching portions (111, 211) are made of the above-mentioned silicon. Considering ease of manufacture, stability, Mass production and other factors, will use the mold for manufacturing. The advantage of using mold manufacturing is to ensure simplicity, stability, accuracy, mass production, etc., as well as structural and aesthetic design with a high degree of control. Such a manufacturing method can be produced in the transverse direction of the SMARTMASK (10, 20) according to the invention in the range of 0.1 mm to 900 mm. May be produced in the longitudinal direction thereof in the range of 0.1 mm to 900 mm. Can be produced in the range of 10 to 1000 m.

The mold for manufacturing the SMARTMASK (10, 20) is preferably made of a first mold for manufacturing left and right fixing portions (112, 212) integrally with the left and right attaching portions (111, 211), 230), and a third mold for manufacturing the left and right upper and lower lanyard materials. Specifically, the first mold is divided into upper and lower molds, and consists of an upper mold and a lower mold. The upper mold consists of a concave portion corresponding to the face image, and the lower mold is composed of a convex portion corresponding to the face image. The top and bottom molds are positioned on the corners of the upper and lower molds to determine the relative positions of the upper and lower molds, and the upper and lower jaws are aligned with the guide pins and guide holes.

In addition, in order to reduce the risk of injury to the skin, the end surface can be rounded. To avoid the indentation of the skin, the end can be ground into a circle. Specifically, the end of the sample can be rounded according to the shape of the mold.

The left and right upper and lower lanyard materials (121, 122, 221, 222) are separately manufactured and manufactured according to the description herein. After the separation and manufacture of the left and right attaching parts (111, 211), the lid parts (130, Can be reassembled. (121, 122, 221, 22) may be added after the left and right attachment portions (111, 211) and the lid portions (130, 230) are integrally manufactured 2). This production process can be in a clean working environment, unskilled workers can easily produce. As a result, large-scale high-tech facilities or complex manufacturing technologies are not required.

The SMARTMASK (10, 20) according to one aspect of the present invention is formed by a silicone material which is harmless to the human body and is excellent in stretchability, and is capable of integrally affixing the face to the skin in contact with the face Department of skin. As a result, the facial skin and the attachment site are integrated, and there is no fear of friction caused by movement of the SMARTMASK (10, 20) during or during sleep. Therefore, there is an advantage that the SMARTMASK (10, 20) can be worn more comfortably.

In the present embodiment, the left and right attaching portions (111, 211) are slightly reduced from the upper portion to the lower portion (vertical direction) at a magnification of not less than 90% and not more than 100% for the size of the wearer, (113, 213) to the left and right fixed portions (112) (in the horizontal direction) at a magnification of 80% or more and 100% or less. As a result, during sleep, the left and right attachment portions (111, 211) are maintained in a state of pulling the skin of the face in the diagonal direction or the head direction. Therefore, the facial skin will pull up and maintain its shape, coupled with the increase in moisture retention capacity, promote metabolism, correct the face becomes beautiful.

In this embodiment, the left and right attaching portions (111, 211) are slightly reduced from the upper portion to the lower portion (vertical direction) at a magnification of not less than 90% and not more than 100% for the size of the wearer, And is reduced at a magnification of 80% or more and 100% or less from the central opening to the left and right fixed portions (112) (horizontal direction). As a result, during sleep, the chin (114, 214) will support the jaw to prevent the chin from opening naturally, to induce nasal breathing, to prevent the occurrence of molars, snoring symptoms and xerostomia, to help enter the sleeping state.

In the present embodiment, the support jaw portions (114, 214) are provided at the lower central portion of the left and right symmetrical left and right attaching portions (111, 211). Such a structure functions as a "position characteristic portion" from the viewpoint of vision, position, distance, and direction when the SMARTMASK is worn. (10, 20) can be easily worn as long as the jaw is aligned with the chin (114, 214).

In the present embodiment, the left and right upper / lower lanyard materials (121, 122, 221, 222) are connectable with the left side lanyard material (121L, 122L) and the right side lanyard material (122L, 122R) Paste each other. As a result, just a simple banding, you can let
The right and left upper / lower lanyard materials (121, 122, 221, 222) are attached to each other so that the SMARTMASK (10, 20) can be quickly worn or removed.

In the present embodiment, the left and right upper / lower lanyard materials (121, 122, 221, 222) may have stretchability similar to that of the left and right attachment portions (11 1,211). As a result, the left and right attaching portions (111,211) and the left and right upper / lower lanyard materials hold the tension force while stretching, so that the pressing portions do not concentrate on one point, and the stress is evenly dispersed, To make sense of oppression. Therefore, it is possible to wear the SMARTMASK (10, 20) for a long period of time without wearing a pressing feeling.

In the present embodiment, the inner space (133d) of the upper portion (133, 233) of the lid portion is formed in accordance with the adhesion site (137), the cushion material (136), the wall body (131) The shielding portion (131a) and the filtering portion (139) are formed in a small size that is almost the same as the size of the nose in order to put only the nose. (133d) of the upper portion (133, 233) of the small-sized cover portion when the user breathes, and the internal gas space (133d) of the user's nose is located only inside the upper portion of the lid portion), The inhale can comfortably breathe the clean air of the external space, exhaled rapidly to the external space to produce carbon dioxide in the human body, to prevent anatomical dead space, can be beneficial to health effects.

In the present embodiment, the inner space (133d) of the upper portion (133, 233) of the lid portion is formed in accordance with the adhesion site (137), the cushion material (136), the wall body (131) The shielding portion (131a) and the filtering portion (139) are formed in a small size that is almost the same as the size of the nose in order to put only the nose. In this structure, only the user's nose is located in the upper portion of the lid portion and / or the inside air space (133d). When the user is breathing, the inside of the lid portion (133, 233) In the heating / humidification of the air flow into the upper respiratory tract, nasal cavity and respiratory tract area to maintain a proper temperature rise of the state. Therefore, it will feel easy to breathe, and ease the symptoms of inflammation in the mouth.

In the present embodiment, the front cover (134, 234) and the left and right adhesive parts (111, 211) are not integrated, and the mouth and the chin are covered with the mouth and jaw being separated by a specific distance. On the other hand, in contrast to this, a structure which is bent and opened from the upper part can be used. As a result, the mouth can be free to move up, the mouth of the hot air can also be quickly to the external environment. Therefore, the user does not feel that the cheeks, the mouth, and the chin feel damp or uncomfortable, and the user is free to talk

The configuration of the SMARTMASK (10, 20) is at least stuck to the wearer's face at the time of wearing, and is formed into a shape when unfolded, flattened when folded, and reduced in size when refolded Carry in your pocket. In addition, the SMARTMASK (10, 20) does not fold wrinkle. Therefore, the SMARTMASK (10, 20) can maintain its original shape and function even when it is stored and / or reused after being carried.

The SMARTMASK (10, 20) is worn on the face skin in the manner described below. First, the user holds the grip of the left and right upper lanyard materials (121, 221) with both hands (first stage). The pront chin is aligned with the support jaw portion (114, 214) of the "positional feature" (second stage). Then, the left and right upper lanyard materials (121, 221) with sufficient availability to the wrap head are pulled up and fixed to the user's head area (210) (third stage). Next, the left and right lower lanyard materials (122, 222) are secured to the user'ss posterior brain site (220) (fourth stage). As a result, the SMARTMASK (10, 20) has been quickly and easily worn on the user's face.

### [1] First embodiment

### [1.1] Left and right parts of the paste

During the sleep period and during exercise, a SMARTMASK (10) is worn on the human facial skin. The right and left attaching portions of the main structure of the SMARTMASK (10) need to be repeatedly stretched and contracted with the user's action as long as they are attached to the face skin. When the left and right attachment portions 111 have a limited elasticity and poor skin conformability, the left and right attachment portions 111 cannot follow the skin's elongation and contraction smoothly, and the skin may be irritated. The present invention provides a left and right attaching portion (111) having improved skin conformability that can be maintained over time. The left and right adhesive portions (111) can maintain an improved skin comfort for a long period of time. The above-mentioned "time" means that, in general, the left and right attaching portions (111) can be worn for 10 minutes or more, and not more than 10 days, irrespective of the surrounding environment.

The right and left attaching portions 111 are composed of left and right attaching surfaces 111c, left and right upper / lower profile portions 111a and 111b, an opening 113, a living hinge 111, (119), a support jaw portion (114), a left and right lug (128), and a bezel (113a, 114a). The right and left attaching portions 111 are symmetrical about the center of the opening 113 and the supporting chin portion 114 from the top to the bottom and the left and right sides. In the present embodiment, the left and right sides have a substantially identical shape. As shown in Fig. 16, the left and right attaching portions (111) are worn on the whole face skin including the left and right sides of the neck, the left and right carotid arteries, and the left and right ears except for the left and right eyes, forehead and hair.

The left and right adhesive portions (111) have skin conformability and softness and provides a medial side surface which is non-irritating and has no side effects for the user's skin. Incidentally, the left and right sticking portions (111) have hydrophobicity, and the liquid or water cannot be absorbed or passed, and the harmful substances cannot be contaminated or penetrated. Moreover, even if there is moisture on the surface, as long as gently shake can shake to remove, help users often maintain a soft state, giving a comfortable feeling.

In view of the manufacturing convenience and the mass production of the left and right adhesive portions (111), the mold should be used for manufacturing. The left and right attaching portions and the right and left fixing portions (112) will be integrated. To this end, the mold is manufactured using a mold in which the left and right attaching portions (111) and the left and right fixing portions (112) are integrated. The advantage of using molds for manufacturing is ease of manufacture, stability, accuracy and mass production, and a high degree of control over structural and aesthetic design.

This manufacturing method can be produced in the transverse direction of the SMARTMASK (10, 20) according to the invention in the range of 0.1 mm to 900 mm. May be produced in the longitudinal direction thereof in the range of 0.1 mm to 900 mm. Can be produced in a thickness range of 10 to 500 m. For example, in the case of the left and right adhesive portions (111) manufactured by the mold, the weight is reduced and the feeling of wearing is improved, and light weight manufacturing is required. Regardless of size, a good weight should be in the range of 10 to 100 g, more preferably in the range of 30 to 70 g.

The left and right adhesive portions (111) are manufactured by using a mold using a silicon material. Therefore, it is possible to form different double or multiple thicknesses. The reason for using such a different thickness structure is that the frame of the left and right adhesive portions (111) having different thicknesses has different functions depending on its thickness. That is, the left and right attaching portions 111 are formed of a thin adhered surface 111c and an opening frame 113a which is relatively thicker than the adhesive surface 111 c, a chin support portion frame 114a, a living hinge 111 a, (119), and left and right upper / lower profile portions (11a, 11b). As a result, the elasticity and smoothness are varied according to the thickness, and the SMARTMASK (10) does not easily break when worn. Moreover, improved skin comfort can be maintained over time.

As shown in Fig. 1, the left and right upper lacing materials (121) are held by both hands in order to ensure the integration of the left and right attaching portions (111) and the facial skin from time to time. , The three-dimensional shape can be maintained. As shown in Fig. 16, when the SMARTMASK (10) is worn, the ergonomic design is deformed according to the three-dimensional shape of the face. On the contrary, do not wear wisdom
When the mask (10) can be folded, it can be folded freely (folded), folded several times, and then carried in a pocket or a cosmetic bag.

FIG. 20 is a view for explaining the size of the left and right adhesive portions.

When the left and right attaching portions (111) are manufactured as described above with reference to Fig. 20, the average face size and the average face length of the user are taken into account. (113b) to the left and right fixed portion upper boundary points (11If), and from about 70 mm to about 140 mm (about 70 mm to about 150 mm) from the upper edge (113b) of the open frame to the chin support surface (114d) (11Id) to the left and right fixed portion lower boundary points (111e), from about 30 mm to about 140 mm (from about 30 mm to about 140 mm) from about 20 mm to about 50 mm from the chin support surface (114d) to the lower profile boundary point, From about 70 mm to 140 mm from the left upper fixed portion upper boundary point (111f) to the left and right fixed portion lower boundary points (111e).

As described above, the left and right contour portions 111a and 111 b pass through the upper end edge 113b of the opening to the upper boundary point of the left and right fixed portions, and the size of the user is 80% or more 100% under the contraction rate, tension work, you can achieve close to the face size (100%) of the zoom rate, and can also be extended to 120% of face size. Of course, since the right and left contour portions (11a, 11bb) have excellent stretchability, it is possible to stretch more greatly.

SMARTMASK (10) is formed on the bottom surface (114d) of the lower jaw support portion via the upper end (113b) of the opening frame, and a shrinkage rate of not less than 90% and not more than 100% of the size of the user is formed. , It should be close to human body size (100%) of the zoom rate. This length is almost the same as when wearing or not wearing a SMARTMASK. Of course, since the right and left attaching portions (111) have excellent stretchability, it is possible to stretch more naturally.

In the present invention, the reason for forming the different shrinkage magnification in the vertical and horizontal directions is that when the SMARTMASK (10) is worn, in order to allow the left and right attachment portions (111) to extend in the direction of the head, long. As a result, the left and right attaching portions (111) and the facial skin become a mechanism, and the facial skin can be pulled from the bottom up. That is, the cheek, chin, and temple areas of the user are integrated, and a certain amount of pressure is applied as compared with the other parts. Therefore, the chin can be natural support, and to the direction of the arrow pulling the skin of the face. As a result, it is possible to reliably support the substantial part by retrograde gravity, whether under normal condition or during sleep, to prevent oral respiration and to convert to nasal breathing, and to support and correct the skin stably.

The inventors of the present invention have confirmed that, when the SMARTMASK (10) of the present invention is worn during sleep, it has an effect of correcting the skin, improving skin elasticity and maintaining a clean and healthy skin. This is because the left and right attaching portions (111) having the above-mentioned structure maintain the balance of the bones and muscles, reduce the load on the muscles and the fat, prevent the skin from drying, improve the moisturizing power, and improve the muscle tissue and adipose tissue from the upper part Of the resilience and metabolism.

Further, the muscle tissue, blood vessel tissue, and lymphoid tissue are present on the face, but the left and right attachment portions 111 of the present invention do not unnecessarily unnecessarily severely compress the face. Therefore, the wearing comfort can be ensured without damaging bone or muscle, vascular tissue, lymphoid tissue, or nerve tissue.

(113a) around the left and right adhesive surfaces (111c) capable of maintaining an improved skin conformability and an improved stretchability, and a chin support portion frame (113a) which is capable of sustaining the passage of time, 114a), the living hinges (119), and the left / right upper / lower contour portions (111a, 111b) are formed to have a thicker thickness and constitute the left and right sticking portions (111). Here, the left and right fixing portions 112 and the left and right upper and lower fixing members 121 and 122 cooperate with each other or assist each other to pull and fix the left and right attaching portions 111 to the upper portion. As a result, the left and right attaching portions (111) exhibit skin comfort and stretchability, and can function as a mechanism for integration with the facial skin. This special feature, unlike conventional masks, will provide a comfortable feel during sleep. Further, the biological signal measuring apparatus 200 can be stuck to a desired position without affecting the skin of the face even if the following biological signal measuring apparatus 200 and various apparatuses are attached.

The left and right adhesive surfaces (111c) according to the preferred embodiment of the present invention should have ultraviolet, wind and liquid impermeability (water dispersibility) together with gas permeability (moisture permeability). In addition, it is desirable to have, in addition to the specified portion, a thick, good thickness of 100 or less, preferably 10 to 50 thick, and more preferably 20 to 40 thick. As a result, it is possible to stretch 700% to 1000% while increasing the soft touch. In particular, it is possible to obtain air circulation to the skin, that is, air permeability, to eliminate the fear of unnecessarily hindering the skin from breathing. Thickness less than 10 µm, because it is too thin and difficult to manage, will easily damage the thickness of more than 50 µm, it will affect the air permeability, moisture permeability, flexibility, not only difficult to obtain high moisture permeability, not smooth To follow the skin's elongation and contraction. Therefore, the skin will increase the discomfort. This may result
Including skin rash, including a variety of stimuli.

In order to provide the following advantages, it is important that the thickness of the left and right adhesive surfaces (111c) be 20 to 40 thick.
- Increased air permeability.
- Small forces can also increase elongation.
- Increase the distortion of the three-dimensional shape.
- Improved skin comfort that can be maintained over time.
- Rapid temperature change of the skin is formed according to the ambient temperature change.
- Increase sweat discharge..
- Improve touch.
- Reduce mechanical irritation.
- Reduce weight.
- Easy to wear and carry.

Here, the left and right adhesive surfaces (111c) should be capable of being responded to only by the pressure of a human finger and capable of extending in all directions (360 °) regardless of directivity. That is, when the left and right adhesive surfaces (111c) are pulled with a single finger with a small force, 500 to 700% elongation should be obtained without causing a significant structural damage. It is preferred to be able to flexibly elongate from 700% to 1000%. Also, the release pressure will quickly return to its original position. The left and right adhesive surfaces (111c) are integrated with the skin of the face covered with the left and right adhesive surfaces (111 c) to support or lift the skin of the face as described above, and are worn on the facial skin while maintaining the orthopedic state.

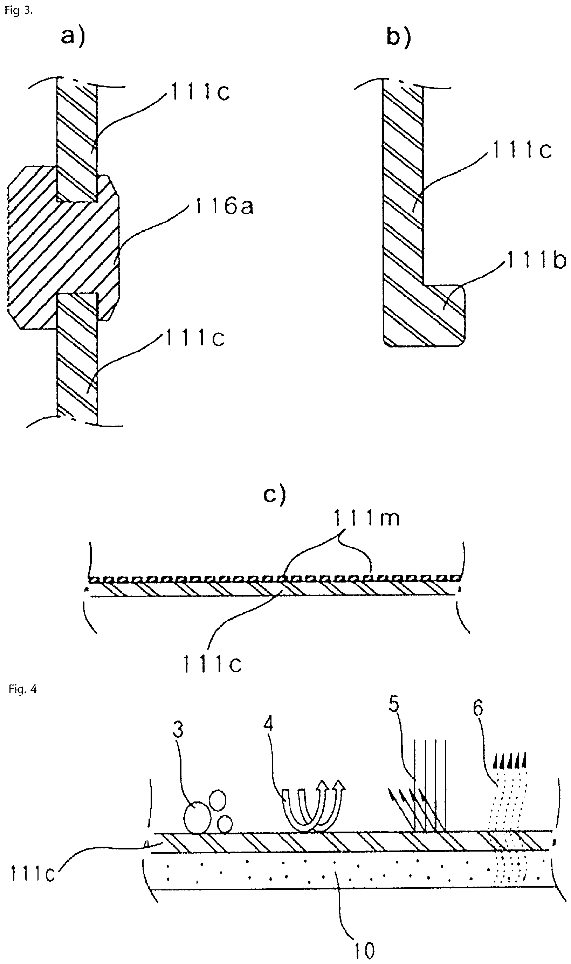

FIG. 4 is a schematic view of moisture permeation and windproofing of left and right adhesive surfaces according to a preferred embodiment of the present invention.

the left and right adhesive surfaces (111c) according to the preferred embodiment of the present invention are located on the upper portion of the facial skin layer (10), and the left and right adhesive faces prevent the water molecules (3), ) And the ultraviolet ray (5), and the moisture (sweat) emitted from the skin layer (10) is passed through the left and right adhesive surfaces (111c) in the form of water vapor (6). This is because the left and right adhesive surfaces (111c) are made of silica gel according to a preferred embodiment of the present invention and can be produced by water vapor generated from the body, that is, water vapor having a diameter of about 0.0004 thick, Water-based: water splash nature) and specificity, cannot achieve the penetration of ultraviolet and water, can provide proper maintenance of body temperature and bacteria cannot penetrate the anti-bacterial (Anti-vims). In particular, the unique properties of heat resistance, even in the 250 □ environment placed 4 days, but also to maintain 10% within the elongation changes and skin comfort. In addition, in the 450 □ environment, it will not lose its elasticity

The left and right adhesive faces (111c) according to a preferred embodiment of the present invention should be made of silica gel having a hardness (Shore A) of 20 to 30, a thickness of 20, a tensile strength (MPa) of 7.5 or more, a tear strength (N / mm) 14 or more, an air permeability of 0.2 to 0.8 cc / cm 7sec or more, a water pressure resistance of 7,000 to 12,000 mmH2O or more, an elongation (%) of 700 to 1000, a moisture permeability of 700 to 10,000 g / Degrees 2 to 10 m2Pa / w. The above-mentioned moisture resistance degree represents the degree of resistance of the team to perspiration or water vapor, and more accurately indicates the comfort parameter of the wearing feeling compared with the moisture permeability. The lower the parameter is, the higher the comfort is, the moisture permeability is 10 m Pa / W or less, will provide comfort. At the same time, all parts of the SMARTMASK (10) are not required to have air penetration. In particular, the upper portion (133) of the lid portion is preferably made of air impermeability.

The following embodiments are described in more detail.

### Examined Example 1

The above-described synthesis of silicon is performed. First, in order to combine the properties of the liquid silica gel, various additives were prepared and the composition was prepared. In this case, the elasticity and the stretchability of the formed product are maintained, and the softness is maintained. The appropriate hardness (Shore A) is in the range of 20 to 20, resulting in a rubbery state or a gel state. The milling machine was sufficiently prepared with a milling machine to face the twin drum, and the composition was spread over a wide range of rolls on one side by rotating the drum face-to-face with a prescribed distance. As the composition is repeatedly milled between two rollers, the mixing of silicon becomes uniform and the degree of bonding becomes stronger.

After the synthesis operation is completed, the final composition spread over a wide range on one side of the drum is cut to a certain size and thickness, and the mold is ready for operation. After the upper mold is opened to the upper side, the composition cut in the appropriate thickness and size after the synthesis is stacked on the lower mold. While the upper die is grooved, compression is performed for a predetermined period of time by pressing the pressure and temperature of the other devices. The composition corresponds to the shape of the upper / lower mold, and the excess composition is extruded out of the upper / lower mold frame, leaving only the desired shape on the mold. In this manner, a thin surface having a tensile strength (MPa) of 7.5 or more, a tear strength (N / mm) of 14 or more, an elongation (%) of 700 to 1000,

### Examined Example 2

A composition having the same composition as that of Experimental Example 1 was used to produce a thin surface having a thickness of 40 by the same manufacturing method.

### Comparative Example 1

The same composition as that of Experimental Example 1 was used to produce a thin layer having a thickness of 120 by the same manufacturing method.

### * Experimental method

### 1. Air Permeability - JIS L1096 A (Frazier method) - TEST area 10 cm2, pressure 125 Pa

### 2. Water pressure resistance -JIS L1092B / KS K 0592 (high water pressure method)

It is possible to determine the degree to which the water level of the fabric can be made impermeable by measuring the water content of the water drops on the surface of the fabric by applying pressure to the surface of the fabric using water resistance of the fabric to water leakage or water immersion. Can be used to understand the concept of the opposite of moisture permeability. The thicker the water-resistance parameter is, the higher the parameter is required for functionality, moisture permeability, and water-resistance.

### [332] 3. Permeability - JIS L 1099 A 1 (calcium chloride method)

The amount of moisture passing through the area of 1 cm2 over 24 hours was measured. After the initial weight was measured and placed in a temperature-resistant humidity chamber (24 °C, 65%), the weight was measured 24 hours later to confirm the amount of moisture loss.

### 4. Resistance to evaporative transmission (RET) -ISO 11092 / ASTM E96

Permeability and moisture permeability were measured by the difference in water vapor pressure under wet conditions. The upper part of the test specimen was 100% saturated with water vapor, and 40% of the water vapor was vaporized by the false sweat state. When the water vapor moved from the upper part (the saturated state) to the lower part, the lower part was heated, Of the amount of water vapor, will start power supply. The larger the power, the better the moisture permeation effect (the more water vapor moves), which is the result parameter of the heat supplied per unit area, and the lower the parameter, the better the performance. In general, when the parameter of the moisture permeation resistance is lower than 10, it is judged that the moisture permeability is good.

### 5. Measure thermal imaging

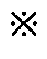 Shooting conditions

- Room temperature: 270C
- City humidity: 68% RH
- Experimental equipment: artificial climate chamber, ultraviolet thermal imaging camera, skin temperature and humidity measuring instrument (sensor 8X2 units)
   (1) Wear a SMARTMASK (10).
   (2) Take a picture just after entering the artificial climate chamber and measure the skin and the SMARTMASK (10).
   (3) After entering the room, in the artificial climate chamber motionless, ten minutes after the photo, and then measure the skin and smart mask (10) temperature and humidity.
   (4) Exercise for 10 minutes.
   (5) Take the picture 5 minutes after the end of the exercise, and measure the temperature and humidity of the skin and the smart mask (10).

Table 1

**[Table 1]**

| No. | Classification | Unit | Example 1 | Example 2 | Example 3 |
|---|---|---|---|---|---|
| 1 | Air Penetration | cc/cm²/sec | 0.5 | 0.15 | 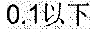 |
| 2 | Water Pressure Resistance | MmH2O | 9,000 | 10,000 | 15,000 |
| 3 | Moisture Permeability | g/m²/24hr | 3,000 | 1,000 | 300 |
| 4 | Permeability Resistance | m²Pa/w | 6.20 | 9.80 | 40.2 |

As the data appearing in the above Table 1, the left and right adhesive surfaces (111c) according to the present invention exhibit more excellent characteristics as compared with Comparative Example 1. As shown in Fig.

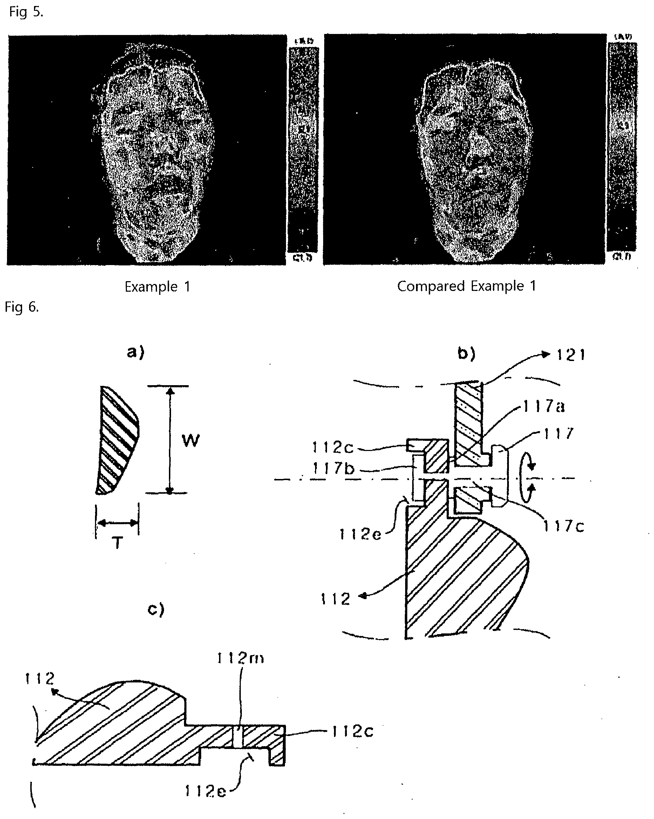

Fig. 5 is a thermal imaging measurement result after wearing a smart mask according to an embodiment of the present invention.

Fig. 5 is a graph showing the results of thermal imaging after wearing the smart mask (10) using the left and right adhesive surfaces (111c) of the first embodiment according to an embodiment of the present invention. 5, in order to wear the smart mask (10) of the present invention during sleep or during exercise, it was found that after the thermal imaging, compared with Comparative Example 1, the significant decrease in the red spot resulted in the post-exercise sweat becoming water Steam quickly discharged from the body, so that the wearer to maintain a comfortable state, the appropriate adjustment of the face of the heat, excellent show the skin comfort.

In the present invention, the mold surface and the die surface of the corresponding adhesive surface (111c) can be smoothed by the use of a mold and a lapping technique during the production of the left and right adhesive portions (111). When the mold surface becomes smooth in this way, the left and right adhesive surfaces (111c) also become smooth. As a result, the adhesive force of the left and right adhesive surfaces (111c) increases. In addition, will increase the phenomenon of paste with the skin. That is, the silicon surface used in the present invention becomes brighter, and the phenomenon of sticking to the skin is increased. Therefore, the inner side surface providing the skin comfort becomes uniform, not only improving the touch, but also the left and right adhesive surfaces (111c) Of the adhesive force. Therefore, stimulation due to the flow of the left and right adhesive surfaces and the skin (111c) can be prevented, thereby improving the moisturizing power and promoting metabolism. As will be described later, various advantages such as prevention of air flow by the internal gas space (113d), improvement of the sealing force, and the like are provided.

In addition, although the polishing side is not particularly limited, the total area of the left and right adhesive surfaces (111c) should be in the range of about 10 to 50%. It is difficult to obtain a sufficient sealing force if the area ratio of the polished side is less than 10%, and it is difficult to obtain the desired passage of time of the present invention and maintain the improved skin conformability).

Therefore, only the upper portion of the left and right adhesive faces (111c) may be polished, or only one of the upper and lower portions may be polished. In this case, it is preferable that the edges of the left and right adhesive surfaces (111c) are polished (that is, the upper / lower outline portions (111a, 111b)). This will ensure that the portion does not bulge.

### Figure 3c) is a cross-sectional view from B-B 'from Fig.1

Figure 3c), the left and right adhesive surfaces (111c) of the present invention include a concavo-convex surface on at least one side of the inner surface in contact with the skin. In this case, the uneven surface includes the adhesive surface (111m) and the non-adhesive surface (the portion not bonded to the skin). More specifically, it is a combination of the very small adhesive surface (111m) and the non-adhesive surface. That is, a circle, a quadrangular shape, a polygonal shape, or a combination of these adhesive faces (111m). Fig. 3 (c) shows an example in which the adhesive surface (111m) has a rectangular shape. As a result, the phenomenon of facial skin sticking can be reduced. Meanwhile, the pitch and the thickness between the adhesive surfaces (111m) are adjusted to various sizes and thicknesses depending on the purpose of use.

Figures 1 and 2, the following biological signal measuring devices are attached to the right and left attaching portions (111) and left and right ear portions, left and right ear portions, and right and left carotid arteries, left and right ear portions, and the like, respectively, as shown in Fig. 1 and Fig. A plurality of bonding grooves. The bonding (116) has an inner diameter of about 5 to 10 mm, an outer diameter of about 15 to 20 mm, and a thickness of about 1 to 1.5 mm. The ring (116) has a hole (opening) ) Into one. The diameter and the thickness of the bonding (116) may be appropriately designed as required. The bonding is a circular which is thicker than the left and right adhesive surfaces (111c), because the biosignal measuring device (200) is somewhat more in weight, even when the biological signal measuring device (200 (200) and the skin of the face can be stuck to the thickness of the biological signal measuring device (200) so as to accurately measure the biological signal to prevent the occurrence of such a phenomenon.

The bonding (116) serves to hold the biological signal measuring device (200) tightly at a desired position such as the carotid artery and the ear when the biological signal measuring device (200) is attached, When the biological signal measuring apparatus (200) is not used, it functions as a general adhesive surface. That is, a detachable cover (116a) is provided in each of the plurality of engaging grooves (116) to which the biological signal measuring apparatus (200) is not attached. The lid (116a) has recesses in the middle portion thereof to form an annular lid for assisting the lid and the engagement (116) when the lid (116a) is sandwiched in the joint (116) Are joined together to completely seal the engagement (116). At this time, the diameter of the cap should be slightly larger than the diameter of the engaging (116). This is because, even if the lid is larger than the engaging 116, the bonding 116 can be enlarged by pressing and affixing, and can be inserted in a detachable manner in a soft and comfortable manner. 3 (a) of the cover (116a) of the cover (116a) inserted into the engaging (116), that is, the face contacting the wearer's skin, should be in contact with the left and right adhesive faces (111c) Of the inner side of the same inner surface composition.

According to an embodiment of the present invention, the coupling (116) can be incorporated not only with the biological signal measuring device (200) but also with various information communication devices or instruments. For example, a combination of a skin measuring device for helping to measure the skin, a skin stimulating device and an ultrasonic device for stimulating the skin, an earphone-type device for hearing or talking, a measuring device for measuring ultraviolet and ozone, etc., Information and convenience.

Figs. 1 to 2, the left and right attaching portions (111) may include adjacent ones of the continuous 360 ° extended wearing smart masks, the skin immediately under the eyes of the user, and the left and right temples The left and right upper contours (111a) and the neck skin surrounding the chin of the user are arranged on the left and right lower contours (111b) or the contours (111, b) of the carotid artery.

The left and right upper contours (11a) of the left and right temples, which are arranged directly below the eyes of the user, are different from the conventional mask in that, as shown in Figs. 15 to 16, the above-mentioned one end constitutes an opening frame (112a) of the left and right fixed portions (112a) are integrally connected to each other, and the other end is gradually increased in height in order to be moved from one end to the other end. The term "end", as used in this list, does not directly imply the end of the structure, for example, with reference to "the front end " or "the north end of the Nanshan", meaning the approximate area.

Conventional masks have the disadvantage of blocking the user & apos; s field of vision. According to the present invention, when the pulling force of the left and right upper contours (11 a) is activated, the left and right upper contour portions (111a) are pulled in too much direction in the upper direction, thereby affecting not only the visual field but also aesthetic problems. In the present invention, in order to prevent an excessive upward movement of the upper contour portion (111a), the eyes of the user are shielded, and the skin immediately under the eyes of the user is supported in the upper direction by a similar manner and function as the brass wire, The face is directly below the skin to seal the positioning, followed by around the temple, to maintain the state of paste configuration. Therefore, do not cover the eyes. At the same time, close the cover at the same time cover the eyes and the skin is just below the corner of the skin, left and right temple area, not only from the UV and harmful substances in the bottom of the skin and eye protection skin, left and right temple, but also to maintain sustained moisturizing effect, Promote metabolism, can also simply achieve the skin sagging or corneal wrinkle correction.

At this time, in particular, the right and left upper contour portions (111a) are formed with elbow-shaped bent portions (111d) on one side as shown in FIGS. 15 to 16. When wearing a smart mask, the elbow-shaped bend (111d) is disposed around the skin of the user's eye and is selectively arranged at the corner of the eye skin, which functions as anatomical standard, correcting between the eye immediately below and the midpoint of the temple Or protects the skin of the eye corner, and protects the lower surface of the lower eye from being adhered to the left and right adhesive surfaces (111c) of the upper temple. The distance between the skin and the temple below the eye is about 25mm vertically. Therefore, the size of the ideal elbow bend (111d) is about 15 mm to 40 mm.

The wrinkles of the skin of the corner of the eye covered by the elbow-shaped bent portion (111d) are in an unfolded state when the elbow-shaped bent portion (111d) adheres to the skin, and the wrinkles of the portion are stuck in the developed form, So that its morphology can be maintained. Therefore, after the smart mask (10) is removed from the face, the wrinkles become inconspicuous, and the state of the wrinkles can be ensured.

The elbow-shaped bend (111d), as described above, can be used to effectively measure biological signals or shield ultraviolet rays from the source, by, for example, wearing a smart mask on the face during or during sleep. Therefore, the ultraviolet shielding and sagging or wrinkle correction of the basic functions of the skin around the eye can be easily achieved at the same time.

As shown in Fig. 29, the following electroencephalogram electrode (550a) is preferably placed in contact with the skin in such a manner that the skin is moved upward by 25 mm + 5 mm from the skin directly under the eyes and then moved toward the rear side head by 20 mm + 5 mm. The elbow-shaped bend (111d) may be an acute or curved portion that guarantees this relative displacement provided from the anatomical criteria provided by the eye.

As shown in FIG. 29, the carotid artery, which is located in the user, surrounds the chin and is disposed in the chin (111b) of the immediately-lower neck skin is intended to be connected to the bottom surface of the chin supporting portion (114), and the other end is integrally connected to the lower portion (112b) of the left and right fixing portions (112). Structurally, as you move from one end to the other, it gets higher.

29 and 30, when the smart mask (10) is worn, it is maintained around the skin of the carotid artery of the user, and in the case of the selective carotid artery (11), the right and left lower contours (11bb) (111c) on the upper surface of the skin on which the carotid artery is located, and an adhesive layer (111c) is provided on the upper surface of the skin where the carotid artery is located. The position of the carotid artery is shown in Fig. 29, and is the same as the position of the drawing number 500L.

Regarding the carotid artery, as shown in Fig. 28, the following electrocardiographic electrodes (500L) are in ideal contact with the skin. The left and right lower contours may extend from the lower portion of the anatomical standard provided through the mandible by 10 mm to 25 mm. Then, will be located in the peripheral region of the carotid artery (205), optionally in contact with this region. Such a part of the mandible functions as an effective anatomical standard, making it possible to set the repetitive position of the electrocardiographic electrode of the carotid artery, thereby securing intimate contact between the carotid artery (205) and the electrode.

It is important to measure the biosignals in these locations because carotid arteries provide the following advantages to biological signal measurements.
- The position of the carotid artery is stable, so that the location of the carotid artery does not change.

The carotid artery can always maintain a certain positional relationship with the heart and therefore has the advantage of good reproducibility of the measured value. In addition, the example, continuous measurement of the carotid artery, you can find a variety of brain diseases, but also to predict the time of onset later.

The left and right lower profile portions (111b) surround the left and right chin of the user so as to cover the skin of the neck immediately below the chin, and cover the neck skin immediately below the right and left ears where the carotid artery is located, Configure the state of the paste. As a result, the area of the left and right adhesive surfaces (111c) can be unnecessarily enlarged, and the stretchability can be reduced and the wearing feeling can be reduced, while the entire neck skin is stuck.

Therefore, there is an advantage that the chin skin can be affixed to the carotid artery and can be lifted upwardly and downwardly. For example, during the night or during exercise, the mask can effectively measure the biological signal, but also to maintain the role of moisture, by promoting the new generation and correcting sagging chin skin to make it look good.

The right and left contour portions (111a and 111b) have a thicker thickness than the left and right adhesive faces (111c). However, the right and left contour portions (111a and 111b) are formed with a small force, It is necessary for the user's face shape with adequate scalability and adhesion force. As a result, the skin of the user's face is seamlessly adhered to the face curve as a whole, and the essential part of the skin of the face, which is the same around the mouth, the carotid artery, the neck skin and the left and right temples, becomes Integration, correct wrinkles or sagging skin becomes beautiful. Not only that, but also can not be allowed to give a little air sealing surface function. In addition, when the biological signal measuring device (200) described below is combined with the left and right engaging grooves 116, it is possible to prevent a sudden change in the distance between a reasonable position due to the actions of various kinds of users and the biological signal measuring device (200) so that the right and left carotid arteries and the left and right temples and the biological signal measuring device (200) are properly fixed in place to maintain close contact.

As shown in Fig. 3 (b), the right and left contour portions (111a, 11bb) should be formed in the left and right adhesive surfaces (111c) so as to have a function to prevent or damage the left and right adhesive surfaces ) is 10 to 150 m thick, and should be substantially 50 to 140mm thick in thickness. In addition, the width thereof should be in the range of 1 to 10 mm. According to the degree of expansion and contraction of the left and right contour portions (111a, 11bb), the thickness and the width can be uniformed or a gap may be provided between the left and right profile portions (11a, 11b), and various modifications may be made without limitation.

The left and right adhesive portions (111) may be formed in a transparent or translucent form. In the present invention, the right and left attaching portions (111) may be attached to the user's temple from the upper portion, and may be attached to the user's carotid artery from the lower portion to cover the user's face if the left and right adhesive portions (111) are made in an opaque pattern So that the face of the user of the smart mask (10) cannot visually be recognized by others. It is difficult for the user to visually recognize the user of the smart mask (10) when the user wears the smart mask composed of the opaque left and right adhesive portions (111) of the present invention. Such a smart mask (10) for covering the face is highly likely to be used for various crimes because of its excellent wearing feeling and concealing power, and it is not possible to distinguish between sleep and work due to its opacity The user of the smart mask (10) may be annoyed by the surrounding person. Therefore, the left and right adhesive portions (111) and the left and right adhesive faces (111c) should be formed in a transparent or translucent manner to allow the user to visually recognize the user of the smart mask (10).

The left and right adhesive portions (111) of the present invention may have an ultraviolet shielding function. The left and right adhesive portions (111) may be added or printed with ultraviolet light absorbers in order to protect the face skin of the wearer from the UV rays of the outer side face. The right and left attaching portions (111) are composed of a mixture of ultraviolet shielding materials in silicon in the adhesive, and various colors and patterns are printed in order to impart a sense of aesthetics by using a general water transfer method, a thermal transfer method and various transfer methods, Scope, design and production. The ultraviolet shielding material has a particle size in the range of 0.1 to 20, and may be one or more ultraviolet shielding materials and the same substance or an analogue thereof depending on the kind of the ultraviolet shielding material. The type of the UV absorber is not particularly limited, and may be organic or inorganic. For example, an organic ultraviolet ray shielding agent such as glyceryl benzoic acid and cresotriazole and an ultraviolet ray shielding agent such as titanium oxide and zinc oxide (zinc white) may be used. In any case, the ultraviolet-shielding substance does not irritate the user's skin in any case.

As shown in Fig. 1, the right and left attaching portions (111) include left and right ear portions (128) that cover the left and right ears of the user on the left and right side surfaces. The left and right ear portions (128) serve to protect the user's ears from ultraviolet rays and harmful substances. The left and right ears (128) should be formed integrally with the left and right adhesive faces (111c) in the same material and thickness to provide sufficient stretchability. Further, in order to completely cover the user's ear, it is preferable to make it slightly larger than the left and right ears of the user.

As shown in Fig. 1, when the smart mask (10) is not used as shown in Fig. 1, the left and right ears (128) are arranged in such a position that the left and right ear portions (112) (128) maintains a protruding shape. On the other hand, in the case of wearing the smart mask (10), as shown in Figs. 16, 19, and 41, the left and right ears (128) are pulled, and the protruding shape disappears and is maintained on the left and right ears of the user The state of being stuck comfortably.

That is, since the left and right ears protrude from the face structure of the user, a high wearing stress may occur, and the height of the left and right ear portions (128) may be lowered at the same time as the wearing stress. Comfortable to wear stress. Therefore, it is possible to comfortably cover the left and right ears while minimizing the wearing feeling, and also has the advantage of protecting the left and right ears of the user from the external environment. At the same time, there is an advantage that the biological signal measuring device (200) can be brought into close contact with the ear with a small stress when the biological signal measuring device (200) is worn on the ear.

The left and right ears (128) may be provided with the same engaging (116) as the engaging (116) provided at the left and right attaching portions in a position corresponding to the ear of the user, and the left and right engaging grooves (116), and is detachably attachable (116a). The combination slot (116) can be combined with various information communication instruments, body temperature measuring devices, earphones, and the like, in addition to the biological signal measuring device (200).

FIGS. 24 to 25 are views showing a state in which the worn earpiece is worn with a wrist ear covering the wristwatch.

In recent years, with the widespread popularity of smart phones, usually in the mobile, sports, headphones are only inserted in the ears, so in general it is easy to fall out from the ears. In order to prevent the fall out of the way into the headset, when used in a long time will cause pain. In addition, because the shape and size of the ears vary from person to person, individual users will not be able to properly wear headphones. 24 (a) and 24 (b), when the earphone (701) is worn on the ear (750), the ear portion (128) is worn on the headphone, and the ear portion (128) While wearing the structure.

This is to achieve stability during wearing of the headset (701) so that the wearing of the earphone hardly affects the behavior of the user and optimizes the wearing feeling of the user. The earphone (701) can be worn while the ear (750) is gently inserted. When the earphone (701) is inserted into the ear, the headgear (701) does not fall out of the ear because the left and right ear portions (128) naturally hold the headphones while maintaining the worn state.

Figs. 25 (a) and 25 (b), a certain portion of the earpiece is allowed to protrude through the left and right ear protector (128) when worn. Here, the is provided in the head portion of the earphone so as to be joined to the engaging (116) provided in the left and right ear portions. As a result, the earphone (701) can be maintained in a state in which the ear buds (128) are fixed while the headphones are being worn. Therefore, the earphone (701) does not fall out of the ear.

When the user performs a conversation during the wearing of the image forming apparatus or when using the mobile phone, the user may be required to temporarily remove the earphone from one ear in order to listen to the external sound, and the suspended earphone may be suspended by gravity to form an obstacle in the air , But also because of its weight, so that another headset will fall off. In this case, the earphone can be removed from the ear and slightly deviated from the lower position, so that it is not necessary to completely remove the earphone, and the user does not need to take troublesome actions.

As described above, the bio-signal measuring apparatus (200) can be used in combination with the information communication apparatus, the body temperature measuring apparatus and the like, and the bio-signal can be accurately measured.

FIG. 15 is a state diagram of a user wearing the smart mask of the present invention.

Fig. 15, a jaw supporting portion (114) is provided at the center of the front surface of the left and right sticking portions (111) in the opening (113) and the lower portion of the opening (113). When the smart mouthpiece (10) is worn, the opening (113) is provided with a small pulling force applied to the front surface of the left and right attaching portions (111) so as not to be pulled from the nose and mouth or to prevent the nose and mouth And the left and right adhesive portions (111) are contacted. Other than the aforementioned opening (113), and the effect of protecting the skin can be obtained by shielding the inflow of the outside air.

When the smart mouthpiece (10) is worn, the opening (113) is formed, as shown in Fig. 15, in a suitable position and structure at the nose and mouth, for the purpose of breathing or talking. And the nose protruding forward. That is, the opening (113) protrudes integrally with the mouth and the nose without coming into contact with the mouth and nose, and the upper part forms an opening, and the left and right sides include a plate-shaped bezel (113a) Shape. Therefore, the nose and the mouth can simultaneously protrude through the opening (113), and the nose and the mouth do not contact the left and right attaching portions (111).

As shown in detail in Fig. 15, the shape of the opening frame is a right-angled quadrilateral with a predetermined size and a substantially circular shape. When the smart mask (10) is worn, in order to correspond to the length of the nose of various users, from the position immediately below the eye of the user (where the upper contour is arranged) to the philtrum (where the shield is arranged) Is substantially longer than 30 mm, shorter than 70 mm, and about 30 mm to 70 mm in length, and is folded about 7 to 12 mm in width by a cover upper portion frame (133b) and a fixing method, and has a thickness of about 50 to 150, the nose protruding through the opening (113) should be placed at the center, and the left and right sides should be arranged substantially in parallel. As a result, the opening bezel (113a) maintains the sticking state at the boundary point where the nose and the cheek of the user meet.

Next, the bezel (113a) is integrally combined with the cover upper portion frame (133b) described below in a foldable manner. When the integration process is exemplified, the left and right attachment portions (111) and the cover portions are formed on the respective other molds, and the opening frame (113a) and the upper lid frame portion (133b), After the process of combining each other, and then become integrated. Of course, the opening frame (113a) and the cover upper portion frame (133b) may be integrated by integrated manufacturing processes on the same mold. As a result, it is possible to simplify and simplify the manufacturing process by integrating the frame (113a, 133b) without the need to separately produce the laterally integrated parts (111) and the lid (130).

Since the opening mouth groove 113 is provided in the center of the mask 10 when the smart mouthpiece 10 is worn when the opening groove frame 113a and the upper lid frame 133b are integrally formed in this manner, (113) located in the center of the smart mask, the opening groove (113) remains firmly in its original shape and remains adhered at the boundary point where the mouth and nose meet. This is because the opening groove frame 113a and the upper lid frame 133b are joined together as shown in Fig. 17, and as a result, the opening groove 113 contacts the skin at the boundary point where the user's nose and mouth meet, And an upper portion of the lid portion (133) is provided with a fixing force in a combined state. Therefore, the opening space (113) having the fixing force is maintained at the boundary point where the nose and the mouth meet, and the space between the nose and the corners of the mouth is isolated. Therefore, the noses and the mouth do not come into contact with the left and right adhesive portions (111). As a result, there is no fear of contact and no pressure is applied to the noses and the mouth.

Next, the width of the lower portion of the opening groove (113) is widened, and the living hinge (119) can be added to the left and right sides. The living hinge (119) is very important in that the two side frames (113a, 113b) are closer to each other or away from each other with the living hinge (119) as a standard.

It is the intention of the inventor to protect the free movement of the chin when the smart mask (10) is worn by the living hinge (119). The best structure to protect the free movement of the chin is that the branches of the chin structure exist in the left and right sides of the mouth. On the basis of this structure, if the jaw can be designed to accommodate the jaw movement on the living hinge 119, the jaw movement can be effectively protected even without a complicated design structure.

The structure and the arrangement of the living hinge (119) are the same as those shown in Fig. 15. The relative positions, shapes, sizes, and separations of the hinges (119) are arranged so as to correspond to the corners of the user's left and right sides, and are opposed to each other, depending on the specific arrangement and arrangement of the living hinges. (113a) and the lower side frame (113b) of the two side opening grooves (113a, 113b) are selectively disposed respectively. In the configuration shown in Fig. 15, it is confirmed that the living hinges (119) are adapted to be accommodated in the jaw movement even when they are on both sides of the mouth corners of the mouth, and the living hinges (119) are worn repeatedly in the above-described areas, In the mouth above.

The living hinge (119) has a curvature of a radius ranging from about 100 to 200 and a radius of about 20 to 30 mm for the purpose of projecting the mouth, and a "(" shape is formed as a whole.) The living hinge (119) should be capable of easily elongating in the upward and downward directions according to the pressure of the human finger, that is, the force of the living hinge (119), so long as a very small pressure is applied thereto. The opening groove frame 113a and the lower frame 113b can be easily extended in the up and down direction without causing a significant structural damage by pulling the opening groove frame 113a a and the lower frame 113b in the vertical direction.

This structure of the living hinge (119), in the human body acts as the left and right corners and the same function. For example, when the user opens the mouth, the upper / lower lips are opened or closed by using the left and right corners of the mouth which can be accommodated by the elasticity accommodating mouth. At this time, the left and right living hinges 119 arranged at the right and left corners of the mouth are stretchable together with the right and left mouth corners, and the gap between the left and right living hinges 119 is received in a telescopic manner. Therefore, when the user wears a smart mask and performs a large opening or a conversation, the living hinge (119) exhibits a sufficient stretchability and a change in the mouth opening operation. In the present embodiment, the living hinge (119) also serves as a sealing member for preventing the generation of a gap in the mouth.

In general, wearing a regular mask, it will cover the nose, mouth, chin, because the jaw movement will make the mask on the face of serious movement, for the user to bring considerable trouble. This is because the mask cannot accommodate the action of the chin.

In order to improve the above-mentioned conventional problems, the left and right sticking portions (111) and the opening groove (113) are substantially separated from each other by 15 to 20 mm within a predetermined distance to form a chin supporting portion (114). In this way, the chin supporting portion (114) should be arranged at the center lower portion of the left and right attaching portions (111). When the jaw supporting portion (114) is disposed at the lower center portion of the left and right attaching portions (111) of the present invention, the jaw supporting portion (114) functions as a "positional feature portion" 111) and / or the living hinges (119) in an interaction or synergistic or downward direction.

There is a possibility that the chin support portion (114), as illustrated, interacts with or cooperates with the left and right attachment portions (111) and / or the living hinges (119). Therefore, according to the embodiment of the present invention, the chin supporting portion (114) is fixed in the center of the left and right attaching portions (111) and in the distance and direction from the living hinge (119), and has a visually non-adjustable portion Element carrier component.

Examples thereof are shown in Figs.15, 18 and 19. When the SMARTMASK (10) is worn, the front jaw of user is drawn into the chin rest (114) and protrudes forward slightly, as shown in Figs. 15, 18 and 19. Therefore, the chin rest (114) is designed to have a radius in the range of 30mm to 40mm so that the front jaw of the user can be naturally drawn, and has a fixed depth and the same thickness as the left and right contact surfaces (111c); in other words, it has a groove-like shape slightly protruding in the downward direction. The chin rest (114) further comprises a rim (114) at a boundary between the left and right contact surfaces (111c) and the chin rest (114). The upper rim (114a) has a band shape with a thickness of 50 to 200mm and a width of 3mm to 6mm. The upper rim (114a) is thicker than the left and right contact surfaces (111c) and the chin rest (114), and therefore the surplus protrudes outward. The rim (114a) stably fixes front chin in the chin rest (114). Because the rim (114a) has a relatively low elasticity compared to the chin rest (114), the front jaw is fixed by the rim (114a) when held in the chin rest (114). Accordingly, the chin rest (114) has a sufficient elasticity, naturally holds and supports the front jaw of a user. In addition, since the chin rest (114) has a shape, it provides a visual identification area on the basis of wearing the SMARTMASK (10).

The aforementioned structure allows the chin rest (114) to fix jaw against the pulling force and supports jaw with its the "location-specific" role as a chin guard. The front jaw of a user is drawn into and fixed onto the chin rest (114) so that the SMARTMASK (10) is not deflected excessively upward. Such structure allows the chin rest (114) to hold front jaw tightly to keep it from dropping unconsciously by the relaxation of oral muscles and gravity in sleep or in daily routines, and thereby prevents mouth breathing to replace it with nasal breathing.

On the other hand, particularly in cases where the user consciously opens his or her mouth, the chin rest (114) can stretch to relocate to whichever direction with respect to the living hinge (119) and/or the left and right contact portion (111) via interactions in between. Such interactions or collaborations in between the chin rest (114), the living hinge (119) and/or left and right contact portion (111) ensures a comfortable jaw movement and thus allows users to be comfortable in speaking.

As described above, the chin rest (114) is "location-specific", and is used when the SMARTMASK (10) is worn. In other words, when the SMARTMASK (10) is worn, the user brings the front jaw to the chin rest (114) and at the same time, pulls the left and right upper band (121) appropriately to fix onto the top of the head. Then, the chin rest (114) smoothly covers the jaws, while the left and right surface contact, with sufficient elasticity, come into close contact with face. At this time, to ensure that the SMARTMASK (10) is accurately and easily worn, the user is required to attach a front jaw to the chin rest (114) which serves a "location-specific" role. This ensures sustainability upon repeated uses. Hence, the chin rest (114) provides an ideal landmark for wearing SMARTMASK (10) correctly.

As a result, the variation problem faced by users without sufficient knowledge on how to use SMARTMASK (10) is addressed by providing visible "location configuration" on its center. With "location-specific" chin rest (114) that ensures correct SMARTMASK use, left and right contact surface (111c) can steadfastly be applied on the face skin or on the area of interest.

In addition, as shown in Fig. 18, a discharge (114b) is formed in the chin rest (114). A small size discharge (114b) at the center of the chin rest (114) helps discharge sweat from the face. Because the discharge is located on the bottommost part on the SMARTMASK (10), and also because SMARTMASK (10) is designed impermeable to moisture, sweat from the face are collected on the chin rest area (114), and then immediately exits out through the discharge (114b). Quick discharge through the discharge prevents any discomfort from moisture. Of course, multiple discharge grooves may be placed on the mask.

### [1.2] Left and right fasteners

As depicted in Figure 1-2, in addition to the left and right contact portion (111) with an upper contour portion (111a), a lower contour portion (111b), a left and right tight contact surface (111c), the SMARTMASK (10) further consists of extended left and right fasteners with upper (112a) and lower (112b) parts. The left and right fasteners (112) ensures the upper contour portion (111a) and lower counter portion (111b) to have a fixed distance in between, so that the mask maintains its form upon its use.

As shown in the figure 6, the upper (112a) and lower (112b) portions are designed to have greater width (W) than thickness (T) to keep a fixed distance in between so as to be opposed to the left and right contact portion (111). To be specific, it is designed to be bigger than user's ears, approximately 60mm to 140mm in length, 300um to 800um in thickness and 15mm in width, making ")" shape with a gentle curvature at the intermediate point.

The fastener (112) has a large surface area and is plate shaped, and when the left and right upper and lower bands (121, 122) are connected to pull the left and right fastener (112), the upper (112a) and lower (112b) portions pulls in the left and right fasteners in three dimensions while maintaining their shapes. The upper (112a) and the lower (112b) portions are gently bent inward in accordance with the shape of its user's temporal region, allowing tight contact with no space in between the left and right fasteners (112) and the temporal region.

As shown in the figure 22, this left and right fastener design (112), upon the SMARTMASK (10) use, allows its upper portion to be positioned on the user's temple region while its lower portion be positioned on the neck area, locating the upper and lower portions selectively and extensively up and down; the area aforementioned functions as an anatomical reference, ensures the left and right ear lids (118) be located on ears, and bio-signal measure electrode be in intimate contact with the ear.

Therefore, the left and right fasteners (112) are disposed on the left and right auricles and the left and right fasteners (112) move along as left and right upper/lower band (121, 122) are slightly elongated toward the back of the head by the pulling force, allowing left and right ear lids (128) cover both ears, and left and right fasteners be placed on the user's left and right temporal regions (behind ears). Left and right contact surface (111) tightens as it is elongated by the left and right fasteners (112), maintain its three-dimensional shape. The upper contour portion (111b) contact the temple, the lower contour portion (111b) tightly attach onto the neck right below chin and are supported with enough force to pull face skin evenly. The left and right contact portion (111) and the contour portions (111a, 111b) hold tighter to the face skin, overall allowing user's face skin and the left and right contact portion (111) to hold tightly to each other.

On the other hand, the left and right fasteners (112), to increase the adhesion in between the face contact area and the SMARTMASK, and easy attachment of the left and right upper/lower bands (121, 122) and the attachment portion (112c), where the upper and the left and right upper/lower bands are connected, is formed on the outer surface of the upper (112a) and lower (112b) portions.

Here, as shown in the figure 6b) and 6c), considering the ease of installation for the left and right upper/lower bands (121, 122), it is advised that the upper and lower attachment parts of the left and right fasteners (112c) have more extensive structures than the upper (112a) and the lower (112b) parts. Therefore, the left and right upper/lower bands (121, 122) becomes easily attached and detached from the upper and lower attachment portions (112c).

At this time, the upper and lower attachment portions (112c) are rigidly formed, and a support plate (112e) is formed so that the support plate (117b) protrudes so as not to contact the user's skin. In the center of the support plate (112e), a penetration hole (112m) is formed, so that the support plate is inserted and connected. This allows the bolt to be inserted into the penetration (112m) so that the attachment portion (112c) and the left and right upper/lower band (121, 122) are tightly coupled and can rotate with respect to each other. To manufacture the rigid upper and lower attachment portions (212c), they are first molded with hard silicon with a hardness of approximately 50 to 80 degrees, then be molded secondarily around the attachment portion (212c) where it has been previously molded with soft silicone having a hardness of approximately 10 to 30 degrees.

As described above, the left and right tight contact portions (111) are manufactured using silicon as their building material. However, any innocuous material with excellent elasticity and gas penetrability to allow breathing, and with adequate wind and moisture block, can be used.

### [1.3] Right and left upper and lower bands

As shown in the figure 2, the SMARTMASK of this invention (10), includes left and right upper/lower bands (121, 122) to provide comfort in wearing. The left and right upper/lower bands (121, 122) are designed to be integrated with the user's face skin with the size of the user's head in mind. In other words, the left and right upper/lower bands (121, 122) are designed to support the left and right fasteners (112) maintain its three-dimensional shape against the left and right contact portion (111), as well as to aid the left and right upper/lower bands (121, 122) maintain their states with pulling force toward the direction of interest, allowing the left and right contact portion (111) to be integrated with the user's face skin.

In the present invention, the configuration and function of the left and right upper/lower bands (121, 122) are very important. For example, in cases where a user wears a mask and do daily routines, sleep, engage in vigorous exercises, or measure bio-signals, the SMARTMASK (10) must not slid off from its desired position and should be comfortably fixed for a long time. The fixed type of the present invention must maintain its function even when the user is moving unconsciously while sleeping, or wearing a protective headwear such as a hat or a helmet. In particular, it should provide comfort and reliability, be freely positioned at the top or back of the head, and be securely fastened on the head without sliding

Although the left and right upper and lower bands (121, 122) does not have a clearly defined elasticity, it is estimated to be similar to that of the left and right tight contact portions (111). For example, its width is estimated to be 5 mm to 20 mm, the thickness to be 100um to 300um in the form of a soft long strap shape. It may be manufactured separately to fit various head sizes- large, middle, and small, in order to accommodate various sizes. In addition, it may be manufactured in various forms, for example, with a single average head size design.

Furthermore, the left and right upper and lower bands (121 and 122) may go through a post-process to be capable in preventing static electricity. The left and right upper and lower bands (121,122) may be formed of hard silicon or soft silicon, or may be formed of any hardness, and may be transparent or translucent. The length may be adjustably configured so that the overall length may be reduced or lengthened and may be permanently or arbitrarily mounted to the left and right fasteners (112).

The left and right upper/lower bands (121) and the left and right lower band (122) formed by the procedure aforementioned have flexibility and elasticity. For example, it is formed to have a elasticity similar to that of the left and right tight contact portions (111), and has an excellent flexibility. The flexibility and elasticity simultaneously stretches the left and right contact portion (211) when the pulling force is applied on the left and right upper/lower bands (121, 122) upon the use of the SMARTMASK (10). In other words, the pulling force is distributed onto the left and right tight contact portions (111) and the left and right upper/lower bands (121 and 122), so that the pressing portion is not concentrated in a part, minimizing the pressure and allowing easy shape change at the same time.

Accordingly, it is possible to distribute the force to the right and left tight contact portions (211) and to provide a structure that can ensure comfort regardless of various ways of wearing it, such as for long workouts, working or sleeping, by minimizing the pressure felt on the head portion.

As shown in the figure 2, the left and right upper/lower bands (121, 122) has the left and right free end portion (122k) on the opposite of the left and right rotator portion (122j), which allows free adjustment in lengths of the left and right rotator portion (122j) and the left and right upper/lower bands (121, 122) in the left and right fastener (112). In other words, the left and right upper band (121) consists of the left and right rotator portion (122j) rotatable with the upper portion (112a), while the fastener (117) consists of the left and right lower band (122) rotatable with the left portion (112b) in the left and right fastener (122).

In order to allow the left and right upper/lower bands 121 and 122 to be easily attached to and detached from the left and right fastener (112) as shown in Figure. 2- 6b)∼6C, the bolt of the pressing plate (117a) formed at the lower center of the fastener (117) is inserted into the (112m) formed in the rotator portion (112c). The pressing plate (117a) is formed on the lower center projection (117c) of the fastener (117) and the bolt is projected to the lower center of the pressing plate (117a). In this state, the pressing plate (117a) presses the attaching portion (112c), and at the same time, with the bolt passing through the of the attaching portion 112c, the pressing plate (117a) is fastened with the receiving plate (117b) having the nut, and is fixed to the attaching portion (112c).

As the fastener is threaded, the fastener (117) can be detached from the attachment portion (112c), by rotating the nut of the support plate (117b) to separate it from the bolt of the pressing plate (117a) and then detaching the fastener (177) from the attachment portion (112c).

Particularly, as shown in the figure 6b), the perforation (not shown) can be fitted to the fastener (117) in an interference fit by forming the perforation (not shown) in the rotation end portion (122j), The structure of the fastener is simplified so that the left and right upper/lower bands (121, 122) can be freely rotated around the fixing means 117 as the rotation center.

The left and right free end portions (122k) can be adjusted in length using the slit (127), which allows adjusting the length of the left and right upper/lower bands (121, 122) in accordance with the head size. The left and right free ends (122k) are detachable with fastening parts capable of fixing the left and right free ends (122k).

Specifically, the left free end (122k) passes through the layer of the toggle (122L), and then the strong toggle (122L) is installed by fixing the portion of the left free end (122k) which folds back to overlap with the slit (127), and the weak (122R) detachably connected to the water tightening member (122L) is provided at the right free end 122k. The toggle (122L) has a pair of elastic claw pieces at its tip end, is elastically inserted into the elastic claw-shaped claw-and-socket fastening member 122R, and protrudes to both sides of the arm fastener. The elastic claw pieces in such a fixed state can be pushed from both sides to narrow the width in between, thereby being pulled out from the inside of the arm fastener. Here, the left and right free ends (122k) can be replaced with any fasteners that can fasten the left and right free ends (122k) in a detachable manner.

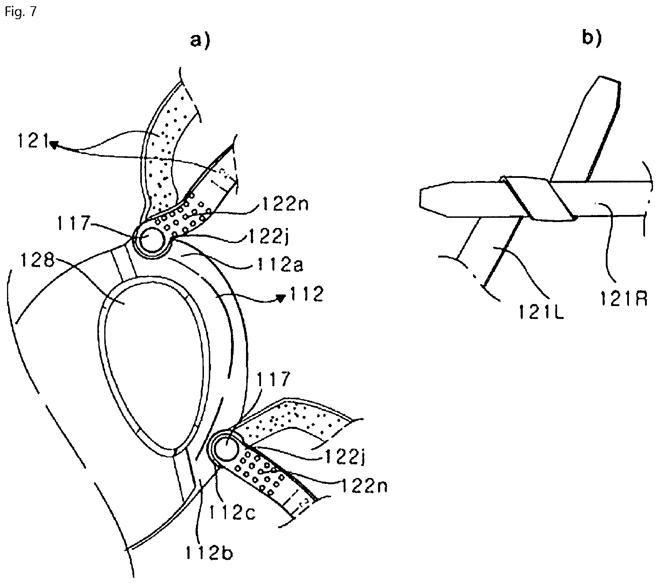

Figure 1-2 shows a structure where the left and right upper/lower bands (121, 122) is fixed via contracting structure on its head part; however, it is not the only structure possible, and any structure can be deployed for the upper/lower bands (121, 122), as long as the SMARTMASK (10) can be worn onto the face tightly without excess pressure. For instance, figure 7b shows the left and right upper/lower bands (121, 122) do not consist of contracting structure, and uses friction resistance in between to adhere. As a method of raising the frictional resistance, the mold surfaces corresponding to the left and right upper and lower bands (121, 122) are smoothly curved by a lapping technique in the process of manufacturing the left and right upper/lower bands (121, 122). The left and right upper and lower band members (121, 122) are formed in the same shape as the upper and lower bands (121, 122). In this case, it is placed on the head, as shown in figure. 7b)

As a method of raising the frictional resistance, the mold surfaces corresponding to the left and right upper and lower bands (121, 122) are smoothly curved by a lapping technique in the process of manufacturing the left and right upper/lower bands (121, 122). The left and right upper and lower band members (121, 122) are formed in the same shape as the upper and lower bands (121, 122). In this case, it is placed on the head, as shown in figure. 7b)

By simply folding the bands (122L) and (122R) together, the force of the left and right tight contact portions (111) on the head from the user's face acts to increase the head force on the head, and the left and right upper/lower bands (121, 122) are attached to each other. As described above, since the binding friction force between the left and right upper/lower bands (121, 122) is maintained to be further increased, even if a tensile force acts, the binding of the left and right upper/ lower bands (121, 122) is not canceled. Therefore, binding and termination operations can be performed quickly and easily. The left and right upper/lower bands (121, 122) can be constituted in a simpler configuration and the method of wearing can be further simplified. An advantage is that it is possible to make a quick settlement even in a difficult situation such as a sudden pollution of the environment.

The left and right upper/lower bands (121, 122) may be made of an elastic material such as a stretch yarn or an elastic rubber such as span texture for easy adhesion or wear of the SMARTMASK (10), elastic material of fabric or non-woven fabric, or an auxiliary member for adjusting the length of the attachment means may be used

On the other hand, as shown in the figure 7a), the left and right upper/lower bands (121, 122) are provided with a scale and a numerical scale (122m) in which numerical values increase from the left and right fastener (112) to the left and right free ends (122k). The unit scale (122m) according to this example, is configured such that the right and left tight contact portions (111) suitably pull the user's face skin symmetrically by the pulling force of the left and right upper/ lower bands (121) and performs a function of confirming whether or not it is pulled out. The unit scale (122m) is used to measure the body signal by attaching and detaching the body signal measuring device (200) to the right and left tight contact portion (111). The living body measuring device verifies whether or not a uniform contact force is maintained at the position, and confirms whether or not the correct state of wearing the bio-signal measuring apparatus (200) is maintained.

The unit scale (122m) is provided on the inner side surface or the outer side surface of the left and right upper/lower bands (121, 122) so that it can be smoothly identified and confirmed from the outside. In particular, the unit scale (122m) may use any pattern including numerals, symbols, and scales so that the numerical values of the lengths of the left and right upper/lower bands (121, 122) can be detected

In addition, the inner surface (122h) of the left and right upper/lower bands (121, 122) can be configured to have a slip prevention function. The inner side surface (122h) having such a slippery function serves as a constituent that affects the force pulled by the left and right tight contact portion (111) against each other. The inner side surface (122h) having the slip resistant function may be formed by including inorganic or organic materials having friction on the left and right upper/lower bands (121, 122).

The inner surface (122h) of the left and right upper/lower bands (121, 122) are made of a non-slippery inorganic material mixed with silicon, which is a binder, and are provided so as to have different patterns as necessary. The friction-free anti-slip mineral is a particle with size of 0.1 to 50um, and may be the same or similar to one or more anti-slip minerals in its kind. As a side note, a non-slippery inorganic substance having transparency frictional force may be used for a permanent and perceptive visual function of the left and right upper/lower bands (121, 122).

In this way, when the non-slippery inorganic material having the frictional force is formed on the inner surfaces of the left and right upper/lower bands (121, 122), the frictional force against the user's head can be doubled. Although the inner side surfaces of the left and right upper/lower bands (121, 122) have been described as including a friction-inducing inorganic material so as to have a slip prevention function, any technical method can be used as long as a surface having a slip function can be formed.

In the present invention, when the SMARTMASK (10) is worn, the left and right upper/lower bands (121, 122) are gripped by the left and right upper and lower band member ends (122j) with both hands. Therefore, it is also possible to provide the grip portion (122n) on one end of the left and right upper band members and to show the grip along the grip portion (122n). The grip portion (122n) may be provided by, for example, embossing, printing, or the like. For example, the gripping area and gripping method of the left and right upper/ bands (121) as well as the binding method may be indicated by symbols, illustrations, characters, figures and the like.

As shown in figure 22, the left and right the left and right upper/ bands (121) have various inclination angles and are freely or selectively disposed on the head, depending on the situation and according to the wearing purpose of the user. The left and right upper/ bands (121) are worn by the SMARTMASK (10), and when viewed from the side, an imaginary line L2 linearly connected from the contour portion (111a) to the lower contour portion (111b) through a straight line from the upper portion to the lower portion and an imaginary line L2 connecting the fixed portion intermediate point and the imaginary line L1. The left and right upper/ bands (121) are rotatable connected to the upper left and right fastener (112a) so that they can be freely disposed along any of the rear legs along the arrows. As a result, it is preferable to arrange them selectively as desired. Therefore, it is very important in the concept of triangulation that the left and right upper/ bands (121) are arranged freely or selectively or as desired.

The structural configuration and function of the left and right upper/lower bands (121, 122) is particularly advantageous for preventing the intrusion of the SMARTMASK (10) from the user's face during conversation during sleep. the user only has to place the left and right upper/ bands (121) on the crown of the head and the left and right lower band (122) on the back side. Using the concept of triangulation, this simple operation guarantees that the SMARTMASK (10) is stably worn on the face.

As a result, the functions of the left and right upper/lower bands (121, 122) are shown in figures 22 and 23. The left and right upper band (121) is in contact with the left and right tight contact portion (111), and the left and right upper band (121) and the left and right upper band (121) are dispersed as a whole so as to minimize the pressure feeling of pulling, thereby performing the main force point of tightly adhering the left and right tight contact portion (111) and the left and right lower band (122) to the face skin and the to minimize the pulling feeling so that the left and right lower band (122) serve as auxiliary force points.

These two pointed pull force configurations are such that forces are distributed point-wise in the SMARTMASK (10), using the concept of triangulation, as shown in figure 22. For example, "A" and "B" as shown in figure 23 can be pulled up with a gentle inclination angle in the direction of "A "and" B " The left and right tight contact portion can be pulled out with no sense of urgency so as to be horizontal in the direction of the user's back head region (220). In addition, the same close contact can be pulled in a point manner in the direction of "A", that is, in the direction of the user's apical direction and in the direction of the back of the user, that is, in the "A" and "B" directions.

As described above, this function is exhibited and the fact that the left and right upper/lower bands (121, 122) can freely rotate in the left and right fixing portion (112c) means that the left and right upper/lower bands (121, 122) are dispersed while accommodating the pulling forces themselves, and are free to be placed on the user's head without pressure. The left and right fastener (112) serve as an anatomical reference to be positioned behind the ear to ensure proper upward and downward positioning of the left and right upper and lower band members (121, 122) and to be freely movable in the left and right fixing portion (112c). The SMARTMASK (10) is advantageous in that the left and right contact portion (111) and the left and right upper/lower bands (121, 122) are accommodated at the same time so that the pressure is not concentrated on a part. This is very important in terms of ensuring a comfortable wearing of the wearer.

On the other hand, the left and right upper/lower bands (121, 122) and the left and right contact portion (111) use different molds for manufacturing. It was then explained to be composed by combining with the adjustment method (117). However, it is not limited to this. This invention can be produced as a whole by combining the upper/lower bands (121, 122) and the left and right contact portions (111). The method as a whole uses mold for the left and right upper/lower bands (121, 122) and left and right contact portion for production. Thus, it can be combined as a whole and it can be comparatively easily formed.

As described above, when coupled with the rim (113a) by the coupling, as shown in figure 17, the opening (113) formed by the having the opening at the top is connected by the lid upper portion (133), so that even if a pulling force is applied, it can be stably fixed so as not to be pulled excessively. The lid portion (130) is formed in a cup shape that protrudes forward in a three-dimensional shape. This lid portion (130) is also referred to as a "cup-shape part" or a "nose mouth lid".

### [1.4] Cover Part

As shown in figure 9A, the embodiment of the present invention is described with reference to the drawings. Figure 10 a is the drawing according to A-A' in figure 9b. Figure 10b is the drawing that is displayed according to B-B'.

In biological aspect, the nose is the organ to breath and the mouth is the organ to intake food and to speak. The nose and mouth are completely separated to prevent breathing with the mouth to provide breathing with the nose. When the mask is designed to minimize the mouth part, not only healthy people but people working in factories, respiratory patients, allergy patients, patients with cold, rhinitis patients, elders, and children can wear the mask without inconvenience. Examples of existing masks are being manufactured without detecting these general issues.

The SMARTMASK (10) is advantageous in that the left and right contact portion (111) and the left and right upper/lower bands (121, 122) are accommodated at the same time so that the pressure is not concentrated on a part. This is very important in terms of ensuring a comfortable wearing of the wearer.

As described above, when coupled with the rim (113a) by the coupling, as shown in figure 17, the opening (113) formed by the having the opening at the top is connected by the lid upper portion (133), so that even if a pulling force is applied, it can be stably fixed so as not to be pulled excessively. The lid portion (130) is formed in a cup shape that protrudes forward in a three-dimensional shape. This lid portion (130) is also referred to as a "cup-shape part" or a "nose mouth lid".

As shown in figure 9A, the embodiment of the present invention is described with reference to the drawings. However, the specific configuration is not limited to these embodiments; changes and additions within the scope not departing from the gist of the present invention are included. For example, the side surfaces may be angled as a whole, and the side surfaces may have a predetermined curvature as a whole. The user's nose and mouth protruding through the chin rest (114) can be received at a substantially constant distance from the inner surface of the lid (130). In such a structure, when the SMARTMASK (10) is worn, the user's nose, mouth, and inner surface of the lid are worn apart from each other, so that the nerves in which the nose and mouth cover 130 are rubbed out are eliminated.

Here, the lid (130) includes a top (133) and front covers (134). The top cover (133) and the front cover (134) are completely folded when they are folded from the top to the bottom in order to facilitate carrying and wearing of the SMARTMASK (10).

The lid portion (130) can be manufactured by a known technique such as injection molding and press molding. The material used is preferably made of silicone so that it has skin tightness and the whole is suitable for most users. An additive such as an air blocker, a pigment, an ultraviolet screening agent, a sterilizing agent, an adsorbing material such as activated carbon, an oxygen generating material, an anion generating material and the like may be added to the composition forming the cover 130. It is natural that it can also be made opaque or transparent or translucent.

The lid upper portion (133) may be made of silicon having a hardness of 50 degrees so as to be able to maintain the approximate shape of the lid portion or to support the filter portion (139), which is approximately 100 to 200um thick. Due to this relatively thick configuration, the lid portion (130) can maintain the approximate shape of the lid portion and is fluid or air impermeable, and the inspired air or exhaled air cannot pass through the lid portion (133).

The main purpose of the upper lid portion (133) is to be so small as to arrange only the nose so as to secure the ultimate internal gas space (133d) which is thoroughly isolated from the external environment. In addition, the lid portion (130) is supported in a desired shape as a whole, thereby applying a fixing force to the shape of the opening (113) and preventing the front cover (134), which will be described later, from collapsing against the user's mouth It is helping. Therefore, even if an external force is applied to the upper lid portion (133) of the cover portion, the shape of the upper lid portion (133) does not collapse and it is necessary to have a layered elasticity, shape memory, and the like so that it can be restored to its original shape even if deformed many times.

In order to allow air to flow into the small internal gas space (133d) only through the filter unit (139) to be described later, the filter unit (139) is provided with a coupling (not shown) in which the filter unit (139) is detachably attached to the left and right, is provided. The typical location of the engagement on the lid upper portion (133) is the front left and right in front of where the user's nose hole will be, as shown by the dashed line in figure 12b) when the SMARTMASK (10) is worn. The gap between the nose hole and the engaging disposed immediately in front of the nose hole in front of the nose hole is approximately 5 mm so as to arrange the filter portion (139) in front of the nose hole or alternatively to arrange the nose hole and the filter portion as close as possible, up to 20 mm Arranging the coupling (not shown) and the filter portion (139) at such a position assures a comfortable breathing environment by minimizing the breathing resistance by inflating the inhalation and exhalation pressure generated by the user during breathing.

As shown in figure 10b and 10b), the upper lid portion (133) is provided with a folding portion (137), a rim (133b), a full layer (136). The wall (131) includes a shielding portion (131a) and a filter portion (139) and includes a folding portion (137), a rim (133b), a top layer member (136) and a wall portion (131) consisting of a shielding portion (131a). The small internal gas space (133d) is created between the user's nose and the inner surface of the cover lifting portion (133) by the elastic portion (139).

As shown in figure 10b and 10b), the upper lid portion (133) is provided with a folding portion (137), a rim (133b), a full layer (136). The wall (131) includes a shielding portion (131a) and a filter portion (139) and includes a folding portion (137), a rim (133b), a top layer member (136) and a wall portion (131) consisting of a shielding portion (131a). The small internal gas space (133d) is created between the user's nose and the inner surface of the cover lifting portion (133) by the elastic portion (139).

As shown in the 12b) with dotted line, to ensure that the inside of the lid portion and / or the small internal gas space (133d) is secured over the entire length of the skin, it is sufficient to simply wear the upper part of the lid part with respect to the image. Since the folded portion (137) of the upper lid portion (133), the rim (133b), the full layer (136), the wall (131) and the shielding portion (131a) are organically cooperated with each other, It is ensured that the inside of the upper part and / or the small internal gas space 133d are completely isolated from the external space, even in repeated use.

As shown in the 12b) to 13a), the inside of the lid part and / or the small internal gas space (133d) are in close contact with the nose of the user to ensure the air tightness of the breathing air, The outer periphery of the upper portion (133) is preferably formed into a substantially isosceles triangle reflecting the shape of the nose. And it is preferable that it is formed in such a small size as to cover the left and right COBO from the tail portion to the human body portion, and in a plan view, in figure 12b, only the nose of the triangular shape. If it is small size, it is on the floor. This is because, unlike conventional masks, the inside of the lid portion and / or the small internal gas space (133d) is formed in a small size similar to the nose size, and does not contact the nose and the inside the upper lid portion (133). It is important that the nose of the user and the inner surface of the upper part of the lid are entirely spaced apart from each other by an interval of 1 mm to 10 mm so as to cover the nose at the same time.

In details, when the length from the left nose ball to the right nose ball is "C" in figure 12b, the size of C is 30mm. When the length of the left/right nose balls to the middle of the forehead is "B", the size of "B" is 50mm. Also, in figure 13a, when the length from the philtrum to the end of the nose is "D", then the size of D is 15mm. The size for the small internal gas space (133d) and/or the inside of the upper part of the cover part is 35mm for Size C, 55mm for Size B, and 20mm for Size D. Thus, the best area is from 30∼70 for the small internal gas space (133d).

These sizes are slightly larger than the normal nose size because the nose width and the nose height are slightly different as well as the nose width according to the anatomical standard. Furthermore, in order to ensure a more comfortable fit, it is desirable that the interior of the upper portion of the lid and / or the small internal gas space (133d) is varied in size, similar to the shoe size, desirable. In the present invention, such a small size of the small internal gas space (133d) is very important for heating and humidification of the intake air, respiratory resistance during breathing, and anatomical dead space, as will be described later. It is more preferable that the sizes of the joints are customized depending on the size of the user's nose. In the present invention, such a small size of the ultimate internal gas space (133d) is very important for heating and humidification of the intake air, respiratory resistance during breathing, and anatomical dead space, as will be described later.

One of the major drawbacks of the conventional mask is that the mask is pressed against the skin of the corresponding area due to the contact between the mask and the interpupillary distance, which causes the wearing pressure to be high. In addition, in the case of a wearer wearing glasses, the wet eyes are discharged to the between the concave spaces, and the glasses are blurred, so that the user's vision is blurred. The problems of these conventional types are caused by the unstable mask being designed to be in line contact between the spaces and the mask.

In order to solve the above problem, according to the present invention, as shown in figure 9b, the folded portion (137), the wall (131), the shielding portion (131a), the full layer member (136) and the rim (133b) is integrally connected. The folded portion (137), the wall (131), the shielding portion (131a), the filler member (136), and the lid upper frame (133b) interact with each other or cooperate with each other, In the region of "A", "B", and "C", that is, a region formed between the eyebrows in the upper direction along the left and right nose ball clearances from the human eye on the face of the user, Respectively. By doing so, it is possible to prevent curved skin crevices.

Since the inner surface of the folding portion (137), the wall (131), the shielding portion (131a), the top layer (136) and the upper edge (133b) of the lid portion are in direct contact with the skin, higher safety is required. However, for instance, it may be desirable to provide a non-magnetic contact surface that provides a swirling, soft touch throughout. In addition, it is preferable to be formed to have a substantially uniform and superimposed adhesion force.

When the external force is applied after each of the constituent elements is closely attached to each of the skin portions that are in contact with each other, the skin portion (137), the wall portion (131), the shielding portion (131 a), the top layer (136), the top layer border (133b) are naturally adhered to each other along the two nostrils of the right and left nostrils along the two nostrils of the left and right nostrils, depending on the shape of the nostrils and the left and right nostrils, And the front cover 134, which is the open space (134d) in which the outer space and / or the mouth is located, are completely distinguished from each other,.

The folding part (137) is worn across the gap between the user's eyes (between the eyes) and between the user's eyes (between the eyebrows) when the SMARTMASK (10) is worn, and is configured to have a close contact with the skin part depending on the situation. In other words, the folding part (137) can prevent interpupillary distance between the user's body when it is desired to protect the user's respirator from contaminated air, or when it is desired to keep humidified air in the small internal gas space (133d), So that external air cannot flow into the internal gas space (133d).

Here, the curved portion (137e) is formed as shown in figure 13 a) by being gently curved and curved at the upper portion (133) of the cover. The curved portion (137e) is formed at the boundary between the upper portion (133) of the cover portion and the start point of the folded portion (137). When the SMARTMASK is worn, the inside of the cover portion (133) and/or the folded portion (137) formed on the upper portion of the curved portion (137e) maintains a large area from the interpupillary distance to the uppermost portion in order to prevent direct skin contact with the skin, as shown in Fig. 13a).

Here, it is made thinner so as to be tightly contacted between the copper spaces and between the copper spaces while maintaining the same shape as covering the copper spaces and between the copper spaces, extending integrally from the curved portion (137e) upwardly, the folded portion (137) is formed. Since the nerve is distributed anatomically on both sides of the nose of the face and accordingly the nerve is pressed, the folding part (137) is not in direct contact with the nose to minimize the feeling of wearing pressure.

The mask is worn over a large area since it is covered from the interpupillary distance to the front. Therefore, as shown in figure 6b), the folded portion (137) is formed so as to be covered from the interpupillary distance to the middle, "U-shaped"

It is a matter of course that the folded-back portion (137) is formed to have the shape and size from both eyes to between the eyebrows so as not to cause harm to both eyes. Therefore, it is preferable that the length thereof is longer than the width thereof, and the width of the semicircular phase thereof is substantially 10 mm to 15 mm, and the length thereof is approximately 10 mm to 20 mm. Particularly, the thickness is preferably smaller than that of the lid part upper part (133) so as to adhere to the skin of the user, and it is preferable that the thickness is substantially 30 to 70 mm. The specific size of the folding part (137) is manufactured in accordance with the body size of an adult, and it is natural that a small folding part is applied to a small body such as a child.

In particular, as shown in figure 14a), the folded-back portion (137) is not formed in a curved shape so as to extend from the space to the innermost space, but as shown in figure 14b). When the SMARTMASK (10) is worn by a flat shape, if a pressing force is applied, it is deformed along the original shape by being simply worn as shown in figure. 14a), and is curved and tightly contacted. Of course, the folded-back portion (137) may be formed in a slightly curved state from the space to the innermost space so as not to significantly deviate from the gist of the present invention.

Further, the inner surface of the folded portion (137), which contacts the space between the user's moving spaces, must be formed to have a substantially uniform and superimposed adhesion force so as to be brought into close contact with the skin in order to ensure that a good seal is formed do. Here, the method of forming the side surface adhesion force in the folded portion (137) at a high level can be formed in the same manner as the method of increasing the adhesion of the left and right close contact surfaces (111c) as described above, so detailed description is omitted in here.

The folded-back portion (137) configured as described above is tightly attached to the skin from the user's human space to the skin as shown in the area "A" actually shown in figure 12b) when the SMARTMASK (10) is worn. As shown above, the left and right upper contour portions (111a) are arranged on the skin just below the left and right eyes as shown in the above description, as the braces and the left and right eye bars support the lower skin. Since the folded portion (137) is protruded from the opening (113) to the upper portion thereof, it is worn in a substantially "⊥" shape when viewed from the front. When the skin is worn as such, there is an advantage that the folded portion (137) protects the skin from between the coaxial space to the skin. At the same time, it also plays a role of correcting the wrinkles that occur from the space between the spaces to the sea, so as not to be conspicuous.

In particular, the lid part (137) explained above can be raised according to appropriate situation and can be fixed. For example, the lid part (137) can be raised as indicated with the arrow in figure 9a during measurement of body signal when sleeping. Then, the adjustment material (137d) and hole (137a) are combined to be fixed. This can be seen in figure 18. The lid part (137) is raised and adjusted between the pupils and the middle of the forehead where the lid part moves and forms 1∼2mm free space between the pupils. Accordingly, the lid part (137) being raised gives free space where the air can flow in and out from the upper part of the small cover part and/or the internal gas space (133d).

In addition, the lid upper portion (133) further includes a nose clip (137c) so as to be more closely contacted between the folded portion (137) and the same space. The nose clip (137c) is provided at the starting point of the folding portion (137), that is, on the outer side of the curved portion (137e), as shown in figure 13a).

The nose and the inside surface of the curved portion (137e) are spaced apart from each other by the provision of the nail clip at the starting point of the folding portion, that is, the outside surface of the curved portion (137e). The boundary point between the nose and the left and right balls and the curved portion (137e) are kept in close contact with each other while maintaining the upward / downward gap with the saddle by the pressing force. Since the curved portion (137e) is located at the starting point of the folded-back portion, the folded portion (137) is deformed so as to be curved spontaneously as the curled portion (137) protrudes compared to the space between the curved portions. The nose clip (137c) ensures that the space between the user's living space and the folded-back portion (137) from the user's living space can be shaped to maintain the user's desired spacing according to his or her preference. Thus, the user can form any suitable wearing interval according to the appropriate situation.

The nose clip (137c) may be of any rigidity so as to have appropriate flexibility and to maintain the shape of the curved portion (137e) and to prevent the skin from being damaged. For example, it may be formed of a flexible and soft band of a metal such as aluminum. It may be a form that maintains a constant shape. It is preferable to be provided on the outer surface to improve the feeling of fit. However, the present invention is not limited to this, and may be formed on the inner surface in consideration of the aesthetics of the outer appearance. The length, width, and thickness of the nose clip are not particularly limited, but may be suitably provided according to the size and shape of the curved portion (137e) to be formed into a nose clip.

As shown in figure 13 a), the wall (131) is configured to seal the nose of the user from the tip of the nose (the nasal cavity of the nose) to the neck portion, that is, just below the nose hole. The wall (131) is formed in a plate shape having a thickness of 100 to 200 mm. And one end thereof is integrally formed along a curved inner surface of one side of the inside of the lid part, and the shielding part (131a) is formed integrally with the other end thereof while being bent forward. And a contact piece (131b) is formed in a direction opposite to the shielding portion. As shown in figure 9 b), the wedges (136) are extended from both side edges of the left and right sides to connect with the full layer member (136). Then, the overall shape is formed into an "L" As shown in figure 12 a).

As shown in figure 13 a), the wall (131) and the shielding portion (131a) may be formed in the upper part of the lid part where the nose is arranged in the lid part (130). The open space (134d) in which the mouth 133d and the mouth are disposed, that is, the nose region just below the nose hole, which is the nose hole and the mouth, is tightly sealed.

As shown in figure 10b), in order to minimize the breathing resistance and to ensure a more comfortable feeling of wearing, the lower part of the wall (131) is provided with two respirators (nose holes) And an exhaust (142) is further formed at the lower position. The exhaust (142) may be formed in a similar shape and size to the left and right closely fitting portion coupling grooves (116) as described above, and the exhaust portion (140) may be interposed.

Since the exhaust part (140) is formed directly below the nose hole and the gap between the nose hole and the exhaust part (140) is located very close to 1 mm to 10 mm, the air containing carbon dioxide discharged from the nose hole can be quickly discharged to the outside. As shown in figure. 13 b), the exhaust unit (140) is made of a thin film of rubber material so that the rubber film (141) is in contact with the engaging (116) so that the rubber film (141) can easily flow. The rubber film (141) is separated from the exhaust (142) to allow the inner air to escape through the outer space into the spaced space, while if the air is sucked through the insides, the rubber film (141) is in close contact with the exhaust (142) in accordance with the traveling direction of the air so that the rubber film (141) is completely sealed to prevent the outside air from being sucked. This exhaust unit (140) may use any known exhaust filter which rapidly discharges carbon dioxide-containing air into the outer space in the inner gas space.

As shown in figure 10b), the coupling (116) may be further provided in the lower portion of the wall (131) in a position immediately below the respiratory apparatus (nose hole) in consideration of the positions of both side nose holes The coupling (116) is formed to have a shape and function similar to the left and right tight fitting portion coupling grooves (116). The coupling (116) includes a filter portion 139, a living body signal measuring device, a respiratory disease treating device, a sensor, an oxygen generator, a heater, a fragrance generator or the like may be combined, and these devices may be configured to be easily detachable and attachable.

Since the coupling (116) is configured to be detachably attached to a device or a device according to a specific situation, a connecting member (not shown) may be additionally provided so as to be smoothly coupled or mutually secured. In general, when the apparatus is not engaged, a stopper (not shown) is coupled to the coupling to maintain the coupling (116) in a sealed state.

The upper portion (133) of the lid may include an internal gas space (133d) formed therein as described above. It is natural that the above-described devices can be configured to be connected to each other depending on the usage purpose.

As shown in figure 12 a), the shielding part (131a) is formed to have a thickness of substantially 50 to 100 mm, such that the shielding part (131a) is adhered to the lower part of the wall (131) while adhering to the user's weight part, and is integrally formed. The shielding portion (131a) is configured to ensure that when the external force is applied to the point "C" as shown in figure 12 b), the external force is deformed and adhered to the original finger on the user's face. The central portion (131b) of the shielding portion (131a) is lower along the longitudinal direction of both left and right sides of the two respirators (nose holes) as shown in figure 10 b) It is preferable that the portion (131c) is formed in a substantially U-shape relatively higher than the center portion (131b).

In particular, as shown in figure 12 a), the width "L" of the shielding portion (131a) is preferably 4 mm to 15 mm. Since the width and height of the human body are slightly different from each other, the shielding portion (131a) is adhered to the human body while closely adhering to the human body, so that the inside of the lid portion (133) and/or the small internal gas space (133d). If the distance is less than 4mm, the user will be in contact with the line of the line, thereby causing a sense of pressure and pain. In particular, when the user is in conversation, and external air may be introduced through the clearance. On the other hand, if it is wider than 15 mm, the feeling of pressure and pain are not caused, but the large area may cause discomfort to touch the upper lip out of the lower lip and cause difficulties in manufacturing.

In addition, in order to ensure that the inner surface of the shielding portion (113a) contacting along the user's forehead is adhered while sticking to the skin, a substantially uniform. It must be formed to have adhesion. The shielding portion (113a) configured as described above seals the region "C" substantially in region depicted in figure 12 b), and thus ensuring that the nose and mouth are completely isolated, as shown in figure 13 a), and in any case block the flow of air from the mouth to the nose or from the nose to the mouth

The layer member (136) has been formed to block the external air that comes in through the internal gas space (133d) through the space between the nose and the cheeks. It is closely contacted to the space between the nose and the cheeks. As described before, figure 10 a and 10 b shows the layer member (136) that is connected with the cover part (131a) to form extension of curve towards the internal gas space (133d) in the most left and right ends of the internal part of the upper part of the cover part (133). Thus, it has the shape of '-' shape. The actual width is from around 2mm to 8mm and the thickness is from 50µm ∼ 100 µm. The length is from around 30mm to 70mm. When formed in this way, the lid part (137) that is composed on the top is extended to the starting point and also closes the part with the space between the nose and cheeks.

Then, the layer member (136) transforms according to the space and closely contacts the skin from the bottom part that is right below both eyes according to the nasolobial fold part between the nose and the cheeks in "B" area as drawn out in Figure 12b. Due to 2-layer 3-layer stages of the layer member (136), lid part (137), upper frame (133b), cover part (131a), and wall part (131), the harmful air that flows in through the internal gas space (133d) and/or the internal part of the upper part of the small cover can be completely blocked.

When manufacturing the filter part (139) regarding this invention, the external part (139a) and filter (138) can be manufactured with the technology now or the technology that is to be developed. The external (139a) and the cover can be manufactured by using injection molding. The filter part (139a) can be formed at prescribed height and formed many types of shapes such as circular shape, rectangular shape, and polygon shape. Also, the inside of the external part (139a) includes a cork (139d) to easily replace the filter (138). Even though it is manufactured strongly with certain thickness, it can be combined to the combining hole (not displayed) of the upper part of the cover part as shown in figure 9a. Also, it is best for the external part (139a) to be formed at certain height to form space where the filter (138) can be installed.

Here, as described before, the filter part (139) shall be formed with maximum exterior so that the nostrils can fit. Also, it can be placed just below the nostrils. Thus, the filter part (139) must be positioned to be as close to the user's nostrils. In this invention, the filter part (139) is positioned with interval of 5mm to 20mm between the nostrils and filter part. Also, it is placed from 1mm ∼ 10mm between the nostrils and filter part (139) just below the nostrils.

In this arrangement, when the user breathes through the nose, the air that has been filtered through the filter part (138) does not have resistance where the user can breathe comfortably. Then, the air is quickly discharged through the filter part (139) and the ventilating part (140). The filter part (139) is positioned in front of the nostrils and the diameter is formed largely. The filter (138) and the area of the air become large as well. As a result, the speed of the air that flows through the filter (138) is reduced and the sleep of the air that passes the airway also slows down. Thus, the user that breathes in and out does not have serious change in pressure inside the airway. The muscle of the area that has been contracted within the airway creates suction which prevents snoring and it can also prevent and ease allergic reactions and inflammation within the mouth.

Especially, the filter part (139) and the internal gas space (133d) and/or internal part of the upper part of the cover part play important role of maintaining the temperature and humidity of the air. The warm air that is discharged from the user's nostrils when breathing in and out while wearing the SMARTMASK (10) remains within the internal gas space (133d) and/or the internal part of the upper part of the cover part where it is blocked partially to the filter part (139) and ventilating part (140). While breathing occurs, the internal part of the cover part and/or the internal gas space (133d) maintains warm moisture and air.

In details, when the user wears the SMARTMASK (10) there is an internal part of the upper part of the cover part and an internal gas space (133d) separately placed as the same size of the nose. When breathing through the nose, the cold air comes in to the internal gas space (133d) and/or the internal part of the upper part of the cover part through the filter part (139). The cold air that comes in through the filter part is filtered due to the filter part (138) and passes as slow speed. This air is slightly heated and enters the nasopharynx. Thus, users that wear the SMARTMASK (10) are not exposed to dry and cold air due to the internal gas space (133d) and/or the internal part of the upper part of the cover part and the filter part (139). Also, certain temperature is maintained in the airway part and the nasal cavity. Then, the user can breathe comfortably and can be prevented with decreased body temperature.

Also, the user that wears SMARTMASK (10) can maintain temperature and humidity of the air from the internal gas space (133d) and/or the internal part of the upper part of the cover part when breathing through the nose. Here, the air including the carbon dioxide is quickly discharged to the external space due to the filter part (139) and the ventilating part. Thus, the user that uses SMARTMASK (10) can maintain temperature and humidity in the nasopharynx and the internal gas space (133d) even when breathing through the nose. Also, it reinforces the existing function of the nose. As a result, the mask it useful to the health by strengthening immunity, strengthening lung function, and also preventing and treating respiratory disease.

On the other hand, there is breathing treatment as a treatment method to maintain life, and this treatment is used in patients with different types of diseases. When patients cannot maintain appropriate breathing by own effort, these patients receive breathing treatment. These patients are provided with appropriate gasified air so that the nasal cavity is not damaged. The advantage of the breathing of this kind of air is very important for respiratory diseases and health management. To understand this, it is important to understand the role of the nasopharynx when breathing.

### Role of the Nasopharynx

1) Heat and Humidification of Inspiratory Gas
2) Remove fine dust, yellow dust, flower powder, and bed bugs from the nasopharynx mucous membrane

Breathing in dry and cold air reduces the pulmonary compliance and conductance. This has been researched since the 1980s. Even for normal people, breathing in cold and dry air can cause respiratory diseases such as bronchocontriction due to muscarinic receptor. The inspiratory air under the physiological function of the airway can be heated up to 37°C and the humidity can reach up to 100%. Elements included in the process of the heat and humidification of the inspiratory air has not been clearly proven, but most occur in the nasal cavity with a lot of energy consumption to occur. Here, when hypoxia occurs due to respiratory disease, many patients go through excessive ventilation where one-time breathing amount increases. In this case, the heat and humidification can be reduced when inserting heated and humidified oxygen. Accordingly, the metabolic work is reduced and carbon dioxide can also be reduced.

Nasal cavity is a relatively broad space among organizations, which constitute respiratory bath from nose to alveoli, inside body. This organ plays an important role which is increasing temperature and relative humidity of air inflows during inhalation.
However, carbon dioxide is not emitted completely and stays inside during exhalation therefore becomes an anatomical dead space which impedes efficient supply of oxygen. Such dead space is very natural for normal people but it can cause lack of oxygen for patients with respiratory disease.

When the user wears SMARTMASK (10) and breathes, air including oxygen is inhaled from outside to small space of internal part (133d) through filter part (139) to filter foreign substances. Further, heated and humidified air passes through nasal cavity and lower respiratory tract then is exchanged at lung. During exhalation, air including carbon dioxide, of which gas has been exchanged, passes through inflow route of oxygen then is emitted to outside quickly through external space through filter part (139) and exhaust(140) without even small resistance. At this time, space of internal part (133d) is formed as small as the user's nose. That is to say, respiratory resistance, which occurs when the user does not wear SMARTMASK, does not occur and environment for comfortable respiration is generated. Therefore, admixture of carbon dioxide of existing masks (carbon dioxide is not emitted due to respiration resistance of mask therefore such phenomenon frequently occurs at the space between mask and nostril) occurs relatively less in case of this invention SMARTMASK (10) (admixture does not occur as there is no respiration resistance) therefore the concentration of oxygen supplied into lung is less diluted.

As shown, the locations of aforementioned cover, aforementioned, internal or/ and internal body space(133d), left and right filter parts located both sides right in front of nostril (139), filter part and exhaust (140) located right under two nostrils help the user breath comfortably without respiration resistance during exhalation. Further, this emits carbon dioxide during exhalation therefore anatomical dead space is eliminated.

The patient with sleep disturbance, illustrated in figure 21a), has a part of which diameter of certain area of respiratory tract is rapidly reduced. Further the user of SMARTMASK (10) illustrated in figure 21b) has a part of which diameter of certain area of respiratory tract is minutely reduced.

Mouth of patient with sleep disturbance illustrated in figure 21a) is enlarged through respiration therefore the diameter of certain area is rapidly reduced. In addition, mouth of the user of SMARTMASK illustrated in figure 21b) is closed naturally by SMARTMASK (10) and the patient breathes through nose therefore the diameter of certain area is relatively minutely reduced. If the user breathes through nose, air flow velocity decreases by more than 100% by SMARTMASK (10) compared to that without using SMARTMASK (respiration through mouth).
In this case, the lift of area, of which diameter of respiratory tract is rapidly reduced, is proportional to square of the speed therefore the speed decreases by 50% compared to the case of not using SMARTMASK. If so, suction phenomenon of the area, of which diameter of respiratory tract is rapidly reduced, decreases by 50% therefore snoring decreases significantly. In this case, nostril of the user of SMARTMASK (10) is located in small space of internal part (133d) therefore the air inflows into the space is compressed.
In this case, pressure increase in the area, of which diameter of respiratory tract is rapidly decreased, increases therefore the air pressure of the area, of which diameter is reduced, is set the same with other areas. Accordingly difference in pressures from that of surrounding muscles decreases in area of which diameter is reduced therefore suction phenomenon decreases. Further, pressure which can enlarge narrowed respiratory tract is generated therefore narrowing of respiratory tract by pressure can be reduced.

Meanwhile, the aforementioned filter (138) can be manufactured with various shapes and sizes according to the shape of aforementioned outer and can include various types of filters. For example, filter for heating and humidification (138a), of which ventilation area is broad and air passes easily even in case of absorption of humidity, nano-sterilized free filter, hepa filter, which eliminates fine dust, dust collection filter(138b), which collects dust through electric reaction, deodorization filter, which eliminates smell, and antimicrobial filter can be included. Further, it can be constituted through laminating or conjugation of filter materials which have one or multiple layers or more than two filter materials with one or multiple layers.

The external air, which is inhaled through respiration of the user by wearing SMARTMASK (10), passes through aforementioned filter. At this time, aforementioned filter can recruit filters with various functions. Therefore, such filters foreign substance, such as dust, as well as smell within air passing through the aforementioned heating/ humidification filter (138a), dust filter (138b), hepa filter, dust collection filter and antimicrobial filter and so on to maintain the heated and humidified states over time.

As illustrated in figure 11, heating/ humidification filter (138a), which has relatively large ventilation area and air circulation is easy even in case of absorption of humidity, is located on the most inner part (space of internal part) of filter (138). Next to the aforementioned heating/humidification filter (138a), dust collection filter (138b), which is for elimination of yellow sand, fine dust and house dust mite, is located. Further, hapa filter and antimicrobial filter can be located subsequently. Of course such filters can be arranged comfortably according to the usage of the user. In addition, such filters need to be exchanged after using for certain time.
At this time the aforementioned closure (139d) needs to be removed to exchange the needed filter.

As shown in figure 9 a), aforementioned front cover (134) is formed integrally with the aforementioned part of aforementioned cover (133). The aforementioned front cover (134) is not contacted to the whole chin and mouth of the user but is vertically separated to form an opened space (134d).

The shape of aforementioned front cover (134) of this invention does not collapse due to maintenance power of the upper part of cover (133) or is not contacted to the user's skin. Further, the shape does not collapse by external power which is wind easily.
Especially, the aforementioned front cover (134) is not integrated with contact left and right contact parts (111) by conjunction measurement unlike upper part of cover (133).
That is to say, the aforementioned front cover (134) maintains its shape while is not integrated with left and right contact parts (111). Further, it has opened environment which is for the user can freely fold the device to upper part according to the situation.

For such opened environment, when the user wears SMARTMASK (10), front cover (134) is not contacted directly to chin, left and right cheeks, which are responsive to chin area of the user as illustrated in figure 16, and covers chin, left and right cheeks with distance of 1mm or 10mm and is arranged to have circular hemisphere shape which is curved to outside.

Accordingly, as shown in figure 16, the central part (134a) needs to be located lower than left and right edges (134b) for the front cover (134) to cover chin, left and right cheeks in three dimensions so that the whole shape to be "U" cross section.

As stated above, the central part is formed lower than left and right edges (134b). Therefore, front cover (134) has aesthetic aspect as well as can be curved to the upper part and fixed without using additional fixing measurement according to the situation or selectively. Further, curved and fixed front cover (134) returns quickly to original status before the curved status by simply unfolding the part.

The main objective of the opened structure of the aforementioned front cover *134) is to cover the user's mouth and chin on face to hide mouth and chin as well as to guarantee free motion of chin at the same time. Such unique structure, which does not exist in existing mask(s), fundamentally prevents warm breath comes from the user and displeasure of mouth and chin. In addition, lips of the user are not contacted to sides inside the front cover (134) for talking freely and open mouth widely. Further, the user does not need to take off SMARTMASK (10) to open mouth and chin therefore the user can drink and eat drink or food comfortably.

Also, the mask blocks aerosol particles including saliva, mucus and sweat generated when the user sneeze, cough and talk to prevent the others and objects to be exposed to the emitted aerosol particles. Further, the mask protects chin and mouth from external environments including wind, ultraviolet rays and cold.

Meanwhile, as shown in the figure 9 a), if hole for talking (134d) is pierced on the mouth part of the aforementioned front cover (134), the usage is very effective but front cover is consisted of opened environment therefore there does not need to be hole for talking on it.

As a result, the aforementioned cover part (130) covers both nose and mouth as well as divides opened space (134d) and external space on which upper, inner or / and space of internal parts (133d), which are spaces for covering nose. When the user wears SMARTMASK (10), this part prevents respiration naturally and induces breath through nose. Accordingly, clean air, which is heated and humidified through filter part (139), can be provided for respiration through nose. Further, anatomical dead space is minimized therefore labors work in factory, who need to talk while wearing mask, workers of medication filed, food manufacturers, general patients, who feel distress for wearing mask, patient with respiratory disease, allergic patients, the elderly and children and wear the mask without respiration resistance for a long time. Accordingly, this mask provides merits in medical and industrial perspectives.

Figure 26 shows a whole state of wearing SMARTMASK and sports glasses on it.

It is regarded that wearing glasses for wearing glasses on mask, which uses previous technology, especially for fast-moving sports is very uncomfortable behavior. However, as shown in figure 26, the sports glasses worn on sides, apart from SMARTMASK of this invention, does not slides down in case of unexpected situations including various environments such as shock, external force, load required for exercise, ultraviolet rays, sweat and pollutant. Further, the mask does not cause suppression by wearing the mask. In addition, fog on mask does not appear.

As shown in figure 26, nose keyed, which is constituted on in the central part located between both lenses, is worn on curved part (137e). Further, it can be worn on folding part (137). If so, outline of left and right parts (111a) are worn within lenses of glasses. That is to say, the outlines of left, right and upper part (111a) are not contacted to from right under part of eyes to skin of eye corners but the right way of wearing this invented glasses is to put glasses leg on left or right wear cover (128) or left and right contact parts (111c) and the nose keyed needs to be put on curved part (137e) or/ and curved part (137).

If glasses are worn this way, contact part (111c) fixes left and right legs of the glasses to prevent slide. Further, curved part (137e) or/ and folded part (137) support the central part of glasses from the bottom therefore pressure for wearing glasses and slide down disappear. In addition, breath from mouth coming out from crack, which blears glasses, is not generated resulting in better feeling of wearing the glasses. Further, left, right and upper outlines (111a), left and right contact parts (111c) blocks ultraviolet rays and wind around eyes to prevent skin damage, pigmentation of melisma, freckle or wrinkle. In addition, after manufacturing legs of glasses, anti-slide process for legs of glasses and anti-moisture process for lens, which have been performed previously, after wearing SMARTMASK(10). Of course, when wearing glasses, legs of glasses can be worn on left, right contact parts (111) and inner side. In this case, fixation power of legs of glasses increases through elasticity and contact power of left and right contact parts.

### [1.5] Bio-signal measurer

Figure 27 is a figure which indicates bio-signal measurer for this invention briefly. Figure 28 is a schematic diagram of contact of bio-signal measurer illustrated in figure 27 to carotid artery of the user. Figure 29 is a schematic diagram of measurement of bio-signal by using SMARTMASK and left bio-signal measurer in figure 1. Figure 30 is a schematic diagram of measurement of bio-signal by using SMARTMASK and bio-signal measurer illustrated in figure 1. Figure 31 is a schematic diagram of measurement of bio-signal in accordance with figure 27. Figure 32 is a schematic diagram of whole system for measurement of bio-signal by using bio-signal measurer illustrated in figure 27.

Referring to figure 27 or 32, bio-signal measurer (200) of this invention is put on and off from conjunction hole (116) that it is contacted to the user's skin and measures bio-signal. In contrast, the aforementioned bio-signal measurer (200) is not attached to the aforementioned SMARTMASK (10, 20) and the bio-signal measurer (200) measures bio-signal of the user unilaterally.

This configuration makes the aforementioned bio-signal measurer (200) to be attached on SMARTMASK (10) easily and uses SMARTMASK (10) and bio-signal measurer unilaterally in general. Further, if the user moves a lot and wants to measure bio-signal during exercise and sleep stably, this device can be put on and off freely for the user's convenience. Further, this can measure bio-signal of the other user. Moreover, this device can prevent damage on bio-signal measurer (200) caused by cleaning and external factors.

As shown in figure 20, bio-signal measurer of this invention is contacted to inside of ear and ear conch (207) of right side of part 2 at the same as shown in figure 30. Further, the aforementioned SMARTMASK (10) fixes it by covering the bio-signal measurer (200) through tensile force therefore can measure bio-signal stably during sleep or intensive exercise.
In addition, the aforementioned SMARTMASK (10) covers bio-signal measurer (200) completely and blocks external light or environment which can impede accurate measurement of bio-signal therefore more stable measurement of bio-signal is possible.

The SMARTMASK (10), to which the aforementioned bio-signal (200) of this invention is attached has the same function with the aforementioned example 1.

The bio-signal measurer (200) of this invention can measure bio-signal which is generated by physiological potential difference. The bio-signal measurer of this invention can measure electrocardiogram (ECG), electromyogram (EMG), electroencephalogram (EEG) galvanic skinreflex (GSR) and so on. Further, this device can calculate blood flow rate index, pulse rate, glycemic index, momentum index, electrocardiogram index, blood oxygen concentration and body temperature index.

The bio-signal measurer (200) of this invention is attached to conjunction hole (116) on the left side of the aforementioned SMARTMASK (10) and consists of bio-signal measurer (200a) of the left side, which is contacted to part 1 of the user, and bio-signal measurer (200b), which is contacted to part 2. In an example of usage of this invention in a right way, the aforementioned part 1 can be carotid artery of the left side of the user while the part 2 can be the ear on the right side of the user. The bio-signal measurer (200a) on the left side and bio-signal measurer (200b) on the right side can be connected to both edges of neck band (985) and can be combined and separated from each other. The aforementioned neck band (985) can be connection line. The bio-signal measure (200a) on the left side and bio-signal measurer (200b) on the right side make a pair.

The aforementioned bio-signal measurer on the right side (200a) and bio-signal measurer (200b) on the right side can measure bio-signal of both sides at the same time and can measure bio-signal on either of left and right sides. Accordingly, display, button and power parts can be equipped to control performance status and performance of the device.

In case of the aforementioned bio-signal measurer (200a) on the left side and bio-signal measurer (200b) on the right side, bio-signal measurer (200a) on the left side and bio-signal measurer (200b) on the right side are symmetry to measure carotid artery on the left side of the area 1 and ear of the area 2. The reason is that carotid artery and ear, which pass left and right faces make plane symmetry. Accordingly, if bio-signal measurers (200) is attached on each left and right conjunction holes (116) of the aforementioned SMARTMASK (10), bio-signal measurer (200) can be attached on the same body parts of the left and right sides with the same pressure and contact power from the left and right sides..

One or both parts of the aforementioned bio-signal measurer (200a) on the left side and bio-signal measurer (200b) on the right side can be consisted of sound signal generator (930), application part (800), central processing unit (900), database (960) and communication device (950). According to this invention, at least parts of sound signal generator (930), application part (800) and central processing unit (900) can communicate with service server (980), user terminal (700), N terminal (730), 119 (710) and doctor (720) through communication network (970).

Referring to figure 27, bio-signal measurer (200a) on the left side is connected to body part (201 a) and body part covering ear completely that it consist of clip (201b) which is hung on ear and improves feeling of wearing. The aforementioned body part (201 a) and ear clip (201b) can be empty case and consists of plastic or rubber material and can have certain level of elasticity. Further, it can be manufactured in various forms taking into account curve or movement of face. The aforementioned body part (201 a) and ear clip (201 b) includes central processing part (900) which includes acceleration sensor (530), sound sensing part (540), speaker (290), vibration stimulator (930), button part (210), connection port (990), power part (270), display part (215) and CPU (Central Processing Unit). The aforementioned sensor parts, central processing part, button part, speaker, display part, connection port and power part are all connected with connection line.

The aforementioned body part (201a) is configured with size and shape, which can cover ear conch. Further, it includes 1 electrocardiogram sensor part (500L) which is equipped with electroencephalogram contacted to carotid artery of neck. The aforementioned body part (201 a) and 1 electroencephalogram sensor (500L) protrude with certain height and head part (201d) is formed. Further, attachment hole (202) can be configured on the aforementioned head part (201d). In addition, it is recommended to configure aforementioned attachment hole (202) which can be easily put off and on conjunction hole of SMARTMASK (10). The aforementioned body (201a) can be fixed or contacted more stably on carotid artery through the conjunction hole (202).

Connection line (201c) connects 1 electroencephalogram sensor (500L) and body part (201a). The connection line can include data line, which can send bio-signal data, and power line, which can supply power. The connection line can be connected to 1 electroencephalogram sensor (500L) and body part (201 a) to connection port through connector which is connected to both edges. Further, connection line can be fixed to sensor (500L) and body part (201 a) by using soldering. Such connection line (201 c) can have flexibility and elasticity for free movement of the user when measuring bio0signal. Connector is a snap type which can be put on and off from connection ports (990).

The aforementioned bio-signal measurer (200b) on the right side is connected to body part (201 a) and covers ear completely. This consists of ear clip which is hanging on ear and improves sense of wearing. The aforementioned body part (201a) and ear clip (201b) include 2 electroencephalogram sensor (500R), electroencephalogram sensor (500), body temperature sensor (520), PPG sensor (510), vibration stimulator (930), speaker (290), button part (210), display part (215), connection port (990) and power part (270)
Each of the aforementioned sensor parts, button parts, speaker, connection port and power part is connected with connection line.

The aforementioned body part (201a) and ear clip (201b) can be empty case.
The aforementioned body part (201 a) is formed with certain size so that it can be contacted to ear conch of ear. Further, it protrudes with certain height and head part (021d) is formed and attachment hole can be configured on a part of the aforementioned head part (201 d). It is recommended to configure aforementioned hole (202) with a structure which can be easily put on and off from attachment hole (116) of SMARTMASK (10). Further, the aforementioned body part (201a) can be fixed or contacted to carotid artery, temporal region and ear more stably. The aforementioned ear clip (201b) is PPG sensor part (51) and electroencephalogram sensor part (550).

As illustrated in figure 27, the aforementioned sensor part (510) is located on the contact part between ear clip (201b) and ear conch and consists of contact-type PPG sensor. The aforementioned PPG sensor part is configured for ear clip (201b) can be opened and closed. Light emission part (201d) of PPG sensor, which is ultraviolet sensor (or wide pulse wave IR sensor), is located on the area contact to ear clip (201b) of sensor. Further, on the aforementioned body part (201 a) on the other part of PPG sensor, light detection part of the PPG sensor is located on the area in which the light emitted from light emitting area of the area contact to ear conch (207). That is to say, in case of ear conch installed with PPG sensor (510), light is emitted from light emitting part (201d) toward ear conch. Further, a light detection part (201 c) is located on the opposite side of ear conch to detect light emitted from ear conch (207). At least one sensor can be installed on ear clip (201 b) or many sensors can be installed as well.

The aforementioned 1 and 2 electrocardiogram sensors part (500L, 500R), EEG sensor part (550), body temperature sensor part (520), acceleration sensor part (530), sound sensing part (540), PPG sensor part (510) are contacted with at least one of the body parts of the user and consists of at least one sensor which senses various bio-signals from the aforementioned contact parts. This can consists of one or more channels. Especially, the 1 and 2 electrocardiogram sensor parts (500L, 500R) can consists of multiple channels. The aforementioned sensors can protrude on the surface of body part (201 a) and ear clip (201b) and can be contacted to directly to body. The aforementioned sensors will be described in details later.

The aforementioned body part (201a) includes insert part (201 f) which is to be inserted into ear and for contact. The insert part (201f) has at least one of the speaker, body temperature sensor, pulse sensor, electroencephalogram sensor and electrocardiogram sensor. Further, at least one of the speaker, body temperature sensor, pulse sensor, electroencephalogram sensor, electrocardiogram sensor of the aforementioned insert part (201f) is connected with signal line which detects measurement result to outside of insert part (201f) through connection line. The aforementioned speaker, body temperature sensor, pulse sensor, electroencephalogram sensor, electrocardiogram sensor can be minimized through micro machine and LSI technologies and can be installed inside insert part (201f). The aforementioned insert part (201f) can be installed with at least one of these various sensors and various sensors can be installed as well.

The aforementioned button part (210) is for the user to choose starting and ending point of bio-signal sensing through On/Off the bio-signal measurer (200). It is recommended to configure this part on the aforementioned head part (201d) and expose it to external environment on conjunction hole (116) of SMARTMASK (10) for manual control. If the user turns on button part (210), the aforementioned bio-signal measurer (200) is supplied with power from power part (270) and starts sensing and sends the sensing signal to central processing part (900). In addition, if the user turns off button part (210), the aforementioned bio-signal measurer (200) is not supplied with power from power part (270) therefore finishes sensing.
In addition, the user can directly control change in external environment of button part (210). A case in which parts of the following roles and functions of all systems can be set on button part (210) can be introduced.

The aforementioned display part (215) is configured on the protruded part of the aforementioned head part (201d) and it is recommended to set this part to be exposed to external environment from conjunction hole of SMARTMASK (10). The display part (15) indicates performances of the device including power and battery consumptions.

The aforementioned speaker (290) can be located inside ear and be realized through external device. In accordance with examples of using this invention, the aforementioned speaker (290) can output sound signal the same with general speaker. However, in other examples of using this device, it can be electroencephalogram speaker (290) which is configured to deliver sound through vibration or sound wave by contacting to cranium. The aforementioned speaker (290) can be inserted to insert part (201f) of bio-signal measurer (200) and be installed. Further, the aforementioned speaker (290) is attached to ear clip (201b) and can be contacted to behind the user's head especially behind ear.

The aforementioned connection port (990) can be equipped with multiple connection ports for connection of devices, which configures bio-signal measurer (200), to other devices. In the examples of this invention, connection ports can be used by converting input and output of bio-signal according to the usage. Further, power can be supplied though connection port, bio-signal measurement program and MP3 music files can be downloaded.

The aforementioned stimulator (930) can be vibration motor. The aforementioned vibration stimulator is installed on the aforementioned ear clip (201b). The aforementioned vibration stimulator provides dynamic vibration to the aforementioned bio-signal measurer (200) through the aforementioned ear clip (201b). Accordingly, the user of bio-signal measurer can sense the aforementioned vibration. Or the aforementioned vibration stimulator can be transformed to give vibration to human body directly.

If the aforementioned vibration stimulator operates and gives vibration stimulation to the user of bio-signal measurer, the aforementioned bio-signal detection sensor's output and/ or the output of acceleration sensor can be abnormal. Accordingly, if the aforementioned vibration stimulator operates, the aforementioned central processing part (900) can ignore output of the aforementioned bio-signal detection sensor or output of the aforementioned acceleration sensor.

Vibration stimulator (930) is equipped on the aforementioned ear clip (201b) and provides dynamic vibration to human body. Vibration stimulator (93) is equipped on the aforementioned ear clip (201b) and permit vibration to human body. The aforementioned vibration stimulator (930) can be allocated on area, which is most close to human body, from the aforementioned ear clip (201b).

The aforementioned central processing part (900) is configured including CPU(Central Processing Unit)(300). CPU(Central Processing Unit)(300) can be operation processor includes storage which can record bio-signal to be mentioned later. The CPU(Central Processing Unit)(300) of this invention is a device which interprets commands and process arithmetic and logic or data. Further, it performs calculation so that bio-signal measurement system can work well. In addition, it can generate signal which controls bio-signal measurement system according to central processing part (900).

The aforementioned central processing part (900) receives and process electrocardiogram signal, PPG signal, body temperature signal, acceleration signal, sound signal received from A/D transformation part (920). Further, this device stores the result in database part and sends the result to the user's terminal (700) and service server (980).
That is to say, the bio-signal measured in bio-signal measurer is sent to external device.

The aforementioned central processing part (900) receives electrocardiogram signal, PPG signal, body temperature signal, acceleration signal and sound signal received from A/D transformation part. In addition, this part analysis each of the aforementioned sensing signal, process and calculate various bio indexes. Especially, the aforementioned central processing part (900) furnishes a program which can calculate various bio indexes from sensing signal. The examples include programs, which calculate blood flow rate index and pulse from blood flow rate sensing signal, and programs, which can blood pressure index, blood oxygen saturation from blood flow rate sensing signal and electrocardiogram sensing signal. Such bio-signal calculation program is generally well known therefore the details of the program are mentioned hear.

The aforementioned central processing part (900) uses such programs to calculate various bio indexes by analyzing each sensing signal sent from A/ D transformation part (920). Then it sends the results to user's terminal (700) to output the results as visual figure or graph. For example, in the example of operating this invention, the aforementioned central processing part (900) calculates PPT and heart rate (HR) by using PPG signal or electrocardiogram signal. Further, it can calculate index related to blood pressure by using the aforementioned PTT, HR and body temperature sensing signal (SKT)

Further, the aforementioned central processing part (900) generates alarm signal through speaker (290) and vibration stimulator (930) to inform the user if the aforementioned various bio indexes are out of the preset bio indexes. At this time, the change rate of bio index for alarm can be adjusted according to the condition of the user that the aforementioned central processing part (900) analyses bio profile of each user and to calculate statistical data for each state for calculation of appropriate setting value. Further, it can be set by professional such as doctor.

In addition, the aforementioned central processing part (900) can control flow of each data flow. That is to say, the central processing part (900) of this invention controls data flow between each component of bio-signal measurer (200) to make each configuration to perform each unique function. Further, it can figure out and predict sleeping and health status accurately. The central processing part (900) analysis bio-signal and calculates various bio indexes. Further it sends this to mobile communication terminal (700) and output it as visual figure or graph. If the calculated various bio indexes are out of change rate of preset bio indexes, it can generate alarm signal through speaker (290) and make auditory alarm to the user.

In addition, the aforementioned central processing part (900) can figure out toss, turn and abnormal situations including convulsion, seizure, epilepsy and syncope during sleep. Especially, it can cope with risky and emergency situations which occur during sleep quickly. If the situation of the user is very cautions or emergent, it informs the situation to the user and doctor (720) through event including alarm. Especially in emergency status, it makes a call to 119 (710) automatically or sends message to the preset phone number.

The aforementioned central sound signal generator (930) can output alarm or voice signals to inform bio-signal to the user according to the analysis on bio-signal of the user. Further, it can output sound wave or alarm signal, which induce deep sleep, based on the results from analysis on respiration signal of the user. Additionally, the sound signal generator (930) can generate MP3 music file.

The aforementioned application part (800) outputs image of organs, which are displayed as injury on the user's terminal according to the bio-signal situation so that the user can see it directly. Application part (800) enables the user to control surrounding external devices and can provide interface by which the user can see his/ her health conditions in person. Application part (800) can be set for the user to control the measurement and part of the system indicated in this specification in person.
Especially, this application part (800) can provide platform of training program which is for health and sleep statuses. In addition, the user can receive training through the application provided by application part (800).

In the aforementioned data base (960), various bio signal analysis information for each situation and information, which interlink information of the user and bio-signal can be stored. Meanwhile, the data base part of this invention (960) is a concept which includes recording medium, which can be interpreted by computer. If data can be generated by searching even simple group, it can be data base part (960) of this invention. The aforementioned data base (960) can receive at least body information from age, gender, height and weight of the user. Further, it can be referred or used for various bio signal analysis information, bio signal information of each user, sleeping status information and active mass later.

The aforementioned communication part (950) can work for sending and receiving from and to sound signal generator (930), service server (980), application part (800), central processing part (900) and user's terminal (700). The aforementioned communication part (950) can be equipped with at least wireless communication modules including Bluetooth, short distance wireless communication chip (NFC) ZigBee, wireless LAN, and line communication modules including serial and USB to use line/ wireless communication measurements.

The aforementioned power part (270) can supply power to 1, 2 electrocardiogram sensor part (500L,500R), electroencephalogram sensor part (550), body temperature sensor part (520), acceleration sensor part (530), sound signal sensor part (54), PPG sensor part (510), button part (210) and central processing part (900). Power part (270), for example, can be equipped with disposable battery or rechargeable battery. Further, power supply status and residual battery can be displayed on display part (215).

The aforementioned communication network (970) can be connected directly through short distance. In addition, this can be different communication network from the internet and mobile communication network.

The aforementioned user terminal (700) can include application which is for management of health status by utilizing bio-signal. Bio-signal information can be sent from central processing part (900) and the user can set the entire bio-signal measurement system or change and control function directly by using application included in the user terminal (700). In addition, the aforementioned mobile terminal (700) can be configured with GPS receiving and sending devices. The aforementioned GPS receiving and sending parts have been prepared to the user's location information by communicating with satellite through antenna.

Examples of user terminal (700) include smart watch, smartphone, tablet PC, personal computer, laptop, digital music player, PMP (Portable Multimedia Player) and are available for all devices in which data can be received and sent through communication with the aforementioned mobile blood pressure device.

The aforementioned 119('H0) and doctor (720) can cope with the situation for the user quickly in case of caution or emergency status.

The aforementioned service server (980) can be set to store user's bio-signal and measured data through line and wireless communication. The service server (980) can be a server which handles the entire exercise, health and body of the user.

The aforementioned N terminal (730) can cope with the situation of the user in case of caution and emergency situations displayed on central processing part (900) or the aforementioned mobile terminal (700).

Figure 33 is a block diagram which indicates the process of bio-signal detection through bio-signal measurer illustrated in figure 27.

This will be explained by examining measurement of electrocardiogram by using bio-signal measurer of this invention. Connection line (201c) is connected to all sensor parts to provide electric connection between modules. This consists of 1 electrocardiogram (250L), which is contacted to carotid artery under neck of the left side of the user, and 2 electrocardiogram sensor part (500R), which is contacted to ear conch on the right side of the user. Therefore, it detects potential difference between two electrocardiogram sensors then sends the results to electrocardiogram analyzing part (505) of the bio-signal analyzing part (940).

The aforementioned 1 electrocardiogram sensor part (500L) and 2 electrocardiogram (500R) are equipped with body-connected ECG sensor. Further, the aforementioned 1 electrocardiogram sensor part (500L) extends from virtual body part (201a) with certain length and is attached to mask conjunction hole (116) and is contacted to carotid artery of neck on the left side. Further, the 2 electrocardiogram sensor (500R) can be connected to ear conch of the other side (that is to say, the opposite side of the area on which ECG sensor is installed). That is to say, if 1 electrocardiogram ECG sensor is installed on bio-signal measurer (200a) on the left side then contacted to carotid artery of neck, the 2 ECG sensor is installed on bio-signal measurer (200b) on the right side therefore can be contacted to ear conch (207). Through this, electrocardiogram sensors on the left and right sides become a pair and generates lead II(Lead II) to detect electrocardiogram signal.

The aforementioned electrocardiogram sensor part (550) consists of 1 or 3 EEG sensor (550a, 550b, 550c) and can be allocated on temporal region (550a) (temple) on the right side, skin in front(550b) of and behind ear (550c) (backside of ear), contact-type EEG sensor therefore detects electrocardiogram signal. The detected electrocardiogram signal is sent to electrocardiogram signal analysis part (555) of bio-signal analyzing part (940). Connection line (201c) connects 1 EEG sensor (550a) and ear clip (201b). Connection line (201c) can include data and power supply lines which can send data and power respectively. Connection line (201c) can be connected to 1 EEG sensor (550a) and ear clip connection port through connector connected to both edges. Such connection line can have flexibility and elasticity for the user to move freely during measurement of bio-signal.

Meanwhile, sensor is located on forehead part to gain electrocardiogram or safety signals in general. However, according to the example of operating this invention, this is located around temple, in front of and behind ear. In this case, safety and electrocardiogram signals can be obtained stably through differential amplification of output signals of the aforementioned sensors. EEG provides important information to brain function of the patient. Scalp EEG measures the sum of current exist in post-synapse of extracellular space caused by flow of ion from dendrites bonded by neurotransmitters or to inside dendrite. Brain wave is classified into four types according to the areas of brain activity and brain wave appears on electrocardiogram (EEG) are different from each other. Brain wave includes beta wave (13∼30Hz), alpha wave (8∼12Hz), theta wave(4∼7Hz) and delta wave (1∼3Hz). Brain wave of human shows different frequencies according to the body condition that he wave appears in awakening period is called beta wave. If body is relaxed when the patient goes to bed and feel sleepy, frequency of brain wave decreases little bit and become alpha wave. However, the alpha wave indicates that the patient is awake. In addition, if the person falls asleep, brain wave starts from theta to delta ones. If the person turns in to stable sleeping status, the patient turns into REM state in which theta and delta wave appear on regular basis. Sleeping and health statuses of the user can be figured out and predicted accurately taking into account the bio-signal features of electrocardiogram (EEG).

The aforementioned PPG sensor part (510) is installed on the aforementioned body part (201 a) and ear clip (201b). Further, light emitting and detecting parts are prepared to detect PPG signal and send it to PPG signal processing part (515) of bio-signal analyzing part (940). Light emitter is installed on a part of ear conch of sensor part (510) while light detector is installed on the other part of ear conch. That is to say, light emitter, to which sensor is contacted, is installed into ear conch. Further, the outside of ear conch is installed with light detector which detects light reflected or transmitted passing through human body. The whole figure is configured as transmission-type sensor structure.

In case of the aforementioned body temperature sensor part (520) body temperature sensor is installed on the aforementioned body part (201 a) and ear insert part (201 f). Further, it is contacted to inside of ear on the right side and detects body temperature signal. Such detected body temperature signal is sent to body temperature signal analyzing part (525) of bio-signal analyzing pat (940).

The aforementioned acceleration sensor part (530) is a method for measurement of activity that acceleration sensor is installed on body parts on the left and right sides (201a). Further, changing voltage of three axis including x, y and z axis are detected by using 3-axies acceleration sensor. The outputs of three axis sensed on the acceleration sensor part (530) is used for determining activity of the central processing part (900) which will be mentioned later.

Sound detection sensor is installed on the aforementioned left and right body parts (201a) in case of the aforementioned sound signal sensor part (540). Further, this is a sensor for detection of sounds generated by the user during sleep (single elderly or child patient etc.) including respiration, snoring, crying and sleep talking. This can detect at least one of the aforementioned respiration, snoring, sleep talking, which occur during sleep. Further, it can figure out respiration status of the aforementioned users taking into account interval, intensity of respiration and snoring.

The aforementioned body signal analysis part (940) boosts bio-signal received from sensor parts and eliminates noise to make the signal to be processed by the central processing part (900) that it can include filter which can eliminate noise occurred during measurement of bio-signal. The aforementioned bio-signal analysis part (940) is equipped with electrocardiogram analysis part (505), PPG signal analysis part (515), body temperature analysis part (525), acceleration signal analysis part (535), sound signal analysis part (545) and electroencephalogram signal analysis part (555).

Electrocardiogram signal analysis part (505) receives and boosts electrocardiogram signal detected from 2 electrocardiogram sensor part (505R) which is contacted to 1 electrocardiogram sensor part (500L) and ear conch (207), which are contacted to left carotid artery. Further, it eliminates noise.

Electrocardiogram signal analysis part (555) boosts electrocardiogram signal received from electrocardiogram signal sensor part (550) and eliminates noise. PPG signal analysis part (515) boosts PPG signal received from PPG sensor part (510) and eliminates noise. Body temperature signal analysis (525) boosts signal received from body temperature sensor part (520) and eliminates noise. Acceleration signal analysis part (535) eliminates noise from the signal received from acceleration sensor part (530). Sound signal analysis part (545) boosts sound signal received from sound sensing sensor part (540) and eliminates noise. The aforementioned A/D transformation part (920) transform analogue signal received from bio-signal analysis part (940) to digital signal and sends it to central processing part (900).

Figure 34 is a schematic diagram which explains the performance of central control part illustrated in figure 32.

Referring to figure 29, 30, or 34, for measurement of bio-signal of the user by using the aforementioned bio-signal measurer (200), the user needs to wear bio-signal measurer (200a) for left ear on left ear and wear bio-signal measurer (200b) for right ear on right ear. After that, the aforementioned SMARTMASK (10) needs to be worn on bio-signal measurer (200) then attachment hole (202) of bio-signal measurer needs to be attached on conjunction hole (116) of SMARTMASK (10). In this case, if button part (210) of bio-signal measurer (200a) on the left side is turned on, the operation power is sent to at least a sensor part (500L, 530, 540) from power part (270) and waits for sensing.
If button part (210) of bio-signal measurer (200b) on the right side is operated, power is sent to at least one sensor part (500R, 520, 510,
550) from power part (270) and measurement begins.

(S911) is the process of receiving bio-signal that it receives electrocardiogram signal, PPG signal, electroencephalogram signal, sound signal, body temperature signal, acceleration signal and store these in buffer.

(S912) is a process for comparing sizes of bio-signal. In this process, if the size of the bio-signal can be processed in the central ddd part (900) is compared with the stored area by comparing the received bio-signal to the minimum size. If the size is smaller than the minimum size for processing, boost rate of detected bio-signal increases (S913) and returns to the bio-signal size comparing (S912) process. Increasing bio-signal at this point increases boost rate in bio-signal analysis part 9940) or A/D transformation part (920) or the bio-signal data is received by using the boost rate which increased in bio-signal receiving process.

(S914) is bio-signal measurement process. If bio-signal received from bio-signal size comparing process (S912) is larger or the same with the minimum size, the signal is processed from electrocardiogram signal, PPG signal, electroencephalogram signal, sound signal and acceleration signal to detect bio parameters which includes heart rate.

(S915) is process for evaluation of health state, sleep state and exercise level that these states are processed from bio parameters including heart rate and electroencephalogram detected from bio-signal measurement process (S914) to calculate health state and exercise level parameters. In the process for evaluation of health state, sleep status and exercise level, exercise level parameter can be detected by using parameter including activity and so on.

(S916) is a process for evaluation of health state, sleep state and exercise state.
This process evaluates if the exercise parameter obtained from the process of evaluation of health state, sleep state and exercise level is out of the exercise standard stored. If the figure is out of exercise standard in the process of evaluation of health state, sleep state and exercise level, (S917) alarm control signal or display control signals are sent to the sound signal generator (930), user terminal (700), service server (980) and doctor (720) according to the level of risk situation.

(S918) is a process for selection of data storage. This evaluates if the measurement data is set to be sent to data service server. If the data is not set to be sent to service server, data is stored in data base part (960).

(S919) is a process for storage of data in service server. If it is set to store measurement data in computer in data storage selection process (S918), measurement data is stored in computer by using line and wireless communications.

(S920) is a process for evaluation of completion of measurement.
The user can set measurement completion flag by setting button part (210) or application part (800). If the completion flat is set, measurement is finished and if not, it returns to bio-signal receiving process (S911).

It is examined that various examples of operation of this invention will provide various important differences and merits for masks which use previous technologies.
The table 2 below compares previously noticed mask(s) with SMARTMASK (10) which has adherence power in this invention.
Table 2

**[Table 2]**

| | | |
|---|---|---|
| Classification | Simple structure which hides nose, mouth and chin (example of comparison) | 3-D structure which hides nose, mouth and chin (example of invention_ |
| Structure | Made of synthetic resin, spandex, non-woven fabric and cotton. Worn on mouth and nose. If the user wear it on both mouth and nose and use it for a long time, movement of uncomfortable mouth during uncomfortable respiration exercise and simple uncomfortable receiving design's anatomical death space occur. | |
| Interest point | Protection of respiratory tract, cold protection, UV block, design | Protection of respiratory tract, skin, measurement of health, sleep, exercise wearable, internet of things, bio-signal. |
| Moisturizing | None | Maintain moisture and temperature of skin and respiratory tract |
| Adherence | None | Integrated with skin |
| Friction of skin and mask | Serious | Integrated with skin |
| Sealing (haze of glasses) | Serious | Excellent sealing |
| Movement of mask | Serious | Never exist |
| Movement of chin (talking) | Uncomfortable | Free |

According to table 2, SMARTMASK (10) of this invention provides comfortable feeling of wearing during exercise and sleep. Further, it is transformed well according to the face size and line through excellent elasticity therefore is integrated with face skin.
Accordingly, bio-signal can be measured and measurement device or bio-sensor can be put on and off. Further, dry of skin is prevented and mask does not move. In addition, mouth movement can be accepted and comfortable respiration is possible therefore children, the elderly as well as patient with respiratory disease can wear this. Further, symptom is alleviated and treated if patient with respiratory diseases, snoring and rhinitis.

In contrast, pre-existing masks pesters the user and severe pressure is added on ear part. Further, vision of the glasses user is blurred and the design is not beautiful. Anatomical death space occurs due to wearing of mask as well as uncomfortable respiration occurs therefore this mask can be rather bad for pregnant women patient with respiration disease

SMARTMASK (10) is worn on face skin as illustrated in figure 16. Firstly, the user needs to retain each of left, right and upper band parts (121) by using both hands (stage 1) then put front chin to chin support part (114), which is "location featured part" (stage 2). After that, pull left, right and upper band materials (121), which have enough availability for covering head, to upper part and fix it on the top of the head (210)) (stage 3). Further, fix the left, right and lower band material (122) to back head (220) (stage 4). If so, SMARTMASK (10) is worn on the face of the user simply and easily.

In this case, left and right contact parts (111c) are contacted to left and right chines, front chin, skin of neck right under left and right ears, temple, which are fundamental parts, and are integrated. Overall, the state is maintained while the user is lifting to upper part or toward horizontal direction.

At this time, nose and mouth of the user (113) protrude through opening hole (113) while ears are covered by left and right cover parts (128). Further, chin is fixed by chin support part (114) therefore automatic opening of mouth is prevented. Accordingly, nose is separated from external environment in upper internal and/ or small internal body part of the cover part (139) therefore the user can in hale heated and humidified clean air through filter part (139). Further, mouth is ventilated with external environment therefore the user can talk freely.

To measure bio-signal of the user by using the aforementioned bio-signal measurer (200), the user needs to wear bio-signal (200a) on left ear and wear right bio-signal measurer (200b) on right ear. Then the aforementioned SMARTMASK needs to be worn covering bio-signal measurer (200) (refer to figure 24, 25, 27, 28, 29 and 30). After that, attachment hole of left and right bio-signal measurer (200a, 200b) needs to be combined with conjunction hole (116) of SMARTMASK (10). Then button part (210) needs to be turned on for measurement of bio-signal when left and right sensors are contacted closely to skin (figure 31).

The aforementioned SMARTMASK (10) is worn on face during sleep in the night and inhales effectively heated and humidified clean air therefore is beneficial for health and face can be adjusted. At the same time, bio-signal can be accurately and simply measured.

### [2.1] 2 Implementation Form

Figure 35 shows wearing state of SMARTMASK in the 2 implementation example.
Figure 36 shows another wearing state of SMARTMASK of 2 implementation example.
In 2 implementation example of this invention, SMARTMASK is specified as figure signal 20.

As shown in figure 35 or 36, SMARTMASK (20) in 2 implementation example according to the example of right way of implementation of this invention, left and right cover parts (128) are not formed in 1 example on the aforementioned left and right contact parts (211). Therefore, basic structure and function are the same with the 1 SMARTMASK (10) in the aforementioned implementation example of this invention apart from the fact that length from outer part of opening hole to left and right fixing parts' upper guard of upper outline of the aforementioned left and right contact parts (211) decreases, the length of lower outline (211b) decreases from chin support part (214d) to left and right fixing boundary (211e) (refer to figure 39), shape, structure, size and thickness of left and right fixing parts (212) and accordingly living hinge is additionally included, similar material number is attached to similar components, detailed description and duplicated explanation are omitted.

The aforementioned SMARTMASK (20) is formed with combination of opening hole (213), which is opened so that the user's nose to protrude, living hinge (218), which protrude for mouth to protrude, support part, on which lower chin is fixed (214), contact part (211c), which is contacted closely to face skin and has skin conformability, left and right upper/ lower outline (211a, 211b). Further it is integrated with contact part (211), the aforementioned left and right contact parts (211) on which bio-signal measurer (200) can be configured with putting off and on measurement. In addition, it is also configured to be put on and off from left and right fixing parts (212), the aforementioned left and right fixing parts (212), 1, 2 and 3 attachment (212c, 212d and 212e) so that it is toward left and right contact parts (211). In addition, it is configured to be put on and off from left and right earring parts (124), the aforementioned left and right fixing parts (212), 1, 2 and 3 attachment parts (212c, 212d, 212e) for wearing SMARTMASK (20) on face. Further, it is combined with the aforementioned upper opening hole's boarder (213a) so that it is not contacted to nose which protrudes through left and right upper/ lower band materials (221, 222) and the aforementioned opening hole (213) for ventilation of air through separately prepared filter part (239) indirectly only to seal around nose by steel part, upper boundary, expansion material, wall body and lung part. Inner part consists of upper part (233), in which internal air space only nose is allocated, and opened space which covers mouth without contacting to protruded mouth through the aforementioned living hinge (218) and for ventilation to external environment is configured including cover part (230) which includes front cover (234) inside the opened space

Here, SMARTMASK (20) has been mentioned but left and right covers (128) are not configured on left and right contact parts (111) unlike SMARTMASK of 1 implementation example.
In such configuration, length from opening hole (213) to left and right fixing part (212) boundary (area in which left and right fixing parts are connected) is minimized of SMARTMASK (20) of 2 implementation example therefore the aforementioned fixing part (212) is located in front of ear skin of the user. The ears of the user (207) are worn as shown in figure 35 and 36 even if it is exposed to external space. At this time, especially the length from opening hole (213) to chin support lower part 9214d), which is the vertical length, is the same with SMARTMASK (10) of the 1 implementation example.

Figure 39 is a diagram for explaining the size of left and right contact parts.

As illustrated in figure 39, such left and right contact parts (211) are manufactured taking into account the average face size and length of the user. The length from the upper part (213b) of opening hole to left and right upper boundaries (21If) needs to be set between 40mm and 100mm. Further, the length from fixed upper part of boundary of opening hole (213b) to lower part of chin support part (214d) need to be about 70mm and 140mm. In addition, the length from lower part of chin support part (214d) to lower outline (211d) needs to be about 20mm or 50mm. The length from boundary of lower outline (21 Id) to left and right lower boundary (211e) needs to be about 30mm or 100mm. Finally, length from the fixed upper boundary (21 If) to fixed lower left and right parts needs to be about 40mm or 120mm.

At this time, left and right outlines (211a, 211b) are formed with more than 80% and less than 100% of reducing rate for the size of user through upper part of (213b) of opening hole's boundary to boundary of left and right fixed parts (211f) so that it reaches near to 100% of face size when pulling power is applied. Further, the size can increase by 120% of the face size.
Of course, the aforementioned left and right outlines (211a, 211b) have excellent elasticity therefore can be increased more.

Meanwhile, it is recommended to set the size with more than 90% and less than 100% of reducing rate regarding the figure from upper boundary of opening hole (213b) to lower part of chin support (214d) to set the size with nearly 100% of body figure (100%) for wearing SMARTMASK. This is almost the same length for wearing or not wearing SMARTMASK (20).

The reason for configuring vertical and horizontal reducing ratio differently is for left and right contact parts (211) to be increased toward top of the head, which is the arrow direction illustrated in figure 39, when wearing SMARTMASK (20). If so, left and right contact parts (211) are contacted to face skin closely as well as face skin can be lifted from down to upside.

In figure 35, the aforementioned left and right contact parts (211) are asymmetry according to lower part from upper part centered at opening hole (213) chin support part (214) located in the central part. This implementation form is the same for both left and right sides. If the user wears SMARTMASK (20), the aforementioned left and right contact parts skin around nose and mouth of the user, left and right chins. That is to say, it is not worn on the entire face. The opening hole (213), living hinge (218), chin support part (214), left and right contact parts (211c), conjunction hole (not shown) are the same with 1 SMARTMASK (10) implementation example.

In specific, examining by referring to figure 35, the aforementioned upper outline (211a) configures boundary of opening hole (213a) while the other edge is integrally connected to upper part of fixing part (212a) and is located around sideburns following the skin under eyes of the user. The lower outline (211b) is connected to lower part of chin support part (214d) while the other edge is integrally connected to left and right fixation parts and is located in front of ear covering the user's chin. In this configuration, contact areas of left and right contact parts (211c) are minimized to reduce contact area between face skin and left and right contact parts (211) of the user. In case of such configuration and arrangement, left and right fixation parts (212) and contact parts (211) are contacted to face skin in front of ear adding pressure therefore are contacted closely to face skin. Further, function as sealed surface, in which even little air leakage is not allowed, is added as well as inconvenience caused by movement and friction do to occur.

Figure 38 a) is a diagram which is for explaining left and right fixing parts.

According to figure 38 a), elasticity of left and right contact parts (211) of 2 implementation examples increases by left and right contact parts (111) of the 1 implementation example. The reason is that areas of left and right contact parts (211c) of 2 implementation examples are smaller than that of left and right contact parts (111c) of 1 implementation example. In addition, the left and right fixation parts, which will be mentioned later, (212) have "Z" shape. Configuration of such left and right contact parts (211c) and fixation parts (212) minimizes contact of face skin with left and right contact parts (211c) as well as improves adhesion power.

The SMARTMASK (20) of 2 implementation example is configured including fixation part (212), which includes upper (212a) and lower parts (212b), which are extended integrally apart from internal and external areas of the upper outline (211a) of the aforementioned left and right contact parts, lower outline (21 1b), left and right contact parts (211c). The aforementioned left and right fixation parts (212) has larger width (W) and thickness (T) of contact part compared to left and right contact parts. Further, these parts have smaller elasticity and flexibility compared to those of left and right contact parts (211). In specific, this can be configured with 50mm or 300mm of thickness and 3mm or 6mm of width.

Like this, it minimizes the contact between the left-right contacts (211 c) and the left-right fixture (212). At the same time, it maintains the three-dimensional shape not to make gaps between the upper (211a) and the lower surfaces (211b).

As illustrated in the diagram 35, the upper (212a), outer side of the left-right fixture (212) can be placed separately with fixed gap, and the upper (212a), outer side of the left-right fixture (212) can be mounted and detached with the left-right, upper/lower band (221, 222) and the left-right hooks for ears (124). It makes the upper and lower side of the left-right, upper/lower band (221, 222) and the left-right hooks for ears (124) be placed separately with fixed gap. Therefore, when there is pulling force in here, the left-right contacts (211) do not bend but keep their shape to make it more closely to a user's skin.

As illustrated in the diagram 38 a), when looking side of the left-right fixture (212), giving that there is the line L1 connecting the upper (212a) and outer side of the left-right fixture (212), it is appropriate to shape about "L" by bending them towards a user's nose.

The diagram 40 is the state wearing the SMARTMASK formed with another left-right fixture.

The left-right fixture (212) can be formed into another shape. For example, as illustrated in the diagram 38 cc) or 38 d), the middle point of the upper (212a) and lower (212b) side can make "<" shape, or "(" which were bent more smoothly. For example, it is same with the illustration 40. In illustration 40, the left-right contacts (211) is shaped fitting into the left-right fixture (212).

The left-right fixture (212) can further include living hinge (219). As illustrated in the diagram 36 a), living hinge (219) in formed on the point where the upper (212a) and lower (212b) side meet. By constructing this way, living hinge above (291) can be the standard to improve the upper and lower side (212a, 212b). Also, the upper and lower side (212a, 212b) can be improved comfortably by living hinge (219).

Therefore, it is appropriate to shape with thickness and width thinner than the left-right fixture (212) to ensure its elasticity. Specifically, It is formed by thickness from 50 mm to 150 mm and curvature which have radius from 10 mm to 20 mm. This living hinge above (219) can increase its length up and down corresponding to the pressure just by a finger or the force very small. In other words, by pulling the upper (212a) and the lower (212b) up and down, both can be easily increased. For example, as illustrated in the diagram 38 a), the angle for both is small, based on the living hinge (219). As illustrated in the diagram 38 b), the angle for both is increased up and down, based on the living hinge (219), which is relatively bigger. Surely, the living hinge (219) above may not be formed.

The diagram 37 is another state wearing the SMARTMASK in the example 2.

Explaining with the diagram 37, the upper (212a), outer side of the left-right fixture (212) is formed with the first attachment (212c) and the second attachment (212d) to improve the contact between the left-right contacts (212) and the SMARTMASK (20) or to attach the left-right, upper/lower band (221, 222) and the left-right hooks for ears easily. For the outer side of the lower (212b), it is formed with the third attachment (212e).

At this point, considering the ease of installation for the left-right and upper/lower band (221, 222) and the left-right hooks for ears (124), the upper and lower attachments (212c, 212d, 212e) is desirable to have extended structure for the upper (212a) and the lower (212b). Therefore, it makes easy for everyone to mount and detach the left-right hooks for ears (124) to the upper and lower attachments (212c, 212d, 212e). The attachments (212c, 212d, 212e) and the fixture (217) has the same structure and function with the attachment (112c) and the fixture (117) explained in the example 1 for SMARTMASK (10), so the details for them are omitted.

As illustrated in the diagram 35, 39, the left-right hooks for ears (124) will be placed without hanging on ears when wearing SMARTMASK (20) by using the left-right and upper/lower band (221, 222). The left-right and upper/lower band (222) can be connected into the first attachment (212c) by the fixture (217) that can be placed by moving up and down on the back of the head (220). Also, the left-right and upper/lower band (222) can be connected into the third attachment (212e) by the fixture (217) that can be placed on the back of the head. As a result, you can wear SMARTMASK (20) comfortably and safely by the left-right and upper/lower band (221, 222) which can be placed on the back of a user's head or top of the head.

As illustrated in the diagram 35, the left-right and upper/lower band (221, 222) is formed to be detached from the upper and lower attachments (212c, 212d, 212e). It can prevent pressing hair by the left-right and upper/lower band (221, 222). By using the left-right hooks for ears (124), it is possible to wear SMARTMASK (20) more comfortably.

The first attachment (212c) above is appropriate to be formed straight to the above. It can hold the legs of glasses when wearing them, which prevent the wasteful movements of the glasses.

Also, the left-right hooks for ears (124) is formed to be detached from the upper and lower attachments (212c, 212d, 212e) by the fixture (217). As illustrated in the diagram 35, the upper side of the left-right hooks for ears (222j) are connected with the upper side of the left-right fixture (212a), the second attachment (212d), the lower side (222k) is connected with the upper side (212b) and the third attachment (212e). It makes easy for everyone to mount and detach the left-right hooks for ears (124) to the upper and lower attachments (212c, 212d, 212e). The upper and lower attachments (212c, 212d, 212e) and the fixture (217) has the same structure and function with the attachment (112c) and the fixture (117), so the details for them are omitted.

Also, as mentioned above, the left-right hooks for ears (124) will be placed in front of the left and right ears when wearing SMARTMASK (20). In this, if the left-right hooks for ears (124) is pulled, the left-right hooks for ears (124) and the left-right contacts are increased, which leads to make possible to hanger the left-right hooks for ears (124) on a user's ears.
It takes the force to pull with increasing the left-right contacts (111) and the left-right hooks for ears (124) to distribute it over the face, which minimizes the pressure. Also, it makes a user's facial skin and the left-right contacts (111) more closely contacted.

This left-right hooks for ears (124) is appropriate to be made with silicone which is adaptive to skin to hang it on the left and right ears. Also, it is necessary to have the size to take a user's ears and the circle shapes with about 150 mm to 400 mm of thickness. Plus, it should be made to have flexibility and elasticity. If formed as above, the left-right hooks for ears (124) of the circle shape can be increased just by touch of a finger. It means that it can be elastically increased into 700% or 1000% without damages by pulling it with small power. By releasing the pressure, it is moved to the original position.

The diagram 38 e) is to explain the left-right hooks for ears.

With the diagram 38 e), the left-right hooks for ears (124) above include a hook on the one side (222). It is inserted in the left-right hooks for ears (124) to minimize the pressure focusing on both ears. Also, it is formed to easily mount and detach by connecting it in the center of the back of the head with pulling each other. The hooks (222) above has the to insert the left-right hooks for ears (124) internally, which has shape of 20 mm to 40 mm length and 5 mm to 8 mm look.

Also, the left hook (222L) has the through the inside (222d) and the arm hook with shape of (222c) connected with the above. It makes the left hook for ears (124L) inserted inside the (222d), which leads left ear to contact with the left hook (222L). So does The right hook for ears (222R).

Therefore, as the left-right hook (222L, 222R) releases the pressure focusing on ears by contacting sides of a user, which make the user more comfortable. Also, the left-right hooks for ears (124) and the left-right contacts (211) have enough elasticity that can be held by using the hooks (222) on the back of the head. And the hook (222) is easy to be mounted and detached by hands. Surely, it is composed of various materials and structures to hold it tightly and safely.

When wearing SMARTMASK (2), and when the left-right and upper/lower band (221, 222) is pulled, it is desirable to increase the left-right contacts (211) and the left-right and upper/lower band (221, 222) at the same time. As the bands (221, 222) and the contacts (211) are increased, pressure will be distributed all over the area, not on the single point, which leads to minimize the pressure on the face and to transform it more flexibly that user's facial skin and the left-right contacts (211) are contacted more closely.

the left-right and upper/lower band (221, 222) above should be formed with flexibility and elasticity. Also, it should be formed with soft silicone whether it's transparent or not to have similar elasticity with the left-right contacts (211). It needs to be formed with It is appropriate to have soft and long strap for 5 mm - 12 mm of width and 100 - 200 mm of thickness, though they are not defined specifically yet, to put it on the head.

Also, the left-right and upper/lower band (221, 222) has the same structure and function with the left-right and upper/lower band in the SMARTMASK (10) of the example 1 (121, 122). So, it is sure for attachments (212c, 212d, 212e) and fixture (217) to be formed to move up and down. Also, the left-right and upper/lower band (221, 222) should be combined by separate clamps. It is possible to hold tight without clamps (see the diagram 39). Also, there are the unit scales on the surface, which prevent slippery.

The structure and function of the left-right and upper/lower band (221, 222), in particular, are to prevent irritation of taking off or moving while in sleep, working out and communication by putting the left-right and lower band (222) on the back of the head and putting the left-right and upper band (221) on the top of the head - both against the left-right contacts (211).
By using the concept of Triangulation, it ensures that SMARTMASK (20) closely contacts to skin.

Also, the left-right and upper/lower band (221, 222) above are mounted with the measurement unit for vital signal (200), which can be used as a band to fix the unit when measuring the vital signal.

Also, the cover (23) has the same function with those of the cover (130) in the SMARTMASK (10) of the example 1. Multiple joint grooves (116) can be made in the left-right contacts (211c) and multiple essential parts (239) can be composed. The joint (116) above can be mounted and detached with the measurement unit for vital signal (200), providing you with the information for your health.

Therefore, the SMARTMASK (20) in the example 2 is closely contacted with a user's skin, so the user can use it while sleeping, working, daily life, in the work, protecting the respiratory system like preventing fine dust, as a gas mask.

The cover (230) above consisting SMARTMASK (20) in the second example was explained that it is produced with silicone, but it can be produced with sheet material of one or multiple layers. Also, to enhance the filtering, it can be layered for more than two sheet materials and be partly connected. For example, more than the inner sheet materials are in between two outer sheet materials, and the materials such as filters can be in the outer sheet materials. As notified earlier, these materiel include felt such as spunlace, spunbond, meltblown, AMaid, and various fabric, knitting, but the material of the cover (230) is not limited.

Also, the left-right contacts (211), the left-right hooks for ears (140), left-right and upper/lower band (221, 222) can be formed with sheet materials of one or multiple layers and can be layered for more than two sheet materials and be partly connected. As notified earlier, these materiel include felt such as spunlace, spunbond, meltblown, AMaid, and various fabric, knitting, but the material of SMARTMASK (20) is not limited. Also, the outside of SMARTMASK (20) may have patterns or printing for appearance.

### 2 3/4 The third conduct type

The diagram 19 is to show the SMARTMASK which does not have the front cover according to another example. Though the upper front cover (134) is not formed, the structure and function of the top of the upper cover (133), the left-right contacts (111), the left-right fixture (112), the left-right lover band (121, 122) are same with those of SMARTMASK (10) shown in the first example. So, the detailed and duplicated explanation is omitted.

As illustrated in the diagram 19, the lower inner side or outer side of the top wall (131) contains the cutting line (not in the diagram). If you cut it following the above cut line, the user's mouth and the lower chin are exposed to outside. SMARTMASK (10) without this front cove becomes smaller, which can be used for indoor, medical, sleep since it becomes portable and easy to wear with its simple structure.

### [2.3] the forth shape to conduct

The diagram 41 does not show when a user wears SMARTMASK according to another example.

SMARTMASK (10) following this invention can be formed with cutting it into about "n" shape from the upper left-right contacts (111) to the left-right contacts (liic), the lower surface (nib), and can be worn as illustrated in the diagram 41. Except that the upper left-right contacts (111c) is reduced its size, this invention's basic structure and functions are similar to those of SMARTMASK (10), so the similar components have the similar numbers without detailed and duplicated explanations.

As the left-right contacts {11c} is not formed to cover all over the facial skin but only the sides, with the smaller fit cut. As the flexibility of the left-right contacts (111c) improves as the cutting part, which make it more comfortable.

For this reason, if children or patients who feel cramped wear it, or if workers in the factory wear it, or if it has to measure the vital signal, if it should be worn while a user is in sleep, SMARTMASK (10) is much more comfortable to wear.

Though this invention's shape is explained with diagrams, its detailed construction is not limited to its shape, the changes or modifications are also included if they are out of the standard. For example, the cut part of the left-right contacts (11c) is not limited its detailed shapes into the diagrams.

Also, SMARTMASK (10) is built with silicone which can be used repeatedly and semi-permanently. Since it is produced very simple with a mold, cost is affordable, and it's possible to launch in the market. Therefore, it can reduce the cost much more than the existing mask which is a disposable made with felt or fabric.

The range of this invention includes but not be limited to the examples above. Still, it is possible for the technicians in the industries can apply it within its range, based on this invention.

### Industrial Availability

This invention is SMARTMASK, which is formed to maintain the skin's adaptability over time to protect facial skin from sunlight, wind, cold, pollutant in outside while protecting the respiratory system of a user from polluted air from outside. Also, while the user is in sleep, it supports the lower chin which is dragged down by relaxation and gravity not to make mouth open, which avoid mouth breathing while leading to nose breathing. It has humidification and warming function to ease allergic symptoms and inflammation inside mouth. Also, it helps the user to breath in more comfortable while breathing out CO2 generated from body to the outside quickly, which prevents anatomical dead space - the SMARTMASK useful in health.

## Claims

1. This invention includes the hole of the opening of the mouth (113) where the user's nose comes out in the front part, living hinge(119) where the mouth comes out, chin holding part (114) that adjusts the inlet of the bottom of the chin, contact left-right contact surface (111c) that has adaptability to the skin by closely touching the skin, left-right ear cover (128) that covers both left and right ears, and also the left-right top/bottom contour part. The invention also includes left-right contact part that can be composed of attachable/detachable type for the measuring device; The invention is also composed as all-in-one type with the left-right contact part (111) mentioned above. Including the top part (112a) and the bottom part (112b), there is a left-right adjustment part (112) that is composed to withstand the left-right contact part;
The invention is also composed to attach/detach to the top/bottom attachment part of the left-right adjustment part. The invention also has upper/lower band sub-material (121, 122) to wear the SMARTMASK (10) on the face; and
the nose part is closed to the wall part, cover part, expansion sub-material, upper frame, and contact part to be combined with the opening hole frame (113a) so that the air can be indirectly ventilated with the external environment only through the filter part (139) that is prepared separately without having to contact the nose through the opening hole (113). The mouth is covered without touching the mouth through the living hinge (119) and upper part (113) that is composed of small internal gas space (133d) that is arranged for only the nose by closing the nose. For the direct ventilation with the external environment, the opened space (134d) and covering part (130) that includes the front cover (134) are composed for the SMARTMASK (10).

2. The invention is also composed of left-right contact surface (211 c) and left-right upper/lower contour part (211 a, 211 b) that has adaptability to the skin by being closely contacted to the skin, and it also has chin holder (215) that adjusts the lower chin and the living hinge (218) that is opened so that the mouth can stick out. Lastly, it has the opening hole (213) for the nose to stick out. The invention also has left-right contact part (211) that is composed to attach/detach the body signal measuring device (200);
The left-right contact part (211) is composed as a whole with the extension, left-right ear part (224) to wear the SMARTMASK (20) on the face to be composed for attachment/detachment with 1^{st} attachment part, 2^{nd} attachment part, 3^{rd} attachment part (212c, 212d, 212e) of the left-right adjustment part (212); left-right adjustment part (212) composed to include lower part (212b), living hinge (219), and upper part (212a) to withstand the left-right contact part (211);
Left-right upper/lower band sub-material (211,222) to wear the SMARTMASK (20) on the face with composition for attachment/detachment with left-right adjustment part (212), 1^{st} attachment part, 2^{nd} attachment part, and 3^{rd} attachment part (212c, 212d, 212e); The nose part is closed due to the contact hinged part, upper frame, expansion sub-material, wall part, and shield combined with the filter part (239) that is separately prepared so that the opening hole (213) does not contact the nose that comes out. The small internal gas space arranged for only the nose does not touch the mouth that comes out through the living hinge (219) and the upper part (233) while covering the mouth for direct ventilation with the external environment. The opened space is composed of the cover part (230) that includes the front cover (234) which is formed inside.

3. For Section 1 and/or Section 2,
The SMARTMASK (10,20) has the characteristic of being composed of average thickness of 10 µmo 40 µm overall for the left-right contact surfaces (111c, 211 c) For Section 1 and/or Section 2,

4. The SMARTMASK (10, 20) has the characteristic of being manufactured with soft silicon with hardness from 20 degrees to 30 degrees for the left-right contact surfaces.

5. For Section 1 and/or Section 2,
The SMARTMASK (10, 20) has the characteristic of having gas penetrability for the left-right contact surfaces

6. For Section 1 and/or Section 2,
The SMARTMASK (10, 20) has the characteristic of having impermeableness for the left-right contact surfaces.

7. For section 1 and/or Section 2,
The SMARTMASK (10, 20) has the characteristic of having impermeableness for ultraviolet rays, wind, and liquid for the left-right contact surfaces.

8. For Section 1 and/or Section 2,
The SMARTMASK (10, 20) has the characteristic of having penetrability for humid for the left-right contact surfaces.

9. For Section 1 and.or Section 2,
The SMARTMASK (10, 20) has the characteristic of having elongation ratio (%) of 100∼1000 for the left-right contact surfaces.

10. For Section 1 and/or Section 2,
The SMARTMASK (10, 20) has the characteristic of being composed to have adhesive power in the unilateral surface and the inner surface to be adhered to the skin for the left-right contact surfaces.

11. For Section 1 and/or Section 2,
The SMARTMASK (10, 20) has the characteristic of being composed to have adhesive power in the most inner part so that the left-right contact surface does not come up.

12. For Section 1 and/or Section 2,
The SMARTMASK (10, 20) has the characteristic of including the uneven areas to the unilateral part or the inner surface for the left-right contact surfaces of this invention.

13. For Section land/or Section 2,
The SMARTMASK (10, 20) has the characteristic of being formed as transparent, half-transparent, and/or opaque for the left-right contact surfaces.

14. For Section 1 and/or Section 2,
The SMARTMASK (10, 20) has the characteristic of being composed for compatibility that can be placed in a pouch and/or pocket by making the size of the SMARTMASK smaller when the mask is not worn. On the other hand, the SMARTMASK is an ergonomic design that can change according to the formation of the face when wearing the SMARTMASK.

15. For Section 1,
The SMARTMASK (10, 20) has the characteristic of being composed to be fully worn to the skin by including left and right ear part, left and right ceratoid, lower neck part below the chin, and the chin by including left-right temporal, left and right eyes, and the skin below the left and right eyes. However, it is not worn on the head, both eyes, and forehead.

16. For Section 2,
The SMARTMASK (10, 20) has the characteristic of being composed to be fully worn to the skin by including left and right ear part, left and right ceratoid, lower neck part below the chin, and the chin by including left-right temporal, left and right eyes, and the skin below the left and right eyes. However, it is not worn on the head, both eyes, and forehead.

17. For Section 1,
For the upper left-right contact part (111), the SMARTMASK (10) has the characteristic of being composed of 70mm∼140mm from the upper boundary point of the left-right adjustment part (111f) to the lower boundary point of the left-right adjustment part (111e). From the lower contour part boundary point (111d) to the lower boundary point of the left-right adjustment part (111e) is from 30mm ∼ 140mm. The lower surface of the chin support part (114d) to the lower contour part boundary point (111d) is around 20mm∼50mm. The frame upper part of the opening hole (113b) to the lower surface of the chin support part (114d) is around 70mm∼140mm. From the frame upper part of the opening hole (113b) to the left-right adjustment part's upper boundary part (111f) is from 70mm to 150mm.

18. For Section 2,
For the left-right contact part, the SMARTMASK (20) has the characteristic of being composed of 40mm to 100mm from the frame upper part of the opening hole (213b) to the left-right adjustment part's upper boundary point (211f); From the frame upper part of the opening hole (213b) to the lower surface of the chin support part (214d) is around 70mm ∼ 140mm; From the lower surface of the chin support part (214d) to the lower contour part's boundary point (211d) is from 20mm ∼ 50mm; From the lower contour part's boundary point (211 d) to the left-right adjustment part's lower boundary point (211e) is from 30mm ∼ 100mm; From the left-right adjustment part's upper boundary point (211f) to the left-right adjustment part's lower boundary point (211 e) is from 40mm ∼ 120mm.

19. For Section 1,
For the upper contour part (111a), the SMARTMASK (10) has the characteristic of being composed to be arranged according to the skin below the eyes of the user and the temporal to be formed in high position so that the mask can be moved to the upper part of the adjustment part (112a) from the frame upper part of the opening hole (113b) by forming extension to the adjustment upper part (112a) for the other layer, and the single layer forms extension to the frame upper part of the opening hole (113b).

20. For Section 1 and Section 2,
The SMARTMASK (10, 20) has the characteristic of being composed where the upper contour part is moved to the upper part so that the user's eyes are not covered with the method that is similar to the wire of a bra, and the skin below the user's eyes are supported towards the upper direction so that the mask is positioned to the skin that is below the eyes.

21. For Section 1,
The upper contour part (111a) is formed with elbow-type curve part (111d) in the unilateral surface. When the SMARTMASK (10) is worn, this elbow-type curve part (111d) covers the user's eyes.

22. For Section 1,
The upper contour part (111a) is formed with the elbow-type curve part (111d) to the unilateral surface. When the SMARTMASK (10) is worn, the elbow-type curve part (111d) is hung to the temporal from the skin below the eyes where the left-right contact surfaces (111c) are closely contacted.

23. For Section 1 and/or Section 2,
The SMARTMASK (10, 20) has the characteristic of forming 50µm to 150 µm of the thickness for the left-right upper/lower contour parts (111a, 111b, 211 a, 211 b).

24. For Section 1 and/or Section 2,
The SMARTMASK (10, 20) has the characteristic of forming 10µm ∼ 140µm thicker than the left-right contact surfaces mentioned above so that the left-right upper/lower contour parts (111a, 111b, 211 a, 211 b) can function to prevent damage of the left-right contact parts (111,211).

25. For Section 1 and/or Section 2,
The SMARTMASK (10, 20) has the characteristic of being formed from 1 ∼ 10mm for the width of the left-right upper/lower contour parts (111a, 111b, 211 a, 211 b).

26. For Section 1 and/or Section 2,
The SMARTMASK (10,20) has the characteristic of expanding 100%∼120% for the body measurement ratio when wearing the mask, and it can be composed from above 80% to below 100% for the body measurement ratio when formed from the single end to the other end for the left-right upper/lower contour part (111a, 111b, 211a, 211b).

27. For Section 1,
The SMARTMASK (10) has the characteristic of being composed with the lower contour part (111b) covering the neck skin below the left and right ears when wearing the SMARTMASK (10), and the single end forms extension to the lower part of the chin support part (114d). The other end forms extension as a whole to the adjustment part's lower end (112b) so that the mask can be moved from the lower part of the chin support part (114d) to the adjustment part's lower end (112b).

28. For Section 1 and/or Section 2,
The SMARTMASK (10, 20) has the characteristic where the left-right contact parts (111, 211) can change dimensionally according to the structure of the face, which is different for every user, according to the power of pulling the left/right/upper/lower band sub-material. Without any space comes up, the mask can be worn by being closely joined to the skin.

29. For Section 1 and/or Section 2,
The SMARTMASK (10, 20) has the characteristic where the left-right contact parts (111, 211) include various combined holes to attach/detach at least one among the bio signal measurement device, sensor, and bio electrode.

30. For Section 1,
The SMARTMASK (10) has the characteristic where the left-right ear cover part (128) includes various combined holes to attach/detach at least one among the bio signal measurement device, sensor, and bio electrode.

31. For Section 1,
The SMARTMASK (10) has the characteristic where the left-right ear cover part (128) includes various opening holes for ventilation.

32. For Section 1,
The SMARTMASK (10) has the characteristic where the left-right ear cover (128) is composed of sticking out type at a certain height in the left-right contact part (111) when the SMARTMASK (10) is not worn. In the case of wearing the SMARTMASK (10), the power of pulling is implemented to the left-right ear cover part (128) so that the mask can come closer to the left and right ears.

33. For Section 1,
The SMARTMASK (10) has the characteristic where the sound device that is equipped to the SMARTMASK (10) does not fall off from the ears with the left-right cover part (128) structure that adjusts the sound device due to the combined hole.

34. For Section 1,
The SMARTMASK (10) has the characteristic where the sound device that is equipped to the SMARTMASK (10) does not fall off from the ears with the left-right ear cover part (128) structure that covers the sound device.

35. For Section 1,
The SMARTMASK (10) has the characteristic of including the left-right ear cover part (128) structure that that adjusts the bio signal measuring device due to the combined hole so that the bio signal measuring device does not fall off from the ears while the bio signal measuring device is put on to the ears when wearing the SMARTMASK (10).

36. For Section 1,
The SMARTMASK (10) has the characteristic of including the left-right ear cover part (128) structure that covers the bio signal measuring device so that the bio signal measuring device does not fall off from the ears while the bio signal measuring device is put on to the ears when wearing the SMARTMASK (10).

37. For Section 1 and/or Section 2,
The SMARTMASK (10, 20) has the characteristic of forming living hinge on the lower part and forming opening on the upper part so that the nose and mouth can both come out without having to contact the opening hole for the opening hole (113,213), and it is also formed as "U" type including the opening hole frame (113a, 213a) on both left and right.

38. For Section 1 and/or Section 2,
The SMARTMASK (10, 20) has the characteristic where the upper opening hole frame (113a, 213a) has the length of 30mm to 70mm to be combined with the upper frame of the covering part, and the width is around 5mm ∼ 12mm. The thickness is composed to be around 50mm to 150mm, and the nose is not contacted through the opening hole. The nose is set to the center and parallel to the left and right sides.

39. For Section 1 and/or Section 2,
The SMARTMASK (10, 20) has the characteristic where the living hinge (119, 218) has the thickness of 50 µm∼ 150 µm with radius of 20 µm∼30 µm. The overall form is composed as "C" type. The user's mouth is against the mask to the lower left-right parts of the opening hole (113,213) for arrangement.

40. For Section 1 and/or Section 2,
The SMARTMASK (10, 20) has the characteristic of being composed so that the living hinge (119, 218) faces lower left and right sides of the opening hole (113,213) and so that the SMARTMASK allows the movement of the chin.

41. For Section 1 and/or Section 2
The SMARTMASK (10, 20) has the characteristic of being composed so that the living hinge (119, 218) can display enough flexibility for the change of the movement of the mouth.

42. For Section 1 and/or Section 2
The SMARTMASK (10, 20) has the characteristic of being composed so that the living hinge (119,218) is arranged to the left and right side by withstanding the left and right side of the lips when wearing the SMARTMASK.

43. For Section land/or Section 2,
The SMARTMASK (10, 20) has the characteristic of being composed so that the chin support part (114, 214) can support the chin by naturally allowing the lip to not open. On the other hand, when the user unintentionally opens the mouth, the mask expands according to the movement of the mouth to allow the movement of the chin flexibly.

44. For Section 1 and/or Section 2,
The SMARTMASK (10, 20) has the characteristic where the chin support part (114, 214) becomes the standard of wearing the SMARTMASK, and it has the characteristic of executing the role as "Special part of Location' while being composed in the lower center for the SMARTMASK (10, 20).

45. For Section 1 and/or Section 2,
The SMARTMASK (10, 20) has the characteristic of being formed where the chin support part (114,214) is in the hole at a certain depth as the standard for wearing the SMARTMASK (10, 20) while the chin is adjusted to the chin support part.

46. For Section 1 and/or Section 2,
The SMARTMASK (10, 20) has the characteristic of being composed so that the chin support part (114, 214) is composed to the lower part of the SMARTMASK (10, 20) so that the pulling power does not excessively occur and so that the SMARTMASK can play the role of adjustment.

47. For Section 1 and/or Section 2,
The SMARTMASK (10, 20) has the characteristic where the chin support part (114, 214) discharges the sweat that occurs from the skin and forms a hole for discharge

48. For Section 1 and/or Section 2,
The SMARTMASK (10, 20) has the characteristic of being composed with the form where the left-right contact parts (111, 211) overlap completely when folded based on the chin support part and the opening hole (113, 213).

49. For Section land/or Section 2,
The SMARTMASK (10, 20) has the characteristic of being composed so that the left-right contact part (111) plays the role of adjusting the glasses when wearing the glasses.

50. For Section 1 and/or Section 2,
The SMARTMASK (10, 20) has the characteristic of being composed so that the glasses can be worn on the exterior and interior of the left-right contact part (111).

51. For Section 2,
The SMARTMASK (10) has the characteristic where the left-right adjustment part (112) includes the upper part (112a) and lower part (112b). The left-right contact part (111) when wearing the mask is worn dimensionally to be hung to the neck skin below the ears and the temporal. The mask is then arranged to the back of the left-right ears. The thickness is from 300 ∼ 800mm. The width is from 7mm to 15mm. The length is from 60mm to 140mm. The general form is in ')' shape.

52. For Section 2,
The SMARTMASK (10) has the characteristic of being composed so that the left-right adjustment part (212) includes the upper part (212a) and lower part (212b), and the left-right contact part (211) can be worn dimensionally to the front part of the ears when marking the mask. Also, the mask is composed to be arranged in front of the left and right ears.

53. For Section 2,
The SMARTMASK (20) has the characteristic of forming "*L*" shape for the left-right adjustment part.

54. For Section 2,
The SMARTMASK (20) has the characteristic of forming thickness of 50∼ 300mm and width of 3mm ∼10mm for the left-right adjustment part (212).

55. For Section 2,
The SMARTMASK (20) has the characteristic of forming "(" shape for the overall shape for the left-right adjustment part (212).

56. For Section 2,
The SMARTMASK (20) has the characteristic of forming "<" shape for the overall shape for the left-right adjustment part (212)

57. The SMARTMASK (20) has the characteristic of being composed so that the left-right adjustment part (212) includes living hinge that expands the upper part (212a) and lower part (212b) of the adjustment part to upward and downward direction.

58. For Section 2,
The SMARTMASK (20) has the characteristic of being composed so that the left-right adjustment part (212) expands the upper part (212a) and lower part (212b) adjustment part to upward and downward direction.

59. For Section 1 and/or Section 2,
The SMARTMASK (20) has the characteristic of being composed so that the upper part and lower part of the left-right adjustment parts (112, 212) can be connected to the left/right/upper/lower band sub-material (121, 122, 221, 222). At least more than one contact part is composed.

60. For Section 1 and/or Section 2,
The SMARTMASK (10, 20) has the characteristic of being composed so that the upper and lower contact parts can be prevented with the contact with the skin. The support hole that is composed of certain depth is also composed for this mask

61. For Section 1 and/or Section 2,
The SMARTMASK (10, 20) has the characteristic of being composed so that the upper part and lower part of the contact part can be attached/detached easily for the left/right/upper/lower band sub-material (121, 122, 221, 222). Also, the upper and lower contact part can be freely assembled with the adjustment means (117,217).

62. For Section 1 and/or Section 2,
The SMARTMASK has the characteristic of manufacturing left/right/upper/lower band sub-material (121, 122, 221, 222) and the left-right contact part (111, 211). Then, they are combined due to the adjustment means (117, 217) to become all-in-one type.

63. For Section 1 and/or Section 2,
The SMARTMASK (10, 20) has the characteristic of being manufactured as a whole with the left/right/upper/lower band sub-material (121, 122, 221, 222) and the left-right contact part (111, 211).

64. For Section 1 and/or Section 2,
The SMARTMASK (10, 20) has the characteristic of being composed so that the left/right/upper/lower band sub-material (121, 122, 221, 222) to include rotating end part and free end part, and these are combined with the left-right adjustment part (112, 212) based on the assembly of the adjustment measure (117, 217).

65. For Section 1 and/or Section 2,
The SMARTMASK (10, 20) has the characteristic of using the triangulation concept. The left/right/upper/lower band sub-material (121, 122, 221, 222) use the same power to pull the left-right contact part (111, 211) and the other power is used to pull each left-right contact part so that the upper contact part of the left-right adjustment part is combined with the left-right upper band sub-material (121, 221). The mask is also aligned to the back of the head while being combined with the lower contact part of the left-right adjustment part for the left-right lower band sub-material (122, 222)

66. For Section 1 and/or Section 2,
The SMARTMASK (10, 20) has the characteristic where the left/right/upper/lower band sub-material (121, 122, 221, 222) is located in the top of the user's head or the back of the user's head.

67. For Section 1 and/or Section 2,
The SMARTMASK (10, 20) has the characteristic of being composed where the left/right/upper/lower band sub-material (121, 122, 221, 222) can be classified in the exterior of from the exterior surface and/or interior surface. Also, it is composed of unit marking.

68. For Section land/or Section 2,
The SMARTMASK (10, 20) has the characteristic of being composed where the left/right/upper/lower band sub-material (121, 122, 221, 222) has outstanding flexibility.

69. For Section 1 and/or Section 2,
The SMARTMASK (10, 20) has the characteristic of elongation rate (%) from 100~1000 for the left/right/upper/lower band sub-material

70. For Section 1 and/or Section 2,
The SMARTMASK (10, 20) that has the characteristic of being composed where the left/right/upper/lower band sub-material (121, 122, 221, 222) is pulled to be expanded with the left/right/upper/lower band sub-material of the left-right contact part (111, 211). It has the characteristic of also being composed to have flexibility like the left-right contact part (111, 211).

71. For Section 1 and/or Section 2,
The SMARTMASK (10, 20) has the characteristic of being composed so that the left/right/upper/lower band sub-material (121, 122, 221, 222) is pulled to be expanded with the left/right/upper/lower band sub-material and the left-right contact part (111, 211). As a result, the left-right contact part (111, 211) is worn closely to the face. The left/right/upper/lower band sub-material (121, 122, 221, 222) can be worn on the head of the user.

72. For Section 1 and/or Section 2,
The SMARTMASK (10, 20) has the characteristic of being composed so that the left/right/upper/lower band sub-material (121, 122, 221, 222) is pulled to be expanded with the left/right/upper/lower band sub-material and the left/right contact part (111, 211). As a result, the pressure of wearing the SMARTMASK is dispersed to the left/right/upper/lower band sub-material (121, 122, 221, 222) and the left-right contact part (111,211).

73. For Section 1 and/or Section 2,
The SMARTMASK (10, 20) has the characteristic of being formed where the left/right/upper/lower band sub-material (121, 122, 221, 222) uses the sticking condition to attach between each other, and the frictional force is superior.

74. For Section 2,
The SMARTMASK (10, 20) has the characteristic of elongation rate (%) from 300~ 1000 on the left-right earring part (124).

75. For Section 2,
The SMARTMASK (20) has the characteristic of being formed where the left/right earring part (124) minimizes the pressure that is allowed to the left and right ears of the user. Also, the left earring part (124L) and the right earring part (124R) are pulled to adjust the mask to the back of the head.

76. For Section 2,
The SMARTMASK (20) has the characteristic of being composed so that when the left/right earring part is pulled, the left/right earring part (124) and the left/right contact part (211) are expanded. As a result, the left/right contact part (211) is worn closely to the face and the left/right earring part (124) can be hung to the left and right ears of the user.

77. For Section 2,
The SMARTMASK (20) has the characteristic of being formed so that the pulling power to the left-right earring part expands the same time with the left/right earring part (124) and left/right contact part (211). As a result, the pressure of wearing the SMARTMASK is dispersed to the left/right earring part(124) and the left/right contact part (211).

78. For Section 1 and/or Section 2,
The SMARTMASK (10, 20) has the characteristic where the cover part (130, 230) can be located only on the nose of the user due to the upper frame, lid part, expansion sub-material, wall part, cover part, and filter part. The inside has a small internal gas space where the lip and upper part (133, 233) are located. The front cover (134, 234) is divided as well.

79. For Section 1 and/or Section 2,
The SMARTMASK (10, 20) has the characteristic of being composed with the upper cover, upper frame, and opening hole frame that are combined as one. The lid part that is composed of the upper part of the cover is contacted to the middle of the forehead and the wall part is hung to the middle of the philtrum from the upper internal surface of the cover part. The cover part is composed as a whole with the wall part where it is contacted closely to the philtrum part. The mask is also structured with the cover part (130, 230) to be completely separate from the external environment.

80. For Section 1 and/or Section 2,
The SMARTMASK (10, 20) has the characteristic of having adjustment power for the opening hole (133, 233) due to the result of the upper frame of the cover part being combined with the frame of the opening hole to prevent the condition of the opening hole (133, 233) being excessively pulled with resistant to the power of pulling form the left/right/upper/lower band sub-material when the pulling power is implemented.

81. For Section 1 and/or Section 2,
The SMARTMASK (10) has the characteristic of being composed where the cover part's upper part (133, 233) and the internal and/or internal gas space does not contact the internal part of the upper part of the nose and cover where it only covers the nose.

82. For Section 1 and/or Section 2,
The SMARTMASK (10, 20) has the characteristic of being composed with the cover part's upper part (133, 233) and the internal and/or internal gas space completely separates the mouth and the noise of the user, and it is formed as the same size with the user's nose to be placed only for the nose. When looking flat, it has a triangle shape "Δ".

83. For Section 1 and/or Section 2,
The SMARTMASK (10, 20) has the characteristic of being composed where the cover part's upper part (133, 233) does not contact the nose when wearing the SMARTMASK and it hangs to the middle of the forehead between the pupils to include the lid part (137, 237).

84. For Section 1 and/or Section 2,
The SMARTMASK (10, 20) has the characteristic where the cover part's upper part (133, 233) does not contact the nose and the lid part (137, 237), and the curve part (137e, 237e) is composed so that the mask can be closely worn by hanging on the middle of the forehead between the pupils.

85. For Section 1 and/or Section 2,
SMARTMASK (10, 20) has the characteristic where the lid part (137, 237) is closely hung to the middle of the forehead between the pupils and the thickness is thinner in contrast to the upper part of the cover part.

86. For Section 1 and/or Section 2,
The SMARTMASK (10, 20) has the characteristic where the lid part (137, 237) is hung to the middle of the forehead between the pupils.

87. For Section 1 and/or Section 2,
The SMARTMASK (10, 20) has the characteristic where the lid part (137, 237) is hung to the middle of the forehead between the pupils of the user when wearing the SMARTMASK, and the curve part is expanded to upper direction. The part is then worn from between the pupils to the middle of the forehead (to both eyebrows).

88. For Section 1 and/or Section 2,
The SMARTMASK (10, 20) has the characteristic where the lid part (137, 237) can cover the middle of the forehead to form central point. When looking flat, it is in "U" shape which is slightly higher than the central point for the left and right sides.

89. For Section 1 and/or Section 2,
The SMARTMASK (10, 20) has the characteristic of being formed where the lid part (137, 237) is composed by including the nose clip (137c) that presses the contact part with the inner part of the lid part with the nose to adjust interval with the internal surface lid part and the nose.

90. For Section 1 and/or Section 2,
The SMARTMASK (10, 20) has the characteristic where the lid part (137, 237) is hung to the middle of the forehead between the pupils where the lid part contacts the lid part above (137, 237) due to the stress that presses the nose clip when using the SMARTMASK (10,20) with the composition of the nose clip (137c) to adjust the interval with the inner surface of the contact part and the nose.

91. For Section 1 and/or Section 2,
The SMARTMASK (10, 20) has the characteristic where the lid part (137, 237) is adjusted as the lid with free opened space is provided with the middle of the forehead of the user and the lid part according to specific situation when wearing the SMARTMASK (10, 20).

92. For Section 1 and/or Section 2,
The SMARTMASK (10, 20) has the characteristic where the lid part (137, 237) is hung to the middle of the forehead in upward direction from the central point of the left/right upper part's contour part (111a, 211a) to be worn by sticking out. The whole form of wearing looks like "⊥" when looking from the front.

93. For Section 1 and/or Section 2,
The SMARTMASK (10, 20) has the characteristic where the cover part's upper par t(133, 233) and/or the small internal gas space are composed with the filter part, and the filter part (139, 239) is composed in front of both nostrils (left and right).

94. For Section 1 and/or Section 2,
The SMARTMASK (10, 20) has the characteristic where the cover part's upper part (133,233) and/or the small internal gas space are composed with a filter part, and the filter part (139, 239) is composed in front of both nostrils (left and right).

95. In Section 94,
The SMARTMASK (10, 20) has the characteristic where the interval between the nostrils and the filter par t(139, 239) is around 1mm~10mm.

96. For Section 1 and/or Section 2,
The SMARTMASK (10, 20) has the characteristic where the wall is formed as a whole along the curved inner surface of the internal surface. The other end is curved towards the front, and the cover part (131a) is extended. When looking flat, it has the form of "L" shape.

97. For Section 1 and/or Section 2,
The SMARTMASK (10, 20) has the characteristic where the wall is composed of the combined hole and the bio signal measuring device can be attached/detached to measure the bio signal.

98. For Section 1 and/or Section 2,
The SMARTMASK (10, 20) has the characteristic where the wall is formed with combined hole and the combined hole can be attached/detached with the filter part.

99. For Section 1 and/or Section 2,
The SMARTMASK (10, 20) has the characteristic where the wall is composed of the combined hole and the combined hole can be attached with oxygen occurrence device, respiratory treatment device, scent occurrence device, and smart sensor.

100. For Section 1 and/or Section 2,
The SMARTMASK (10, 20) has the characteristic where the wall can quickly discharge the air that includes carbon dioxide. The ventilation is also composed.

101. For Section 1 and/or Section 2,
The SMARTMASK (10, 20) has the characteristic to be composed to close the space in the philtrum along the philtrum which is correspondent to the philtrum of a person when wearing the SMARTMASK (10, 20).

102. For Section 1 and/or Section 2,
The SMARTMASK (10, 20) has the characteristic where the cover part's thickness is from 50~100mm to prevent close contact along the philtrum.

103. For Section 1 and/or Section 2,
The SMARTMASK (10, 20) has the characteristic where the cover part's width is around 4mm~15mm so the surfaces can contact with the philtrum.

104. For Section 1 and/or Section 2,
The SMARTMASK (10, 20) has the characteristic where the cover part has low central part towards the length of the respiratory system (2 nostrils) to closely stick according to the philtrum of the user. Both ends are relatively high compared to the central part. When looking flat, it is composed of "U" Shape.

105. For Section 1 and/or Section 2,
The SMARTMASK (10, 20) has the characteristic where the expansion sub-material is expanded towards the plate towards the internal gas space from the left end of the small internal gas space where the cover part in the lower section is connected and the lid part (137, 237) in the upper section is expanded and connected.

106. For Section 1 and/or Section 2,
The SMARTMASK (10, 20) has the characteristic where the front cover does not combine with the left-right contact parts and the left-right ends are relatively higher than the central point. When looking flat, it has "U" shape and the lid is towards the upper part according to situation.

107. For Section 1 and/or Section 2,
The SMARTMASK (10, 20) has the characteristic where the front cover does not directly contact the mouth and chin where it is placed with prescribed distance. The user's mouth, cheek, chin are covered for protection.

108. For Section 1 and/or Section 2,
The SMARTMASK (10, 20) has the characteristic of being whole with the upper part (133, 233) for the front cover that is like a lid to the upper part.

109. For Section 1 and/or Section 2,
The SMARTMASK (10, 20) has the characteristic where the front cover has a hole for conversation on the part that corresponds to the mouth.

110. The SMARTMASK (10, 20) has the characteristic where the cover part (130, 230) includes the cutting line on the straight surface of the front cover and it can be cut according to the cutting line above. According to situation, the front cover may not be composed.

111. For Section 1 and/or Section 2,
The SMARTMASK (10, 20) has the characteristic where the filter part can be attached/detached to the upper part (133, 233) of the cover part.

112. For Section 1 and/or Section 2,
The SMARTMASK (10, 20) has the characteristic of being composed where the filter includes a filter that removes dusts and filter that heats/humidifies the air when breathing.

113. For Section 1 and/or Section 2,
The SMARTMASK (10, 20) has the characteristic where heated/humidified air is provided to the user through the small internal gas space and/or internal part of the upper cover part in the case the user breathes while wearing the SMARTMASK.

114. For Section 1 and/or Section 2,
The SMARTMASK (10, 20) has left and right bio signal measuring device that is contacted at the same time to the left and right ears or to one ear (either left or right). On Top, the SMARTMASK is worn as covering-type. The left and right bio signal measuring devices are stably attached to the skin to prevent movement. Also, it blocks external light and external environment to measure the bio signal.

115. For Section 1 and/or Section 2,
The SMARTMASK (10, 20) is worn to the face of the user. To use the mask, the user holds and extends left and right upper band sub-material (121, 22) with both hands (Stage 1);
The front chin is placed on the chin support (114, 214), or also known as specific part of location' (Stage 2);
The left and right upper band sub-materials (121, 221) are pulled towards the top to be adjusted to the top of the head (210) (Stage 3);
The left-right lower band sub-material (122, 222) is adjusted to the back of the head (220) of the user (Stage 4); These stages are included in wearing the SMARTMASK (10, 20).

116. For Section 1 and/or Section 2,
The SMARTMASK (10, 20) has the characteristic of being used during sleep.

117. For Section 1 and/or Section 2,
The SMARTMASK (10, 20) has the characteristic of being used to measure bio signal during sleep.

118. For Section 1 and/or Section 2,
The SMARTMASK (10, 20) has the characteristic of being used during exercise.

119. For Section 1 and/or Section 2,
The SMARTMASK (10, 20) has the characteristic of being used to measure bio signal during exercise.
